# EUROPEAN PATENT APPLICATION

(11) **EP 4 549 455 A1**
(43) Date of publication of application: **07.05.2025**
(21) Application number: 23850377.5
(22) Date of filing: 31.07.2023
(51) Int. Cl.: C07K 14/71, A61K 38/00, C12N 15/62

(54) **MODIFIED FUSION PROTEIN AND USE THEREOF**

(30) Priority: 02.08.2022 KR 20220096226
(71) Applicant: Panolos Bioscience, Inc., Hwaseong-si Gyeonggi-do 18471 (KR)
(72) Inventor: LIM, Hyeseong, Seongnam-si Gyeonggi-do 13567 (KR); YANG, Hyungul, Asan-si Chungcheongnam-do 31579 (KR); HONG, Seung Kon, Hwaseong-si Gyeonggi-do 18447 (KR)
(74) Representative: Schulz Junghans Patentanwälte PartGmbB
(86) International application number: PCT/KR2023/011163
(87) International publication number: WO 2024/029876

(57) **Abstract**

The present disclosure relates to a modified fusion proteins in which a vascular endothelial growth factor is associated with a placental growth factor, and a use thereof. The modified fusion protein according to an embodiment of the present disclosure can exhibit excellent productivity and stability. The modified fusion protein according to an embodiment of the present disclosure can also demonstrate excellent blood stability. The modified fusion protein according to an embodiment of the present disclosure, compared to conventionally developed substances, possesses acidic properties and can show an increased ratio of charge isoforms in the acidic and neutral regions of the pI distribution.

## Description

### TECHNICAL FIELD

The present disclosure relates to modified fusion proteins that bind to vascular endothelial growth factor and placental growth factor and their uses.

### BACKGROUND ART

Vascular endothelial growth factor (VEGF) is a signaling protein that plays an important role in vasculogenesis and angiogenesis. Its functions include formation of new blood vessels during embryonic development, in muscles after injury or exercise, and new blood vessels that bypass blocked blood vessels, but abnormal increases in VEGF are closely related to tumor development and metastasis (Carmeliet, P., Jain, RK. 2000. Nature 407: 249-257).

VEGF is classified into five types: VEGF-A, -B, -C, -D, and PLGF (placental growth factor), which are known to contribute to angiogenesis (Carmeliet, P., Jain, RK. 2000. Nature 407: 249-257; Kuwano M, et al. 2001. Intern Med 40: 565-572). On the other hand, there are three types of VEGF receptors: VEGF R1, R2, and R3. The type and binding affinity of ligands differ depending on the type of receptor. Among the receptors, VEGFR-1/-2 are known to be involved in angiogenesis (Veikkola, et. al., 2000. Cancer research 60: 203-212).

Continued efforts have been directed at developing drugs targeting angiogenesis inhibition through removal of vascular endothelial growth factor, which is known to be a key factor in tumorigenesis. Bevacizumab, a drug in the form of a monoclonal antibody targeting VEGF-A (Stacker, et. al., 2013. Chinese Journal of Cancer Research. 32(6):297-302.; Kazazi-Hyseni, et. al., 2010. Oncologist. 15(8):819-825; DrugBank Accession Number: DB00112), is representative. The mechanism of action of monoclonal antibody drugs involves binding to a single vascular endothelial growth factor, but since multiple growth factors are involved in canceration, monoclonal antibodies were not effective drugs to show a remarkable efficacy. Bevacizumab actually induced increased expression of other types of growth factors that were not targeted, by intracellular compensatory mechanisms, resulting in the side effect of resistance (Bagley, et. al., 2011. Clinical cancer research 17(5): 976 -988; Lieu, et. al., 2013. Plos One 8: e77117; Cutsem, et. al., 2020 Clinical cancer research 26(3): 717-725). For this reason, the need to develop drugs that target multiple vascular growth factors related to canceration has gradually increased. In particular, the importance of PLGF, whose expression rate increases due to a compensatory pathway in the late stage of canceration, is being highlighted, but the majority of drugs already developed or currently being developed target VEGF. So, the need for the development of PLGF-targeting drugs is growing.

To overcome the shortcomings of monoclonal antibody drugs, drugs that target multiple vascular endothelial growth factors are being developed. Representative protein drugs are VEGF-Trap (Ciombor, et. al., 2013. Clinical cancer research. 19 (8): 1920-1925. DrugBank Accession Number: DB08885) and VEGF-Grab (Lee. et al., 2015. Mol. Cancer. Ther. 14(2):470-9). Both of these drugs mimic part of the decoy receptor and are recombinant fusion proteins in which the Fc of an immunoglobulin is fused with a heterologous protein VGFR1 D2-VGFR2 D3 in the case of VEGF-Trap, and with a homologous protein VEGFR1 D2-D3 in the case of VEGF-Grab. Efforts to improve physicochemical properties of such fusion proteins targeting multiple vascular endothelial growth factors have continued. In particular, there has been a need for the development of fusion proteins that have high binding affinity to target substances and increased persistence in the body while also having physical and chemical stability that enables high productivity at the level of protein therapeutics in the context of commercial scale production.

### DISCLOSURE

### TECHNICAL PROBLEM

Improved physicochemical properties of a protein can maximize production efficiency by enhancing the physical and chemical stability of the protein during expression and purification and reduce non-specific binding and degradation when the protein is introduced into the body, thereby achieving high persistence in the body.

The inventors of the present disclosure have continuously made efforts to efficiently improve the physicochemical properties and stability of fusion proteins containing an extracellular domain of VEGFR1.

As a result, the inventors of the present disclosure developed modified fusion proteins by introducing, into a fusion protein containing VEGFR1 domain D2 and domain D3, an amino acid variation in a specific region of domain D3, length adjustment and variation of the linker, or a disulfide bond. The present disclosure was completed by ascertaining that this modified fusion proteins exhibit excellent productivity and stability.

### TECHNICAL SOLUTION

1. In one embodiment, the present disclosure relates to a modified fusion protein comprising a VEGFR1 extracellular domain, a linker and a multimerization domain, wherein the VEGFR1 extracellular domain comprises an immunoglobulin (Ig)-like domain D2 and an Ig-like domain D3 of VEGFR1, and the linker is located between the Ig-like domain D3 and the multimerization domain, and wherein the modified fusion protein has one or more of characteristics (a) to (c): (a) one or more amino acid residues on a β1-β2 loop of domain D3, one or more amino acid residues on a β2-β3 loop of domain D3, and/or one or more amino acid residues on a β5-β6 loop of domain D3 have been substituted with an amino acid that reduces the net pI of a protein, an amino acid having a negatively charged side chain, or an amino acid having an electronegative side chain, wherein, the β1-β2 loop of domain D3 includes amino acid residues T236 to T247 of the amino acid sequence of SEQ ID NO: 1, the β2-β3 loop includes amino acid residues T256 to V262 of the amino acid sequence of SEQ ID NO: 1, and the β5-β6 loop includes amino acid residues D299 to L308 of the amino acid sequence of SEQ ID NO: 1; (b) the linker has a length of about 13 to about 35 amino acids; and (c) a disulfide bond is present in the fusion protein, and one of the amino acid residues on the β1-β2 loop of domain D3 or one of the amino acid residues on the β5-β6 loop of domain D3, and one of the amino acid residues at positions -2, -1, 0, +1, +2, and +3 relative to Y329 of domain D3 are substituted with cysteine.
2. In one embodiment, the amino acid substitution in characteristic (a) may be a substitution with: an amino acid residue of the VEGFR2 homologous sequence, an amino acid residue that is serine, threonine, tyrosine, cysteine, asparagine, glutamine, aspartic acid, or glutamic acid, or an amino acid residue having a short side chain which is alanine or glycine.
3. In one embodiment, in characteristic (a), one or more amino acid residues on a β1-β2 loop of domain D3 and one or more amino acid residues on a β5-β6 loop of domain D3 may have been substituted with an amino acid that reduces the net pI of a protein, an amino acid having a negatively charged side chain, or an amino acid having an electronegative side chain.
4. In one embodiment, in characteristic (a), one or more amino acid residues on the β2-β3 loop of domain D3 may have been substituted with an amino acid residue having a smaller side chain than the side chain of the existing amino acid residue or an amino acid residue having a polar side chain of a similar size to the side chain of the existing amino acid residue.
5. In one embodiment, characteristic (a) may be substitution of: one or more of the amino acid residues K241, L243, R244, and H246 on the β1-β2 loop of domain D3; amino acid residue L258 on the β2-β3 loop of domain D3; and/or one or more of the amino acid residues K300, Q302, K304, and K306 on the β5-β6 loop of domain D3.
6. In one embodiment, characteristic (a) may comprise: one or more amino acid substitutions selected from K241T, K241E, L243S, R244V, and H246E on the β1-β2 loop of domain D3; amino acid substitution L258A, L258S, or L258D on the β2-β3 loop of domain D3; and/or one or more amino acid substitutions selected from K300G, Q302T, K304S, and K306Q on the β5-β6 loop of domain D3.
7. In one embodiment, characteristic (a) may comprise: (i) amino acid substitutions L243S and R244V, or (ii) amino acid substitutions K241E, L243S, R244V, and H246E on the β1-β2 loop of domain D3; amino acid substitution L258A, L258S, or L258D on the β2-β3 loop of domain D3; and/or (i) amino acid substitution K300G, (ii) amino acid substitutions K300G, Q302T, and K304S, or (iii) amino acid substitutions K300G, Q302T, K304S, and K306Q on the β5-β6 loop of domain D3.
8. In one embodiment, characteristic (a) may comprise: amino acid substitutions K241E, L243S, R244V, and H246E on the β1-β2 loop of domain D3; amino acid substitution L258A, L258S, or L258D on the β2-β3 loop of domain D3; and/or amino acid substitutions K300G, Q302T, and K304S on the β5-β6 loop of domain D3.
9. In one embodiment, the modified fusion protein may comprise amino acid substitutions R238S and R275N in domain D3.
10. In one embodiment, in the modified fusion protein, the amino acid sequence of the C terminus of domain D3 connected to the linker is KALE based on the amino acid residue K331, provided that one or more of the amino acids K, A, L and E may be deleted.
11. In one embodiment, in the modified fusion protein, the N terminus of domain D2 may begin with the amino acid sequence SDT or the amino acid sequence EF.
12. In one embodiment, in characteristic (b), the linker may have a length of about 14 amino acids or more.
13. In one embodiment, in characteristic (b), the linker comprises a GS repeat sequence, and the GS repeat sequence is a 2 to 60 amino acids long amino acid sequence consisting only of G and S, and the N-terminal amino acid of the linker may be glutamic acid.
14. In one embodiment, the GS repeat sequence is (GS)n, (GSSG)n, (GGGGS)n, or (GS)m(GGGGS)n, wherein n is an integer of 1 to 10, and m is an integer of 0 to 10.
15. In one embodiment, the GS repeat sequence may be selected from the group consisting of GS, GSSG, (GSSG)2, GGGGS, (GGGGS)4, GS(GGGGS), and GS(GGGGS)3.
16. In one embodiment, in characteristic (b), the linker comprises an amino acid sequence of a hinge region derived from an immunoglobulin, and the amino acid sequence of the hinge region may be modified.
17. In one embodiment, the hinge region derived from an immunoglobulin may comprise a sequence derived from the CH1 region, an upper hinge, and/or a core hinge.
18. In one embodiment, the hinge region derived from an immunoglobulin may be derived from human IgA, IgD, IgM, IgE, or IgG.
19. In one embodiment, in characteristic (b), a papain recognition site or a glycosylation site present in the hinge region derived from an immunoglobulin may have an amino acid variation.
20. In one embodiment, in characteristic (b), the amino acid variation in the papain recognition site may be substitution of one or more amino acid residues in the papain recognition site with alanine, serine, tyrosine, proline, or threonine, or insertion of a 1 to 10 amino acids long amino acid sequence comprising at least one amino acid having an aromatic or cyclic carbon in the side chain into the papain recognition site, and the amino acid variation in the glycosylation site may be (i) deletion of serine or threonine present in the hinge region derived from an immunoglobulin, or (ii) substitution of serine or threonine present in the hinge region derived from an immunoglobulin with an amino acid other than serine, asparagine, or threonine.
21. In one embodiment, the amino acid other than serine, asparagine, or threonine may be glycine or alanine.
22. In one embodiment, cysteine present in the core hinge may be substituted with serine or glycine.
23. In one embodiment, in characteristic (b), the linker may comprise: (i) an amino acid sequence selected from the group consisting of GS, GSSG, (GSSG)2, GGGGS, (GGGGS)4, GS(GGGGS) and GS(GGGGS)3; and (ii) a hinge region derived from an immunoglobulin selected from the group consisting of human IgG1, IgG4, or IgD/G1, in that order starting from the N terminus, wherein the amino acid sequence of the hinge region may be modified.
24. In one embodiment, in characteristic (b), the linker may be an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP.
25. In one embodiment, in characteristic (c), said one of the amino acid residues on the β1-β2 loop may be one of the amino acid residues on the amino acid sequence of V240 to T247.
26. In one embodiment, in characteristic (c), one of amino acid residues T241, L243, and R244 on the β1-β2 loop of domain D3 or amino acid residue N303 on the β5-β6 loop may be substituted with cysteine, and one of the amino acid residues at positions 0, +2, and +3 relative to Y329 of domain D3 may be substituted with cysteine.
27. In one embodiment, in characteristic (c), T241 and the amino acid residue at position +3 relative to Y329 of domain D3 are substituted with cysteine; amino acid variations L243C and Y329C are introduced; L243 and the amino acid residue at position +2 relative to Y329 of domain D3 are substituted with cysteine; amino acid variations R244C and Y329C are introduced; or amino acid variations N303C and Y329C are introduced.
28. In one embodiment, the modified fusion protein may have all of characteristics (a) to (c): (a) one or more amino acid substitutions selected from K241T, K241E, L243S, R244V, and H246E on the β1-β2 loop of domain D3, amino acid substitution L258A, L258S, or L258D on the β2-β3 loop of domain D3, and/or one or more amino acid substitutions selected from K300G, Q302T, K304S, and K306Q on the β5-β6 loop of domain D3 are introduced; (b) the linker is an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP; and (c) one of amino acid residues T241, L243, and R244 on the β1-β2 loop of domain D3 or amino acid residue N303 on the β5-β6 loop is substituted with cysteine, and one of the amino acid residues at positions 0, +2, and +3 relative to Y329 of domain D3 is substituted with cysteine.
29. In one embodiment, the modified fusion protein may have all of characteristics (a) to (c): (a) (i) amino acid substitutions L243S and R244V, or (ii) amino acid substitutions K241E, L243S, R244V, and H246E on the β1-β2 loop of domain D3, amino acid substitution L258A, L258S, or L258D on the β2-β3 loop of domain D3, and/or (i) amino acid substitution K300G, (ii) amino acid substitutions K300G, Q302T, and K304S, or (iii) amino acid substitutions K300G, Q302T, K304S, and K306Q on the β5-β6 loop of domain D3 are introduced; (b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP; and (c) L243 and the amino acid residue at position +2 relative to Y329 of domain D3 are substituted with cysteine.
30. In one embodiment, the modified fusion protein may have all of characteristics (a) to (c): (a) amino acid substitutions K241E, L243S, R244V, and H246E on the β1-β2 loop of domain D3, amino acid substitution L258A, L258S, or L258D on the β2-β3 loop of domain D3, and amino acid substitutions K300G, Q302T, and K304S on the β5-β6 loop of domain D3 are introduced; (b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP; and (c) L243 and the amino acid residue at position +2 relative to Y329 of domain D3 are substituted with cysteine.
31. In one embodiment, the modified fusion protein may consist of an amino acid sequence selected from the group consisting of SEQ ID NO: 10 to SEQ ID NO: 70, SEQ ID NO: 77 to SEQ ID NO: 93, and SEQ ID NO: 98.
32. In one embodiment, the multimerization domain may be: (a) Fc region of an immunoglobulin; (b) CH3 region of IgG1, IgG2, IgG3, or IgG4; (c) CH2 and CH3 regions of IgG1, IgG2, IgG3, or IgG4; (d) Fc region of an immunoglobulin comprising an amino acid sequence having at least 85% identity to SEQ ID NO:99; or (e) Fc region of an immunoglobulin comprising the amino acid sequence of SEQ ID NO: 99.
33. In one embodiment, the multimerization domain may be the Fc region of an immunoglobulin and may start from the lower hinge region of the Fc region.
34. In one embodiment, the multimerization domain may be the Fc region of IgG1 consisting of SEQ ID NO: 99.
35. In one embodiment, the multimerization domain may have amino acid substitution D126E, amino acid substitution L128M, and/or the amino acid deletion of K217 based on SEQ ID NO: 99.
36. In one embodiment, the modified fusion protein may be in dimer or multimer form.
37. In one embodiment, the present disclosure relates to a pharmaceutical composition for the prevention or treatment of an autoimmune disease, inflammatory disease, neoplastic disease, cancer, angiogenesis-related disease, or ocular disease, comprising a modified fusion protein according to an embodiment of the present disclosure as an active ingredient.
38. In one embodiment, the present disclosure relates to a nucleic acid molecule encoding a modified fusion protein according to an embodiment of the present disclosure.
39. In one embodiment, the present disclosure relates to a host cell comprising a nucleotide sequence encoding a modified fusion protein according to an embodiment of the present disclosure.
40. In one embodiment, the present disclosure relates to a vector comprising a nucleotide sequence encoding a modified fusion protein according to an embodiment of the present disclosure.
41. In one embodiment, the vector may be a recombinant viral vector.
42. In one embodiment, the present disclosure relates to a pharmaceutical composition for delivering a viral vector to a subject, comprising a recombinant viral vector according to an embodiment of the present disclosure, wherein the fusion protein encoded by the recombinant viral vector is expressed in the subject and is intended for the prevention or treatment of an autoimmune disease, inflammatory disease, neoplastic disease, cancer, angiogenesis-related disease, or ocular disease.
43. In one embodiment, the recombinant viral vector may be a recombinant adeno-associated viral vector.

### ADVANTAGEOUS EFFECTS

A modified fusion protein according to one embodiment of the present disclosure can exhibit excellent productivity, physical stability, and chemical stability.

A modified fusion protein according to one embodiment of the present disclosure can exhibit excellent serum stability.

A modified fusion protein according to an embodiment of the present disclosure has acidic properties compared to previously developed substances and may exhibit an increased ratio of charge variants in the acidic and neutral regions of the pI distribution. Accordingly, the modified fusion protein can have the effect of improved non-specific interactions with the matrix and cell surface in the body.

### DESCRIPTION OF DRAWINGS

FIG. 1a is a 3D structural diagram showing the surface charge distribution of the D2-D3 domains of VEGFR1 and VEGFR2. The diagram shows the binding surface of the ligand VEGF (indicated by a green circle) and the area where the loop of the D3 domain, at which variations can be introduced, is located (indicated by a red circle).
FIG. 1b is a 3D structural diagram showing the positions of exemplary amino acid residues where variations may occur in domain D3 of VEGFR1.
FIG. 1c is 3D structural diagrams showing exemplary amino acid residues in domain D3 of VEGFR1 which can be variated and their structures. In FIG. 1c, (A) is a diagram showing amino acid residues in the variation site and the positions of their side chains using stick representation the, and (B) shows a view obtained by rotating (A) 45 degrees.
FIG. 2 is 3D structural diagrams showing the VEGFR1 extracellular domains. FIG. 2a shows the structure of VEGFR1 when it binds to a VEGF ligand. FIGS. 2b and 2c show a fusion protein in which domain D2 and domain D3 of VEGFR1 are combined with an Fc region (e.g., VEGF-Grab3), when it has an open structure before binding to a ligand (FIG. 2b) and when it has a closed structure after binding to a ligand (FIG. 2c), through 3D modeling. FIG. 2d is a diagram showing the distance from the end of D3 to the dimer formation site of domain D4 when VEGFR1 in its native state forms a dimer.
FIG. 3 is an exemplary schematic diagram for designing a linker introduced in the present disclosure.
FIG. 4a and FIG. 4b are graphs representing the serum stability and the VEGF-A binding ability of proteins.
FIG. 5a to FIG. 5c are graphs representing the serum stability of proteins.
FIG. 6a to FIG. 6f are graphs representing the isoelectric point of proteins.
FIG. 7a to FIG. 7d are graphs analyzing pI distribution of proteins.
FIG. 8a to FIG. 8d are graphs analyzing the thermal stability of proteins.
FIG. 9a to FIG. 9c are graphs representing the VEGF-A binding ability of proteins.

### MODE FOR INVENTION

The various embodiments or examples described in this document are exemplary and explanatory only and are not restrictive of the technical idea of the present disclosure. The technical idea of the present disclosure encompasses various modifications, equivalents, and alternatives of each embodiment or example described in this document as well as selective combinations of all or part of individual embodiments or examples.

Unless defined otherwise, all technical and scientific terms used herein have the same meaning as commonly understood by one of ordinary skill in the art to which the present disclosure pertains.

Use of the singular herein may include the plural unless the context dictates otherwise, and this also applies to singular expressions recited in the claims.

### I. Definitions

In the present disclosure, the term "about" may indicate a typical error range for a particular value recited, as is well known to one of ordinary skill in the art. When used in the context of numerical values or ranges stated in this disclosure, it may mean ±20%, ±15%, ±10%, ±9%, ±8%, ±7%, ±6%, ±5%, ±4%, ±3%, ±2%, or ±1% of a numerical value or range that is recited in an embodiment or claimed. With respect to the length of a nucleotide or amino acid sequence, "about" can indicate that the nucleic acid or protein is not limited to the recited number of nucleotides or amino acids, and a few nucleotides or amino acids added to or removed from either terminus may be included so long as the functional activity is not impeded.

As used herein, expressions such as "comprising," "including," "having," and the like should be construed as open-ended terms that imply the possibility of inclusion of other embodiments in a similar manner to "comprising," unless stated otherwise in the phrase or sentence in which the expressions are included.

As used herein, the term "and/or" may mean, with respect to items associated with the term, any one or more of the items, any combination of the items, or all of the items.

As used herein, the term "amino acid" may refer to all naturally occurring L-α-amino acid. This definition is meant to include norleucine, ornithine, and homocysteine.

As used herein, the term "variation" or "amino acid variation" may refer to a substitution, an insertion, a deletion, or a combination thereof in an amino acid sequence as compared to a reference (e.g., native sequence) polypeptide or protein, and the term "variant" may refer to a molecule that has some differences in its amino acid sequence as compared to a reference polypeptide or protein due to such variations.

The scope of variants herein also includes proteins or fragments or derivatives thereof which exhibit the same or similar biological activity and derivatives which are differentially modified during or after translation, e.g., by glycosylation, proteolytic cleavage, linkage to an antibody molecule or other cellular ligand, and so on.

As used herein, the term "carriers" may include pharmaceutically acceptable carriers, excipients, or stabilizers which are non-toxic to cells or mammals at the dosages and concentrations employed. Often the pharmaceutically acceptable carrier is an aqueous pH buffered solution. Examples of pharmaceutically acceptable carriers include buffers such as phosphate, citrate, and other organic acids; antioxidants including ascorbic acid; low molecular weight (less than about 10 residues) polypeptides; proteins such as serum albumin, gelatin, or immunoglobulins; hydrophilic polymers such as polyvinylpyrrolidone; amino acids such as glycine, glutamine, asparagine, arginine, or lysine; monosaccharides, disaccharides, and other carbohydrates, including glucose, mannose, or dextrins; chelating agents such as EDTA; sugar alcohols such as mannitol or sorbitol; salt-forming counterions such as sodium; and/or nonionic surfactants such as TWEEN^{®}, polyethylene glycol (PEG), and PLURONICS^{®}, but are not limited thereto.

As used herein, the term "effective amount" may refer to an amount sufficient to effect beneficial or desired clinical or biochemical results. An effective amount can be administered in one or more administrations. An effective amount may refer to an amount that is sufficient to palliate, ameliorate, stabilize, slow or delay the progression of a disease state.

As used herein, the term "ligand" may refer to a molecule (e.g., VEGF-A, -B, -C, -D, and PLGF) which binds with high affinity to a modified fusion protein. In other contexts, "ligand" can mean any molecule or agent, or compound that binds specifically to a molecule such as a polypeptide or protein covalently or transiently.

As used herein, the term "individual" or "subject" may refer to a mammal. The mammal includes, but is not limited to, domestic animals (e.g., cattle, sheep, cats, dogs, and horses), primates (e.g., humans and non-human primates such as monkeys), rabbits, and rodents (e.g., mice and rats). In one embodiment, the individual or subject may be a human.

As used herein, the term "pharmaceutically acceptable carrier and/or diluent" may include, but is not limited to, any and all solvents, dispersion media, coating agents, antibacterial and antifungal agents, isotonic agents, and adsorption delaying agents and the like.

An embodiment of this disclosure described herein is to be understood to encompass "comprising," "consisting of," and/or "consisting essentially of" the embodiment.

### II. Modified fusion proteins and components of modified fusion proteins

### 1. Vascular endothelial growth factor receptor (VEGFR)

In one embodiment, a modified fusion protein of the present disclosure may comprise a VEGFR1 extracellular domain as its component. VEGFR1 is also known as fms-related tyrosine kinase (FLT-1) and is encoded by the *FLT1* gene. The amino acid sequence of VEGFR1 is presented as SEQ ID NO: 1, which can be found in UniProtKB, #P17948. In one embodiment, the VEGFR1 is from a mammal such as a human. VEGFR1 has seven immunoglobulin (Ig)-like domains numbered 1, 2, 3, 4, 5, 6, and 7 starting from the N terminus to the C terminus of the extracellular region, as is also the case for VEGFR2 and VEGFR3.

In one embodiment, the VEGFR1 extracellular domain may comprise an Ig-like domain D2 and an Ig-like domain D3. As used herein, the term "Ig-like domain D2" of VEGFR1 refers to the second Ig-like domain found at the N terminus of the extracellular region of VEGFR1, and the "Ig-like domain D3" of VEGFR1 refers to the third Ig-like domain found at the N terminus of the extracellular region of VEGFR1, but these terms are to encompass variants as long as their functionality (e.g., binding to VEGF ligands and/or inhibiting activation of the VEGFR pathway) is maintained. Since there may be differences in the amino acid sequence of the active protein depending on the species, the Ig-like domains D2 and D3 of VEGFR1 are not limited by their origin or sequence and may include wild type or active variants thereof.

In one embodiment, the Ig-like domain D2 of VEGFR1 may comprise the amino acid sequence of G132 to H214 of SEQ ID NO: 1 or an amino acid sequence variant having a binding affinity for a VEGF ligand similar or equivalent to that exhibited by said amino acid sequence. Specifically, in one embodiment, the Ig-like domain D2 of VEGFR1 may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence of G132 to H214 of SEQ ID NO: 1.

In one embodiment, the Ig-like domain D3 of VEGFR1 may comprise the amino acid sequence L215 to A332 of SEQ ID NO: 1 or an amino acid sequence variant having a binding affinity for a VEGF ligand similar or equivalent to that exhibited by said amino acid sequence. Specifically, in one embodiment, the Ig-like domain D3 of VEGFR1 may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence of L215 to A332.

In one embodiment, the modified fusion protein of the present disclosure may further comprise other VEGFR extracellular domains in addition to the Ig-like domain D2 and Ig-like domain D3 of VEGFR1.

While not wishing to be bound by theory, it is thought that the extracellular domain of VEGFR inhibits activation of the VEGF pathway by binding to a VEGF ligand, thereby blocking the interaction between the VEGF ligand and VEGFR. Additionally, while not wishing to be bound by theory, it is thought that the extracellular domain of VEGFR may bind to VEGFR for dominant negative inhibition of the VEGF signaling pathway. In one embodiment, the extracellular domain of VEGFR is capable of binding to one or more VEGF ligands selected from the group consisting of VEGF-A, VEGF-B, VEGF-C, VEGF-D, and PLGF. In one embodiment, the extracellular domain of VEGFR can bind to VEGFR (e.g., VEGFR1, VEGFR2 and/or VEGFR3).

In one embodiment, the VEGFR extracellular domain may or may not comprise a signal peptide which acts as a signal sequence for secretion of the VEGFR extracellular domain or a modified fusion protein comprising the same from a host cell. This signal peptide can be operably linked to a nucleic acid encoding a protein of interest (e.g., extracellular domains of VEGFR1).

### 2. Linker

Components of a modified fusion protein (e.g., extracellular domains of VEGFR1 or a multimerization domain) may be connected by a linking moiety such as a peptide linker. The linkers increase flexibility of the fusion protein components and do not significantly interfere with the structure of each functional component within the fusion protein.

In one embodiment, a linker can be used to connect the C-terminal end of VEGFR1 extracellular domains (e.g., C-terminus of the Ig-like domain D3) to the N-terminal end of a multimerization domain (e.g., IgG1 Fc region).

In one embodiment, the linker may comprise an amino acid sequence of a hinge region derived from an immunoglobulin. The hinge region derived from an immunoglobulin may include a hinge region present in the N-terminal region of the Fc region of the immunoglobulin. In one embodiment, the hinge region derived from an immunoglobulin may be derived from human IgA, IgD, IgM, IgE, or IgG. In one embodiment, the hinge region derived from an immunoglobulin may comprise an amino acid sequence extending up to the core hinge region of the Fc region of the immunoglobulin, which sequence does not include the lower hinge region of the Fc region. For example, if the hinge region is from IgG1, the lower hinge region may be an amino acid sequence corresponding to APELLGGP. In one embodiment, the linker may have the amino acid sequence EDKTHTCPPCP or LEDKTHTCPPCP.

### 3. Multimerization domain

The present disclosure provides a multimerization domain (e.g., the Fc region of an immunoglobulin) that can be a component of any modified fusion protein. A multimerization domain is a portion of a multimeric protein that promotes the association of subunits to form, for example, a dimer, a trimer, a tetramer, and the like. As used herein, the term "multimerization domain" may be used to refer to a dimerization domain, trimerization domain, tetramerization domain, and the like. A fusion protein comprising a multimerization domain can interact with another fusion protein comprising a multimerization domain to generate a fusion protein multimer (e.g., a fusion protein dimer). For example, an IgG Fc region is a dimerization domain that can be fused with the extracellular domains of VEGFR1 described herein. A fusion protein comprising extracellular domains of VEGFR1 and an IgG Fc region can dimerize with another fusion protein comprising an IgG Fc region, creating a fusion protein dimer that can simultaneously bind to a VEGF ligand and a PLGF ligand.

In one embodiment, the multimerization domain can be the Fc region of an immunoglobulin. In a further embodiment, the Fc region of an immunoglobulin may be selected from the group consisting of an IgG Fc region, an IgA Fc region, an IgM Fc region, an IgD Fc region, and an IgE Fc region. In yet a further embodiment, the Fc region of an immunoglobulin may be selected from the group consisting of an IgG1 Fc region, an IgG2 Fc region, an IgG3 Fc region, and an IgG4 Fc region. In one embodiment, the Fc region may comprise the CH3 region of IgG1, IgG2, IgG3, or IgG4. In one embodiment, the Fc region may include the CH2 and CH3 regions of IgG1, IgG2, IgG3, or IgG4. Amino acid sequences encoding immunoglobulins comprising Fc regions are well known in the art. In one embodiment, the multimerization domain may be the Fc region of an immunoglobulin comprising the amino acid sequence of SEQ ID NO:99. In one embodiment, the multimerization domain may be the Fc region of IgG1 consisting of the amino acid sequence of SEQ ID NO: 99.

In one embodiment, the Fc region may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence of the CH3 region or CH2 and CH3 regions of IgG1, IgG2, IgG3, or IgG4. In one embodiment, the multimerization domain may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence of SEQ ID NO: 99.

In one embodiment, the multimerization domain may start from the lower hinge region of an immunoglobulin Fc region. The lower hinge region of the Fc region may start from Ala244 and may correspond to the amino acid sequence of Ala244 to Pro251. Herein, the positions of amino acid residues follow the Kabat numbering scheme (Kabat et. al., in of Proteins of Immunological Interest 5th Ed., US Department of Health and Human Services, NIH Publication No. 91-3242, 1991). For example, the lower hinge region of the human IgG1 Fc region may correspond to amino acid residues 1 to 8 based on the amino acid sequence of SEQ ID NO: 99 or correspond to the amino acid sequence of APELLGGP. The human IgG1 Fc region starting from the lower hinge region is presented as the amino acid sequence of SEQ ID NO: 99.

In one embodiment, the Fc region may be partially glycosylated or not glycosylated at all.

In one embodiment, the Fc region may have amino acid substitution D126E, amino acid substitution L128M, and/or amino acid deletion of K217 based on SEQ ID NO:99. These amino acid variations can increase the half-life of the fusion protein through interaction with FcRn. Said amino acid variations may appear identically in other immunoglobulin Fc regions having a sequence corresponding to the amino acid sequence of SEQ ID NO: 99, and the modified fusion protein of the present disclosure may comprise another immunoglobulin Fc region having the amino acid variations as the multimerization domain component.

Below, characteristics of a modified fusion protein according to the present disclosure are described. In amino acid variations for these characteristics, the positions of amino acid residues are indicated to correspond to the amino acid residue positions according to the entire amino acid sequence of VEGFR1 in SEQ ID NO: 1.

### 4. Characteristics of Surface Charge Transition Variations

In one embodiment, a modified fusion protein of the present disclosure may have surface charge transition variations as a characteristic. In the present disclosure, modified fusion proteins having the characteristics of surface charge transition variations characteristic may be used interchangeably as "surface charge transition variants" or "surface charge variants."

In the characteristics of surface charge transition variations according to one embodiment, a modified fusion protein may have one or more amino acid residues on the β1-β2 loop of Ig-like domain D3, one or more amino acid residues on the β2-β3 loop of domain D3, and/or one or more amino acid residues on the β5-β6 loop of domain D3 substituted with an amino acid that reduces the net pI of a protein, an amino acid having a negatively charged side chain, or an amino acid having an electronegative side chain. In one embodiment, an amino acid having an electronegative side chain may mean that the amino acid turns electronegative depending on the nature of adjacent amino acid residues or molecules after substitution, and may refer to a relative concept arising from the electron distribution within the molecule. For example, amino acid residues containing a hydroxy group (-OH) can turn electronegative due to the lone pair of electrons present on the oxygen atom, and this phenomenon can reduce the net pI of the protein.

In one embodiment, the β1-β2 loop of Ig-like domain D3 may comprise amino acid residues T236 to T247 of the amino acid sequence of SEQ ID NO: 1. In one embodiment, the β2-β3 loop of Ig-like domain D3 may comprise amino acid residues T256 to V262 of the amino acid sequence of SEQ ID NO: 1. In one embodiment, the β5-β6 loop of Ig-like domain D3 may comprise amino acid residues D299 to L308 of the amino acid sequence of SEQ ID NO: 1. In the present disclosure, the β1-β2 loop may be referred to as "Site 1," the β2-β3 loop may be referred to as "Site 2," and the β5-β6 loop may be referred to as "Site 3." The inventors of the present disclosure ascertained that amino acid residues present in said loops are exposed on the surface of domain D3, and have achieved surface charge stabilization and structure stabilization of modified fusion proteins through variations of amino acid residues present in the loops.

In one embodiment, the amino acid residues present in Site 1 and Site 3 affect each other through interactions between amino acid residues due to their structural positions, and accordingly, double, triple or quadruple amino acid variation (e.g. amino acid substitutions) may be introduced at each of the Sites.

In one embodiment, amino acid residues of Site 2 are exposed on the domain surface on the side where domain D3 binds to the VEGF ligand or PLGF ligand, and may affect the binding affinity to the ligands or solubility due to surface exposure of residues. In one embodiment, amino acid residues on Site 2 may be substituted with an amino acid residue having a smaller side chain than the side chain of the existing amino acid residue (e.g., Ala) or an amino acid residue having a polar side chain of a similar size to the side chain of the existing amino acid residue (e.g., Ser or Asp) as long as the protein structure or binding affinity to the ligands and solubility is not significantly affected.

In one embodiment, the three loops should be understood to encompass amino acid sequence variants that maintain their function. For example, in one embodiment, the β1-β2 loop, β2-β3 loop and β5-β6 loop may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequences TPRPVKLLRGHT, TPLNTRV and DKMQNKDKGL, respectively.

In the characteristics of surface charge transition variations according to one embodiment, the amino acid substitution may be a substitution with (i) an amino acid residue of the VEGFR2 homologous sequence, (ii) an amino acid residue that is serine, threonine, tyrosine, cysteine, asparagine, glutamine, aspartic acid, or glutamic acid, or (iii) an amino acid residue having a short side chain which is alanine or glycine, but is not limited thereto.

In the linker variation characteristic according to one embodiment, a modified fusion protein may have one or more amino acid residues on a β1-β2 loop of Ig-like domain D3 and one or more amino acid residues on a β5-β6 loop of domain D3 substituted with an amino acid that reduces the net pI of a protein, an amino acid having a negatively charged side chain, or an amino acid having an electronegative side chain.

In the characteristics of surface charge transition variations according to one embodiment, a modified fusion protein may comprise amino acid substitution of (i) one or more of the amino acid residues K241, L243, R244, and H246 on the β1-β2 loop of Ig-like domain D3, but is not limited thereto. In one embodiment, the amino acid residues on the β1-β2 loop may be substituted with any one of threonine, glutamic acid, serine, and valine.

In the characteristics of surface charge transition variations according to one embodiment, a modified fusion protein may comprise amino acid substitution of (ii) amino acid residue L258 on the β2-β3 loop of Ig-like domain D3, but is not limited thereto. In one embodiment, the amino acid residues on the β2-β3 loop may be substituted with any one of alanine, serine, and aspartic acid.

In the characteristics of surface charge transition variations according to one embodiment, a modified fusion protein may comprise amino acid substitution of (iii) one or more of the amino acid residues K300, Q302, K304, and K306 on the β5-β6 loop of domain D3, but is not limited thereto. In one embodiment, the amino acid residues on the β5-β6 loop may be substituted with any one of glycine, threonine, serine, and glutamine.

In the characteristics of surface charge transition variations according to one embodiment, a modified fusion protein may comprise (i) one or more amino acid substitutions selected from K241T, K241E, L243S, R244V, and H246E on the β1-β2 loop of domain D3, (ii) amino acid substitution L258A, L258S, or L258D on the β2-β3 loop of domain D3, and/or (iii) one or more amino acid substitutions selected from K300G, Q302T, K304S, and K306Q on the β5-β6 loop of domain D3.

In the characteristics of surface charge transition variations according to one embodiment, a modified fusion protein may comprise (i) amino acid substitutions L243S and R244V, or (ii) amino acid substitutions K241E, L243S, R244V, and H246E on the β1-β2 loop of domain D3; amino acid substitution L258A, L258S, or L258D on the β2-β3 loop of domain D3; and/or (i) amino acid substitution K300G, (ii) amino acid substitutions K300G, Q302T, and K304S, or (iii) amino acid substitutions K300G, Q302T, K304S, and K306Q on the β5-β6 loop of domain D3.

In the characteristics of surface charge transition variations according to one embodiment, a modified fusion protein may comprise amino acid substitutions K241E, L243S, R244V, and H246E on the β1-β2 loop of domain D3; amino acid substitution L258A, L258S, or L258D on the β2-β3 loop of domain D3; and/or amino acid substitutions K300G, Q302T, and K304S on the β5-β6 loop of domain D3.

In one embodiment, the modified fusion protein may comprise amino acid substitutions R238S and/or R275N in Ig-like domain D3.

In one embodiment, the modified fusion protein may be such that the amino acid sequence of the C terminus of Ig-like domain D3 connected to the linker is KALE based on the amino acid residue K331, provided that one or more of the amino acids K, A, L and E may be deleted. It could be possible. In one embodiment, the C terminus of Ig-like domain D3 of the modified fusion protein may have K and A deleted; K, A and L deleted; A, L and E deleted; or K, A, L and E deleted. In one embodiment, the C-terminal amino acid of the Ig-like domain D3 of a modified fusion protein of the present disclosure may be D330.

In one embodiment, the N terminus of Ig-like domain D2 of a modified fusion protein may be further modified. A modified fusion protein of the present disclosure can be produced by inserting the nucleic acid sequence or nucleic acid molecule encoding the same into vectors of various origins. In this case, depending on the type or origin of the vector into which the nucleic acid sequence or nucleic acid molecule is inserted, variations may occur in the amino acid sequence at the N terminus the Ig-like domain D2, with the N terminus starting from an amino acid sequence derived from the vector (e.g., amino acid sequence EF) rather than beginning with G132. Additionally, in one embodiment, a modified fusion protein of the present disclosure may further comprise a sequence from the C terminus of Ig-like domain D1 of VEGFR1 at the N terminus of the Ig-like domain D2. In one embodiment, the N terminus of Ig-like domain D2 of a modified fusion protein may begin with the amino acid sequence SDT or the amino acid sequence EF. However, such variation of the N-terminal sequence by a vector-derived amino acid sequence may not affect the productivity or physicochemical properties of a modified fusion protein of the present disclosure.

### 5. Linker variation characteristic

In one embodiment, a modified fusion protein of the present disclosure may have linker variations as a characteristic. In the present disclosure, modified fusion proteins having the linker variation characteristic may be used interchangeably as "linker variants." Due to the linker variation characteristic, (i) a modified fusion protein of the present disclosure can closely mimic the structure in which domain D2 and domain D3 among the native VEGFR1 extracellular domains bind to a ligand, (ii) domain D3 among the native VEGFR1 extracellular domains can have a length spanning to the dimer formation site of domain D4 as a structural characteristic to be secured for smooth ligand binding, and (iii) the linker itself can have excellent structural stability.

In the linker variation characteristic according to one embodiment, a linker may have a length of about 14 amino acids or more. In one embodiment, the linker may have a length of about 14 to about 70 amino acids, about 14 to about 65 amino acids, about 14 to about 60 amino acids, about 14 to about 55 amino acids, about 14 to about 50 amino acids, about 14 to about 45 amino acids, about 14 to about 40 amino acids, about 14 to about 38 amino acids, or about 14 to about 36 amino acids. In one embodiment, the linker may comprise or consist of 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 56, 57, 58, 59, 60, 61, 62, 63, 64, 65, or 70 (inclusive, any value between these numbers included) amino acids.

In the linker variation characteristic according to one embodiment, a linker may comprise a GS repeat sequence. In one embodiment, the GS repeat sequence may comprise a 2 to 60 amino acids long amino acid sequence consisting only of G and S. The N-terminal amino acid of the GS repeat sequence may be glutamic acid. In one embodiment, the GS repeat sequence may comprise or consist of 2, 3, 4, 5, 6, 7, 8, 9, 10, 11, 12, 13, 14, 15, 16, 17, 18, 19, 20, 25, 30, 35, 40, 45, 50, 55, or 60 (inclusive, any value between these numbers included) amino acids. In one embodiment, the GS repeat sequence may comprise or consist of 2 to 20 amino acids.

In the linker variation characteristic according to one embodiment, the GS repeat sequence may be (GS)ₙ, (GSSG)ₙ, (GGGGS)ₙ, or (GS)ₘ(GGGGS)ₙ, where n may be an integer of 1 to 10, and m may be an integer of 0 to 10.

In the linker variation characteristic according to one embodiment, the GS repeat sequence may be selected from the group consisting of GS, GSSG, (GSSG)₂, GGGGS, (GGGGS)₄, GS(GGGGS), and GS(GGGGS)₃, but is not limited thereto.

In the linker variation characteristic according to one embodiment, a linker may comprise an amino acid sequence of a hinge region derived from an immunoglobulin. In one embodiment, the amino acid sequence of the hinge region may be modified.

In the present disclosure, a linker is located between the C-terminus of VEGFR1 domain D3 and the Fc region of a human immunoglobulin produced by papain digestion and may refer to the entire region extending between P243 located at the N-terminus of the CH2 region (A244-K360) and the C-terminus of domain D3. The positions of amino acid residues in the Fc region and the CH2 region included in the Fc region follow the Kabat numbering scheme (Kabat et. al., in of Proteins of Immunological Interest 5th Ed., US Department of Health and Human Services, NIH Publication No. 91-3242, 1991). In one embodiment, the linker may include some region derived from a human immunoglobulin, and the region may be the amino acid sequence of CH1 (K218-V223) or the hinge region (E226-P243), or a portion of such amino acid sequence.

In the present disclosure, when a linker comprises the amino acid sequence of a hinge region derived from immunoglobulin, characteristics of the included hinge region are retained in the linker. For example, the hinge region of an Fc region has cleavage sites for various proteases and can cause oligomerization after being introduced into a fusion protein, through O-glycosylation at Ser/Thr amino acids (Song et. al., 2020. Comput Struct Biotechnol J. 18:3925-3935). Such glycosylation in the hinge region can affect physicochemical properties of proteins during expression or purification and can cause structural interference in the pairing of disulfide bonds in the hinge region, which occurs reversibly during the purification process, thus interrupting normal disulfide bond formation. In a modified fusion protein having the linker variation characteristic of the present disclosure, the amino acid sequence of the hinge region derived from an immunoglobulin was modified to achieve excellent structural stability of the linker itself and improve physicochemical properties.

In the linker variation characteristic according to one embodiment, the hinge region derived from an immunoglobulin may comprise a sequence derived from the CH1 region, an upper hinge, and/or a core hinge. In one embodiment, some amino acid sequence derived from the CH1 region of an immunoglobulin may form a beta-strand or beta-sheet. In one embodiment, the hinge region derived from an immunoglobulin may be derived from human IgA, IgD, IgM, IgE, IgG, or a combination thereof.

In the linker variation characteristic according to one embodiment, a modified fusion protein may have an amino acid variation in a papain recognition site or a glycosylation site present in the hinge region derived from an immunoglobulin included in the linker.

In the linker variation characteristic according to one embodiment, the amino acid variation in the papain recognition site may be substitution of one or more amino acid residues present in the papain recognition site with alanine, serine, tyrosine, proline, or threonine, or insertion of a 1 to 10 amino acids long amino acid sequence comprising at least one amino acid having an aromatic or cyclic carbon in the side chain into the papain recognition site.

In one embodiment, the hinge region included in the linker may be derived from IgG1. In this case, the amino acid sequence near the cleaved amino acids in the papain recognition site may be KTHT. In one embodiment, when the hinge region is derived from IgG1, histidine corresponding to the cleaved amino acid in the papain recognition site may be substituted with proline, proline-proline, or tyrosine.

In one embodiment, when the hinge region is derived from IgG1, a 1 to 10 amino acids long amino acid sequence comprising at least one amino acid having an aromatic or cyclic carbon in the side chain may be inserted before the histidine corresponding to the cleaved amino acid. In one embodiment, the amino acid having an aromatic or cyclic carbon in the side chain may be proline or tyrosine. In one embodiment, the sequence inserted before the cleaved amino acid may include cysteine. If the sequence inserted into the papain recognition site contains cysteine, it can form a disulfide bond with the cysteine located in the same site of the opposing monomer when forming a dimer, thereby imparting rigidity to the linker, leading to stabilization of the loop structure of the linker. In one embodiment, the amino acid sequence inserted before the cleaved amino acid may be the sequence PPTC but is not limited thereto. In the sequence inserted into the papain recognition site, a hydrophobic interaction may arise between aromatic carbons or cyclic carbons due to the amino acid having an aromatic or cyclic carbon in the side chain, and the interaction may stabilize the loop structure of the linker.

In one embodiment, when the hinge region is derived from IgG1, the amino acid sequence KTHT of the papain recognition site having a variation may have been modified into an amino acid sequence selected from the group consisting of KTYT, TPP, ATPT, STYT, KAPP, and ATPPTCP.

In the linker variation characteristic according to one embodiment, the amino acid variation in the glycosylation site may be deletion of serine or threonine present in the hinge region derived from an immunoglobulin or substitution of serine or threonine present in the hinge region derived from an immunoglobulin with an amino acid other than serine or threonine. In one embodiment, the amino acid other than serine or threonine may be glycine or alanine. Potential O-glycosylation in the hinge region can be prevented through amino acid substitution of serine or threonine.

In the linker variation characteristic according to one embodiment, cysteine present in the core hinge may be substituted with serine or glycine. In this case, the disulfide bond present in the core hinge can be removed to increase the flexibility of the linker, and the same effect as achievable by increasing the length of the linker can be expected.

In the linker variation characteristic according to one embodiment, a linker may comprise (i) an amino acid sequence selected from the group consisting of GS, GSSG, (GSSG)₂, GGGGS, (GGGGS)₄, GS(GGGGS) and GS(GGGGS)₃; and (ii) a hinge region derived from an immunoglobulin selected from the group consisting of human IgG1, IgG4, or IgD/G1, in that order starting from the N terminus, and the amino acid sequence of the hinge region may be modified.

In the present disclosure, a hinge region derived from IgD/G1 may refer to a combination of the hinge sequences of IgD and IgG1. Specifically, a hinge region derived from IgD/G1 may be NTGSGGEEKKKEKEKEEQEERSSDKTHTCPPCP. In one embodiment, the hinge sequence of IgD may be NTGSGGEEKKKEKEKEEQEERSS, NTGRGGEEKKKEKEKEEQEER, or a variant thereof. In one embodiment, the hinge sequence of IgG1 may be DKTHTCPPCP or a variant thereof.

In the linker variation characteristic according to one embodiment, a linker may be an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP.

### 6. Disulfide bond variation characteristic

In one embodiment, a modified fusion proteins of the present disclosure may have disulfide bond variations as a characteristic. In the present disclosure, modified fusion proteins having the disulfide bond variation characteristic may be used interchangeably as "disulfide bond variants." Since a modified fusion protein of the present disclosure is a fusion of heterologous proteins, stability is expected to be reduced at the site where different types of proteins are connected. Accordingly, the disulfide bond variation characteristic was introduced to prevent physical cleavage of the modified fusion protein and improve its stability.

To introduce the disulfide bond variation characteristic, positions of amino acid residues where disulfide bond variations may be introduced were searched, centering on the C-terminal region of the Ig-like domain D3 where the linkage of heterologous proteins in the fusion protein begins and secondary structures may be lacking, according to the following criteria: (1) the candidate amino acid residue should be exposed on the surface of the fusion protein so that they can form a disulfide bond, (2) they should be present on a flexible loop-linker where the heterologous proteins are connected, and (3) amino acid residues located in two different sites must be within a distance at which they can form a disulfide bond. Based on these criteria, candidate amino acid residues were selected using a tertiary structure energy prediction program (FoldX in YASARA, Schymkowitz et al.2005 Nucleic Acids Research. 33: W382-388), and these amino acid residues were substituted with cysteine to ascertain that the formation of a disulfide bond leads to improved properties (e.g., productivity, physicochemical properties, ligand binding) of modified fusion proteins (*see* Example 2.3). In one embodiment, the disulfide bond variation characteristic can be introduced through substitution with a cysteine residue at one position in a VEGFR1 domain and one position in the linker, thereby achieving excellent stability of a fusion protein modified by a covalent bond, one of the stable bonding forms.

In one embodiment, for the disulfide bond variation characteristic, (i) one of the amino acid residues on the β1-β2 loop of Ig-like domain D3 or one of the amino acid residues on the β5-β6 loop of Ig-like domain D3, and (ii) one of the amino acid residues at positions -2, -1, 0, +1, +2, and +3 relative to Y329 of Ig-like domain D3 may be substituted with cysteine. By such substitutions, a modified fusion protein of the present disclosure may have a disulfide bond within the protein. In one embodiment, the amino acid residues present at the positions described in (i) and (ii) above for introduction of the disulfide bond variation characteristic may be selected according to the above criteria (1) to (3).

In one embodiment, the one of the amino acid residues on the β1-β2 loop in (i) may be one of the amino acid residues on the amino acid sequence of V240 to T247.

In one embodiment, for the disulfide bond variation characteristic, one of amino acid residues T241, L243, and R244 on the β1-β2 loop of Ig-like domain D3 or amino acid residue N303 on the β5-β6 loop of Ig-like domain D3 may be substituted with cysteine, and one of the amino acid residues at positions 0, +2, and +3 relative to Y329 of Ig-like domain D3 may be substituted with cysteine.

In one embodiment, for the disulfide bond variation characteristic, a modified fusion protein of the present disclosure may have T241 and the amino acid residue at position +3 relative to Y329 of domain D3 substituted with cysteine; have amino acid variations L243C and Y329C; have L243 and the amino acid residue at position +2 relative to Y329 of domain D3 substituted with cysteine; have amino acid variations R244C and Y329C; or have amino acid variations N303C and Y329C.

### 7. Fusion protein

In one embodiment of the present disclosure, a modified fusion protein capable of simultaneously binding a VEGF ligand and a PLGF ligand can be provided. In one embodiment, the modified fusion protein can have a first binding specificity for a VEGF ligand and a second binding specificity for a PLGF ligand. Here, the VEGF ligand may be one or more of VEGF-A, VEGF-B, VEGF-C, and VEGF-D, and may specifically be VEGF-A.

In one embodiment, the modified fusion protein may comprise a VEGFR1 extracellular domain, a linker, and a multimerization domain. In one embodiment, the modified fusion protein may comprise a VEGFR1 extracellular domain, a linker, and a multimerization domain, as components arranged in that order from the N terminus to the C terminus. In one embodiment, the VEGFR1 extracellular domain may comprise Ig-like domain D2 and Ig-like domain D3 of VEGFR1.

In one embodiment, a modified fusion protein may have one or more of (a) surface charge transition characteristic, (b) linker variation characteristic, and (c) disulfide bond variation characteristic.

In one embodiment, the modified fusion protein may have all of the following characteristics (a), (b), and (c):
(a) one or more amino acid substitutions selected from K241T, K241E, L243S, R244V, and H246E on the β1-β2 loop of domain D3, amino acid substitution L258A, L258S, or L258D on the β2-β3 loop of domain D3, and/or one or more amino acid substitutions selected from K300G, Q302T, K304S, and K306Q on the β5-β6 loop of domain D3 are introduced;
(b) the linker is an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP;
(c) one of amino acid residues T241, L243, and R244 on the β1-β2 loop of domain D3 or amino acid residue N303 on the β5-β6 loop is substituted with cysteine, and one of the amino acid residues at positions 0, +2, and +3 relative to Y329 of domain D3 is substituted with cysteine.

In one embodiment, the modified fusion protein may have all of the following characteristics (a), (b), and (c):
(a) (i) amino acid substitutions L243S and R244V, or (ii) amino acid substitutions K241E, L243S, R244V, and H246E on the β1-β2 loop of domain D3, amino acid substitution L258A, L258S, or L258D on the β2-β3 loop of domain D3, and/or (i) amino acid substitution K300G, (ii) amino acid substitutions K300G, Q302T, and K304S, or (iii) amino acid substitutions K300G, Q302T, K304S, and K306Q on the β5-β6 loop of domain D3 are introduced;
(b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP;
(c) L243 and the amino acid residue at position +2 relative to Y329 of domain D3 are substituted with cysteine.

In one embodiment, the modified fusion protein may have all of the following characteristics (a), (b), and (c):
(a) amino acid substitutions K241E, L243S, R244V, and H246E on the β1-β2 loop of domain D3, amino acid substitution L258A, L258S, or L258D on the β2-β3 loop of domain D3, and/or amino acid substitutions K300G, Q302T, and K304S on the β5-β6 loop of domain D3 are introduced;
(b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP;
(c) L243 and the amino acid residue at position +2 relative to Y329 of domain D3 are substituted with cysteine.

In one embodiment, the modified fusion protein may comprise an amino acid sequence selected from the group consisting of SEQ ID NO: 10 to SEQ ID NO: 70, SEQ ID NO: 77 to SEQ ID NO: 93, and SEQ ID NO: 98. In one embodiment, the modified fusion protein may consist of an amino acid sequence selected from the group consisting of SEQ ID NO: 10 to SEQ ID NO: 70, SEQ ID NO: 77 to SEQ ID NO: 93, and SEQ ID NO: 98.

In one embodiment, the modified fusion protein may have at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to an amino acid sequence selected from the group consisting of SEQ ID NO: 10 to SEQ ID NO: 70, SEQ ID NO: 77 to SEQ ID NO: 93, and SEQ ID NO: 98. Such variants may comprise amino acid variations (e.g., conservative amino acid substitutions) in additional amino acid sequences while retaining or maintaining the amino acid variations defined in the characteristics of a modified fusion protein of the present disclosure.

In one embodiment, the components of a modified fusion protein of the present disclosure (a VEGFR1 extracellular domain, a linker, and a multimerization domain) may carry post-translational modifications including, for example, glycosylation, sialylation, acetylation, and phosphorylation.

In one embodiment, a modified fusion proteins of the present disclosure may contain conservative amino acid substitutions at positions other than those of the amino acid variations defined in the characteristics described in the present disclosure. These conservative amino acid substitutions can be introduced into any of the individual components of a modified fusion protein of the present disclosure (e.g., a VEGFR1 extracellular domain, a linker, and a multimerization domain).

In one embodiment, a modified fusion protein of the present disclosure may comprise a signal peptide or signal sequence for secretion of the protein from a cell. For example, a modified fusion protein of the present disclosure may further comprise a peptide of heterologous origin, specifically, a signal sequence or another peptide having a specific cleavage site at the N terminus of the mature fusion protein. In one embodiment, the heterologous signal sequence may be one that is recognized and processed (i.e., cleaved by a signal peptide hydrolase) by a eukaryotic host cell. Information regarding signal peptides is well known in the field and is presented, for example, in Korean Patent No. 10-2228921, but is not limited thereto. In one embodiment, the signal peptide may be a human interleukin signal sequence. In one embodiment, the signal peptide may consist of the amino acid sequence MVSYWDTGVLLCALLSCLLLTGSSSG (tPA), MEFGLSWVFLVALFRGVQC (H7), MKWVTFISLLFLFSSAYS (human serum albumin), MGWSCIILFLVATATGVHS (mouse Ig heavy chain), MDWTWRVFCLLAVAPGAHS (human Ig heavy chain), or MYRMQLLSCIALSLALVTNS (human interleukin-2), but any signal peptide that a person of ordinary skill in the art can use for the production of a fusion protein may be used without limitation.

In one embodiment of the present disclosure, a multimeric fusion protein comprising two or more modified fusion proteins of the present disclosure can be provided. Multimers (e.g., dimers, trimers, tetramers, etc.) may be formed from the same fusion protein (e.g., homomultimers) or may form fusion proteins of heterologous origin (e.g., heteromultimers). In one embodiment, the multimeric fusion protein may comprise at least one modified fusion protein comprising an amino acid sequence selected from the group consisting of SEQ ID NO: 10 to SEQ ID NO: 70, SEQ ID NO: 77 to SEQ ID NO: 93, and SEQ ID NO: 98 or an amino acid sequence having at least 90% sequence identity to an amino acid sequence selected from the group consisting of SEQ ID NO: 10 to SEQ ID NO: 70, SEQ ID NO: 77 to SEQ ID NO: 93, and SEQ ID NO: 98. In one embodiment, such fusion proteins are recovered as protein fusion multimers from host cells containing nucleic acids encoding a modified fusion protein of the present disclosure. In one embodiment, such multimeric fusion proteins can be glycosylated. For example, such multimeric fusion proteins may be glycosylated at an extracellular domain of VEGFR and/or a multimerization domain after release from a host cell.

In one embodiment, a modified fusion protein of the present disclosure may have an IC₅₀ of less than or equal to about 1 mg/ml, 500 ng/ml, 300 ng/ml, 100 ng/ml 70 ng/ml, 50 ng/ml, 45 ng/ml, 40 ng/ml, 35 ng/ml, 30 ng/ml, 25 ng/ml, 20 ng/ml, 10 ng/ml, 5 ng/ml, or 1 ng/ml (inclusive, any value between these numbers included) for inhibition of ligand activity (e.g., inhibition of VEGF activity or PLGF activity).

In one embodiment, a modified fusion protein of the present disclosure may have a Kd that is less than any one of 1.0 mM, 500 µM, 100 µM, 50 µM, 25 µM, 10 µM, 5 µM, 1 µM, 900 nM, 800 nM, 700 nM, 600 nM, 500 nM, 400 nM, 350 nM, 300 nM, 250 nM, 200 nM, 150 nM, 100 nM, 95 nM, 90 nM, 85 nM, 80 nM, 75 nM, 70 nM, 65 nM, 60 nM, 55 nM, 50 nM, 45 nM, 40 nM, 35 nM, 30 nM, 25 nM, 20 nM, 15 nM, 10 nM, 5 nM, 1 nM, 900 pM, 800 pM, 700 pM, 600 pM, 500 pM, 400 pM, 300 pM, 200 pM, 100 pM, 50 pM, 25 pM, 12.5 pM, 6.25 pM, 5 pM, 4 pM, 3 pM, 1 pM, 0.5 pM, 0.1 pM, 0.05 pM, or 0.01 pM (inclusive, including any values between these numbers).

### III. Nucleic acids, vectors, and host cells

### Nucleic acids

In one embodiment of the present disclosure, an isolated nucleic acid encoding any modified fusion protein component of the present disclosure, such as a VEGFR1 extracellular domain and/or a multimerization domain, can be provided. Nucleic acids encoding mammalian VEGFRs have been described for all receptor types. Exemplary nucleic acid sequences can be found in US Patent No. 7,928,072 and WO 2006/113277 but are not limited thereto. mRNAs encoding human VEGFR1 and VEGFR2 can be found under GenBank accession numbers NM_002019.4 and NM_002253.2, respectively. In one embodiment, an isolated nucleic acid may encode Ig-like domains D2 and D3 of VEGFR1 comprising the amino acid sequence consisting of SEQ ID NO:100. In one embodiment, an isolated nucleic acid may encode Ig-like domains D2 and D3 of VEGFR1 comprising an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence consisting of SEQ ID NO:100.

Isolated nucleic acids encoding a multimerization domain (e.g., an Fc region) can be provided in the present disclosure. In one embodiment, an isolated nucleic acid may encode a multimerization domain comprising the amino acid sequence of SEQ ID NO:99. In some embodiments, an isolated nucleic acid may encode a multimerization domain comprising an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequence selected from the group consisting of SEQ ID NO:99.

Isolated nucleic acids encoding a modified fusion protein of the present disclosure can be provided. In one embodiment, an isolated nucleic acid may encode a modified fusion protein comprising an amino acid sequence selected from the group consisting of SEQ ID NO: 10 to SEQ ID NO: 70, SEQ ID NO: 77 to SEQ ID NO: 93, and SEQ ID NO: 98. In one embodiment, an isolated nucleic acid may encode a modified fusion protein comprising an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to an amino acid sequence selected from the group consisting of SEQ ID NO: 10 to SEQ ID NO: 70, SEQ ID NO: 77 to SEQ ID NO: 93, and SEQ ID NO: 98.

An isolated nucleic acid sequence encoding a modified fusion protein of the present disclosure or a component of the fusion protein (e.g., an extracellular domain of VEGFR1 or a multimerization domain) may further comprise a nucleic acid sequence encoding a linker.

In one embodiment, an isolated nucleic acid may further comprise a sequence encoding a signal peptide which serves as a signal sequence for secretion of a modified fusion protein from a host cell. In one embodiment, an isolated nucleic acid may not include a sequence encoding a signal peptide.

An isolated nucleic acid molecule encoding a modified fusion protein of the present disclosure or a component of the fusion protein (e.g., an extracellular domain of VEGFR1, a linker, or a multimerization domain) may be in the form of RNA, e.g., mRNA, hnRNA, tRNA, or any other form, or in the form of DNA including, but not limited to, cDNA and genomic DNA obtained by cloning or produced synthetically, or any combination thereof. Such isolated nucleic acid molecules can be prepared by various methods known in the art (*see* Molecular Cloning: A Laboratory Manual (Sambrook et al., 4thed., Cold Spring Harbor Laboratory Press, Cold Spring Harbor, NY, 2012 ) and Current Protocols in Molecular Biology (F.M. Ausubel, et al. eds., 2003).

### Vectors

In one embodiment, the present disclosure relates to a nucleic acid delivery vehicle or use thereof for introducing one or more nucleic acid sequences encoding a modified fusion protein or fusion protein component into a cell for expression of the protein. The nucleic acid sequence may be the sequence of the isolated nucleic acid described above.

In one embodiment, examples of nucleic acid delivery vehicles include liposomes, biocompatible polymers (including natural and synthetic polymers); lipoproteins; polypeptides; polysaccharides; lipopolysaccharide; artificial viral envelopes; metal particles; and bacteria, viruses such as baculoviruses, adenoviruses and retroviruses, bacteriophages, cosmids, plasmids, fungal vectors and other recombinant vehicles commonly used in the art which have been described for expression in various eukaryotic and prokaryotic hosts. In one embodiment, the nucleic acid delivery vehicle may be an expression vector such as a plasmid. In one embodiment, the nucleic acid sequence included in an expression vector may be operably linked to an expression control sequence.

In one embodiment, the vector may include any element to establish a conventional function of an expression vector, such as a promoter, ribosome binding element, terminator, enhancer, selection marker, and an origin of replication. The promoter can be a constitutive, inducible or repressible promoter. An exemplary promoter is provided in Korean Patent No. 10-0659477 but is not limited thereto.

A number of expression vectors capable of delivering nucleic acids to a cell (e.g., derived from bacterial cell, yeast cell, plant cell, or mammalian cell) are known in the art and may be used herein for production of a modified fusion protein or fusion protein component in the cell. For example, *E. coli* can be used to produce a modified fusion protein if transformed with a plasmid, such as pBR322 (Mandel et al., J. Mol. Biol., 1970, 53:154), engineered to contain a nucleic acid encoding the fusion protein. Expressed modified fusion proteins or fusion protein components can be harvested from the cells and purified according to conventional techniques known in the art and as described in this disclosure.

In one embodiment, an expression vector comprising a nucleic acid encoding a modified fusion protein described above and capable of being replicated in a bacterial or eukaryotic host can be used to transfect the host and thereby direct expression of the nucleic acid to produce the modified fusion protein which may then be recovered in a biologically active form. As used herein, a biologically active form includes a form capable of binding a VEGF ligand or a PLGF ligand.

In one embodiment, an expression vector containing a nucleic acid molecule encoding a modified fusion protein or fusion protein component can be identified by, without limitation, at least three general methods: (a) DNA-DNA hybridization, (b) presence or absence of "marker" gene functions, and (c) expression of the inserted sequence. Details of the above three methods are provided in Korean Patent Nos. 10-0659477 or 10-2228921, but are not limited thereto.

### Host cells

In one embodiment of the present disclosure, a host cell containing the expression vector encoding a modified fusion protein of the present disclosure can be provided. Such host cells are suitable for expression of a modified fusion protein of the present disclosure and may correspond to a host-vector system for producing the modified fusion protein. In one embodiment, host cells can be used to produce viral particles (e.g., recombinant viral vectors).

As used herein, the term "host cell" may include a cell which has been or can be a recipient for a vector(s) of this disclosure and progeny thereof. The progeny may not necessarily be completely identical (in morphology or in genomic of total DNA complement) to the original parent cell due to natural, accidental, or deliberate mutation. In one embodiment, the host cell may be a bacterial cell such as *E. coli,* a yeast cell such as *Pichia pastoris,* an insect cell such as *Spodoptera frugiperda,* or a mammalian cell such as COS, HEK or CHO cell, but is not limited thereto.

In one embodiment, a modified fusion protein of the present disclosure may be expressed in a host cell transiently, constitutively or permanently.

In one embodiment of the present disclosure, a method of producing a modified fusion protein of the present disclosure can be provided which comprises growing host cells or cells of a host-vector system described above under conditions permitting production of the modified fusion protein and then recovering the fusion protein so produced. Modified fusion proteins useful for practicing the present disclosure can be produced by expression in a prokaryotic or eukaryotic expression system. Methods for culturing host cells or producing a fusion protein from host cells are well known in the field and are disclosed, for example, in Korean Patent No. 10-2228921, but are not limited thereto.

In one embodiment, a modified fusion protein of the present disclosure produced from the host cells described above can be purified and identified by a variety of methods. Methods for purifying and identifying a fusion protein produced from host cells are well known in the art and are provided, for example, in Korean Patent No. 10-2228921, but are not limited thereto. For example, a fusion protein produced from host cells can be recovered from the cells as soluble proteins or as inclusion bodies from which they can be extracted quantitatively by 8 M guanidinium hydrochloride and dialysis. For further purification of the fusion protein, any number of purification methods may be used, including but not limited to conventional ion exchange chromatography, affinity chromatography, different sugar chromatography, hydrophobic interaction chromatography, reverse phase chromatography or gel filtration.

### IV. Viral particles and methods for producing viral particles

In one embodiment of the present disclosure, viral particles (or virions) comprising a nucleic acid encoding a modified fusion protein of the present disclosure can be provided.

Viral vectors can be used to deliver a nucleic acid encoding a fusion protein or fusion protein component for expression of the protein in target cells within a particular target tissue (e.g., a diseased tissue). Many species of virus are known, and many have been studied for purposes of delivering nucleic acids to target cells. An exogenous nucleic acid can be inserted into a vector, such as adenovirus, partially deleted adenovirus, fully deleted adenovirus, adeno-associated virus (AAV), retrovirus, lentivirus, etc., for delivery to a cell.

In one embodiment, the cell is within an individual, and the virus can be delivered via intravenous, intramuscular, intraportal or other route of administration. In one embodiment, viral vectors may include those derived from adenoviruses, adeno-associated viruses (AAV), and retroviruses (including lentiviruses, such as human immunodeficiency virus (HIV)). For exemplary viral vectors, see, without limitation, US Patent No. 7,928,072 and WO 2006/113277, which are incorporated herein by reference in their entirety. Viral particles containing a nucleic acid encoding a fusion protein and methods for producing them are well known in the art and are disclosed, for example, in Korean Patent No. 10-2228921, but are not limited thereto.

### V. Methods of treatment using fusion proteins and viral particles

In one embodiment, the present disclosure relates to a pharmaceutical composition for use in the prevention, amelioration, or treatment of a VEGF ligand- or VEGF receptor-related disease or at least one of the symptoms of the disease, comprising a modified fusion protein of the present disclosure as an active ingredient.

In one embodiment, the present disclosure relates to a method for preventing, ameliorating or treating a VEGF ligand- or VEGF receptor-related disease or at least one of the symptoms of the disease, the method comprising administering a modified fusion protein of the present disclosure.

In one embodiment, the present disclosure relates to the use of a modified fusion protein of the present disclosure for preventing, ameliorating or treating a VEGF ligand- or VEGF receptor-related disease or at least one of the symptoms of the disease.

In one embodiment, the present disclosure relates to a modified fusion protein of the present disclosure or a pharmaceutical composition comprising the same for use in a method for preventing, ameliorating or treating a VEGF ligand- or VEGF receptor-related disease or at least one of the symptoms of the disease.

In one embodiment, a modified fusion protein of the present disclosure, in the form of a nucleic acid encoding the same, can be used in a nucleic acid delivery vehicle or viral particles (e.g., a recombinant viral vector) for the prevention, amelioration or treatment of a VEGF ligand- or VEGF receptor-related disease or at least one of the symptoms of the disease. In one embodiment, the nucleic acid delivery vehicle or viral particles may be applied in gene therapy for use in the prevention, amelioration or treatment of a VEGF ligand- or VEGF receptor-related disease or at least one of the symptoms of the disease. In one embodiment, the nucleic acid can produce the encoded protein that mediates a therapeutic effect, i.e., a modified fusion protein of the present disclosure. Any gene therapy method available in the art can be used according to the present disclosure. In one embodiment, the nucleic acid is introduced into a cell prior to administration *in vivo* of the resulting recombinant cell. Cells into which a nucleic acid is introduced for gene therapy purposes encompass all available cell types and include, for example, epithelial cells, endothelial cells, keratinocytes, fibroblasts, muscle cells, hepatocytes, blood cells (e.g., T-lymphocytes, B-lymphocytes, monocytes, macrophages, neutrophils, eosinophils, megakaryocytes, or granulocytes), various stem cells or progenitor cells, such as hematopoietic stem or progenitor cells obtained from bone marrow, umbilical cord blood, peripheral blood, or fetal liver. etc., but are not limited thereto. In one embodiment, the cells used for gene therapy may be autologous cells of the patient.

In one embodiment, a modified fusion protein can bind a VEGF protein and/or a PLGF protein. In one embodiment, a modified fusion protein may have one or more of the following characteristics: (a) bind one or more proteins of the VEGF family, e.g., VEGF-A, VEGF-B, VEGF-C, VEGF-D, or PLGF; (b) block the binding of a VEGF family protein to a VEGF receptor; (c) inhibit activation of the VEGF signaling pathway; (d) prevent, ameliorate and/or treat a disease such as an ocular disease, autoimmune disease, inflammatory disease, neoplastic disease, or cancer; (e) attenuate, contain or prevent growth of neoplasm or cancer; (f) inhibit metastasis of neoplasm or cancer. The activity of a modified fusion protein can be measured *in vivo* and/or *in vitro.*

In one embodiment, a pharmaceutical composition may include a pharmaceutically acceptable carrier and/or diluent. In one embodiment, pharmaceutically acceptable carriers can be sterile liquids, such as water and oils, including those of petroleum, animal, plant, or synthetic origin, such as peanut oil, soybean oil, mineral oil, etc. Saline and aqueous dextrose, polyethylene glycol (PEG) and glycerol solutions can also be used as liquid carriers, particularly for injectable solutions. Such pharmaceutical compositions may further include additional ingredients, for example preservatives, buffers, isotonic agents, antioxidants and stabilizers, nonionic wetting or clarifying agents, thickening agents, etc. A pharmaceutical composition of the present disclosure may be packaged in single unit dosages or in multidosage forms. These compositions are generally formulated as sterile and substantially isotonic solutions. Additionally, compositions may be formulated to have osmotic values compatible with the aqueous humor of the eye and ophthalmic tissues. Such osmotic values can generally be in the range of from about 200 to 400 milliomoles per kilogram of water ("mOsm/kg") or about 300 mOsm/kg. The retina is considered to have an osmotic value of approximately 283 mOsm/kg.

In one embodiment, a pharmaceutical composition may be a parenteral composition. It may be advantageous to formulate parenteral compositions in a dosage unit form for uniformity of dosage and ease of administration.

### Diseases or disorders

In one embodiment, a VEGF ligand- or VEGF receptor-related disease may include an autoimmune disease (e.g., rheumatoid arthritis, multiple sclerosis, or systemic lupus erythematosus), an inflammatory disease (inflammatory arthritis, osteoarthritis, or psoriasis), a neoplastic disease, cancer (e.g., breast cancer, lung cancer, stomach cancer, pancreatic cancer, or leukemia), an angiogenesis-related disease (e.g., atherosclerosis), or an ocular disease (e.g., age-related macular degeneration, choroidal neovascularization, or uveitis), but is not limited thereto.

In one embodiment, cancer may include prostate cancer, urethral cancer, penile cancer, breast cancer, lung cancer, esophageal cancer, small intestine cancer, colorectal cancer, rectal cancer, colon cancer, liver cancer, urinary tract cancer (e.g. bladder cancer), kidney cancer, lung cancer (e.g. non-small cell lung cancer), ovarian cancer, cervical cancer, endometrial cancer, vaginal carcinoma, vulvar carcinoma, pancreatic cancer, stomach cancer, endocrine cancer, thyroid cancer, parathyroid cancer, adrenal cancer, skin cancer (e.g. melanoma), Hodgkin's disease, bone cancer, hematopoietic cancers of lymphoid or myeloid lineage, chronic or acute leukemia, head and neck cancer, nasopharyngeal carcinoma (NPC), glioblastoma, teratocarcinoma, neuroblastoma, adenocarcinoma, cancer of mesenchymal origin (e.g. fibrosarcoma or rhabdomyosarcoma), soft tissue sarcomas and carcinomas, choriocarcinoma, hepatoblastoma, central nervous system (CNS) tumor, primary central nervous system lymphoma, spinal cord tumor, brainstem glioma, pituitary adenoma, Kaposi's sarcoma, or Wilm's tumor, but is not limited thereto. A modified fusion protein of the present disclosure can act as anti-angiogenic agent or anti-VEGF agent to ameliorate, prevent and/or treat cancer or symptoms of cancer.

### Methods of administration and dosages

In one embodiment of the present disclosure, a method of delivering an effective amount of a modified fusion protein to a subject can be provided. The modified fusion protein can be delivered to a subject in a composition. The modified fusion protein can also be delivered to a subject by a nucleic acid delivery vehicle or viral particle (e.g., a recombinant viral vector) containing a nucleic acid encoding the modified fusion protein. In one embodiment of the present disclosure, a composition comprising a modified fusion protein or a nucleic acid delivery vehicle or viral particles (e.g., a recombinant viral vector) comprising a nucleic acid encoding a modified fusion protein can be provided.

In one embodiment, a composition of the present disclosure can be administered to an individual via any route including, but not limited to: intravenous (e.g., by infusion pumps), intraperitoneal, intraocular, intraarterial, intrapulmonary, oral, inhalational, intravesicular, intramuscular, intratracheal, subcutaneous, intraocular, intrathecal, transdermal, transpleural, intraarterial, topical, inhalational (e.g., as mists of sprays), mucosal (e.g., via nasal mucosa), subcutaneous, transdermal, gastrointestinal, intra-articular, intracisternal, intraventricular, intracranial, intraurethral, intrahepatic and intratumoral. In one embodiment, a composition of the present disclosure can be administered by any conventional route, for example, by infusion or bolus injection, by absorption through the epithelial or mucocutaneous linings (e.g., oral mucosa, rectal mucosa, intestinal mucosa, etc.) and can also be administered together with other biologically active ingredients. Administration can be systemic or local. In one embodiment, a composition of the present disclosure may be introduced into the central nervous system by any suitable route, including intraventricular injection and intrathecal injection, wherein intraventricular injection may be administered via an intraventricular catheter, for example, attached to a reservoir, such as an Ommaya reservoir.

In one embodiment, the optimal effective amount of a modified fusion protein, a nucleic acid delivery vehicle encoding the same, or a composition comprising them can be determined empirically and may depend on the type and severity of the disease, the route of administration, disease progression and health, the weight and body area of the individual. Such determinations are within the skill of one of ordinary skill in the art.

In one embodiment, a modified fusion protein may be administered, for example, at about 0.05 ng to about 20 mg per kg of body weight per day. In one embodiment, the dosage of the modified fusion protein may be at least about 0.1 mg/kg, at least about 0.5 mg/kg, at least about 1.0 mg/kg, at least about 1.5 mg/kg, at least about 2.0 mg/kg, at least about 2.5 mg/kg, at least about 3.0 mg/kg, at least about 3.5 mg/kg, at least about 4.0 mg/kg, at least about 4.5 mg/kg, at least about 5.0 mg/kg, at least about 6.0 mg/kg, at least about 8.0 mg /kg, at least about 10.0 mg/kg, at least about 15.0 mg/kg, or about 20.0 mg/kg or less, about 17.0 mg/kg or less, about 14.0 mg/kg or less, about 11.0 mg/kg or less, about 9.0 mg /kg or less, about 7.0 mg/kg or less, about 5.5 mg/kg or less, about 5.0 mg/kg or less, about 4.5 mg/kg or less, about 4.0 mg/kg or less, about 3.5 mg/kg or less, about 3.0 mg/ kg or less, about 2.5 mg/kg or less, about 2.0 mg/kg or less, about 1.5 mg/kg or less, or about 1.0 mg/kg or less. In one embodiment, the dosage of the modified fusion protein may be about 1.0 mg/kg to about 10.0 mg/kg or about 1.0 mg/kg to about 5.0 mg/kg.

In one embodiment, the amount of a nucleic acid delivery vehicle (e.g., a recombinant viral vector) comprising a nucleic acid encoding a modified fusion protein of the present disclosure can be administered to an individual at a DNAse particle resistance (drp) titer of about 10⁴ to about 10¹⁴ drps per dose. In one embodiment, the amount of a nucleic acid delivery vehicle comprising a nucleic acid encoding the modified fusion protein can be administered to an individual at about 10⁵ to about 10¹³, about 10⁶ to about 10¹², about 10⁷ to about 10¹¹, about 10⁸ to about 10¹⁰, about 10⁹ to about 10¹⁰, about 10¹⁰ to about 10¹¹, or about 10¹¹ to about 10¹² drp per dose.

In one embodiment, a composition comprising a modified fusion protein of the present disclosure or a nucleic acid delivery vehicle encoding the same may be administered in a single daily dose, or the total daily dose may be administered in divided dosages of 2, 3, or 4 times daily. In one embodiment, a composition comprising a modified fusion protein of the present disclosure can be administered 6 times a week, 5 times a week, 4 times a week, 3 times a week, 2 times a week, once a week, once every 2 weeks, once every 3 weeks, once a month, once every 2 months, once every 3 months, once every 6 months, once every 9 months, or once every year. In one embodiment, a composition comprising a nucleic acid delivery vehicle comprising a nucleic acid encoding a modified fusion protein of the present disclosure can be administered less frequently, for example, once every 3 months, once every 4 months, once every 5 months, once every 6 months, once every 7 months, once every 8 months, once every 9 months, once every 10 months, once every 11 months, or once every year.

### Diagnosis or detection

A modified fusion proteins of the present disclosure may be labeled with a detectable label such as a radioisotope, a fluorescent label, a toxin label, an enzyme label, a chemiluminescent label, or a nuclear magnetic resonance contrast agent to confirm the ligand-receptor binding interaction. Assay systems applied to chimeric molecules may also be considered. Such detectable labels are well known in the art.

### VI. Articles of manufacture and kits

In one embodiment, the present disclosure relates to an article of manufacture or kit containing a modified fusion protein of the present disclosure, a recombinant viral vector expressing the same, or a composition containing the same in suitable packaging. In one embodiment, the suitable packaging is well known in the art and includes, for example, vials (e.g., sealed vials), vessels, ampoules, bottles, jars, flexible packaging (e.g., sealed Mylar or plastic bags), etc., but is not limited thereto. These articles of manufacture may further be sterilized and/or sealed.

In one embodiment, a kit may further include instructions on how to use the composition for the uses described herein. In one embodiment, the kit may further include other components desirable from a commercial and user standpoint, such as other buffers, diluents, filters, needles, syringes, and package inserts (PIs) with instructions for performing any methods described herein. For example, in one embodiment, a kit may comprise (i) a fusion protein described herein and/or a recombinant viral vector encoding a fusion protein described herein, (ii) a pharmaceutically acceptable carrier, and (iii) any one or more of: a buffer, a diluent, a filter, a needle, a syringe, and a package insert with instructions for performing administration.

Hereinafter, technical features and effects of the present disclosure will be described in more detail referring to working examples. However, these examples are provided only for illustrative purposes to aid understanding of the present disclosure, and the scope of the present disclosure is not limited by the following examples.

### [Example 1] Protein production and evaluation of physicochemical properties and binding ability

### Example 1.1 - Preparation of expression vectors

The coding sequence region (CDS) of a modified fusion protein designed in the examples below was subjected to codon optimization to be in a form suitable for CHO production cells, and the modified fusion protein was prepared in a final pcDNA3.1(+) vector (Invitrogen) via gene synthesis (Geneuniversal). Existing fusion proteins VEGF-Grab1 and VEGF-Grab3 were also produced using the same method.

### Example 1.2 - Production and physicochemical property evaluation of modified fusion proteins

ExpiCHO^{™} cells (Gibco) used for protein production were used for transient expression at a passage number of 10 to 20. VEGF-Grab1 or VEGF-Grab3 was cultured and purified under the same conditions at each step and used as a control for measuring improvements in physicochemical properties of each modified fusion protein. Transient expression of VEGF-Grab1, VEGF-Grab3, and modified fusion proteins was performed according to the Max Titer protocol using Expifectamine^{™} (Gibco) provided by the manufacturer (Gibco). The day before transfection, ExpiCHO^{™} cells were subcultured to a density of 3.0 x 10⁶ cells/ml. The next day, cell density and viability were determined again, and the cells were diluted by adding ExpiCHO^{™} expression medium to a cell density of 6.0 x 10⁶ cells/ml. 25 ml of the cell culture was dispensed into a 125 ml Erlenmeyer flask and the cells were stored in an incubator at 37°C until preparing the transfection mixture. 25 µg of the plasmid gene was mixed well with 1 ml of OptiPro^{™} SFM medium. 80 µl of Expifectamine^{™} CHO cells were mixed well with 920 µl of OptiPro^{™} SFM medium and left at room temperature for 2 minutes. The Expifectamine-OptiPro^{™} SFM mixture was added to previously mixed plasmid-OptiPro^{™} SFM, mixed well, and left at room temperature for 2 minutes. After 2 minutes, the mixture was slowly added to the previously dispensed cell culture and cultured in an incubator set at 37°C, 8% CO₂, and 130 rpm. At 18-22 hours after transfection, 150 µl of Expifectamine^{™} CHO enhancer and 4 ml of ExpiCHO^{™} feed were mixed and added to the culture flask, and cell culture was continued in an incubator set to 32°C, 8% CO₂, and 130 rpm. On day 5 after transfection, an additional 4 ml of ExpiCHO^{™} feed was added. From day 6 after transfection, cell density and viability were measured using a Countess II (Invitrogen) device through trypan blue staining. When the cell viability reached less than 70% to 80%, the final titer was measured using Cedex (Roche) equipment, and then centrifugation was performed at 3000xg for 30 minutes to recover the cell culture containing the protein. The recovered supernatant was filtered through a 0.22 µm syringe filter and the culture solution was stored at -80°C.

Purification of proteins from culture solution was performed by affinity chromatography using an AKTA avant25 instrument equipped with a HiTrap Fibro PrismA column. The culture solution filtered through a 0.22 µm syringe filter was flowed at a rate of 16 ml per minute, and the column was washed with 15 column volumes of a buffer containing 50 mM Na-Pi, 50 mM NaCl, pH 7.0. To remove non-specific binding, the column was washed first with a buffer containing 50mM Na-Pi, 1M NaCl, pH 5.5, followed by 15 column volumes of a buffer containing 50mM Na-OAc, pH 4.6. 15 column volumes of an elution buffer containing 100 mM glycine, pH 3.0 was flowed to elute the bound proteins, which were collected in deep-well blocks or 15 ml tubes. To the collected proteins, 1 M Trizma^{®} base (pH 11.0) solution was added in an amount equal to 1% of the collected protein volume to neutralize the protein solution, and the precipitate was removed using a 0.22 µm syringe filter. Samples before and after neutralization were quantified through UV measurement, analyzed through SE-HPLC, and measured for purity. To measure binding capacity, samples whose purity did not reach 90% were additionally purified by size exclusion chromatography using a Superdex200 increase 10/300 column. The protein concentrated to a volume of less than 500 µl using a Centricon^{®} 30MWCO concentrator was injected onto a column buffered with 250 mM K-Pi, 200 mM KCl, pH 6.2 or 250 mM K-Pi, 200 mM KCl, pH 5.5. The eluted protein was collected in a tube, its purity was checked using SE-HPLC, and then concentration was performed. The final concentrated protein was analyzed through UV quantitative analysis, and the purity was confirmed through SEC-HPLC and SDS-PAGE assay.

Through the above process, one or more of the titer, eluted amount, step yield, high molecular weight species (HMWS) content, low molecular weight species (LMWS) content, and functional monomer content of each protein were determined as its physicochemical properties. Here, the titer refers to the concentration of a Fc-fusion protein present in the culture medium measured at the time of cell culture recovery, the eluted amount refers to the total amount of protein recovered after affinity chromatography, which is the first purification step. The yield refers to a value obtained by dividing the total amount of protein measured in the culture medium by the total amount of the protein recovered after the first purification and represents the recovery rate of protein recovered by the column during the first purification. High molecular weight species (HMWS) are protein species with a mass greater than the predicted functional units of the protein, and low molecular weight species (LMWS) are protein species with a mass smaller than the predicted functional units of the protein. They both represent fractions with abnormal protein folding. A functional monomer refers to a fraction that has the structure and function of a normal protein.

### Example 1.3 - Evaluation of binding affinity to VEGF-A and PLGF

The binding affinities of each protein for VEGF-A and PLGF ligands were measured using an Octet^{®} RED96e (ForteBIO) instrument according to previous literature (Kamat et al, 2017).

First, before measuring the binding affinity, the AHC sensor was stabilized by immersing it in 1x KB buffer (ForteBIO). The substances to be analyzed, VEGF-Grab1 or VEGF-Grab-3, and the modified fusion protein were flowed at a concentration of 20 nM to bind to the sensor, followed by washing with 1x KB buffer to remove proteins remaining after binding. Then, VEGF-A and PLGF were each supplied in 3 concentration ranges (VEGF-A: 40, 20, 10 nM; PLGF: 5, 2.5, 1.25 nM) to measure the binding affinity. Among the binding affinity measurement results, the mean value of the binding affinities measured at two concentrations where indicators for securing reliability, Full X^2 (3 or less) and Full R^2 (0.95 or greater) fall within the window, was calculated and used for relative comparison.

### [Example 2] Design of modified fusion proteins

### Example 2.1 - Preparation and characterization of surface charge transition variants

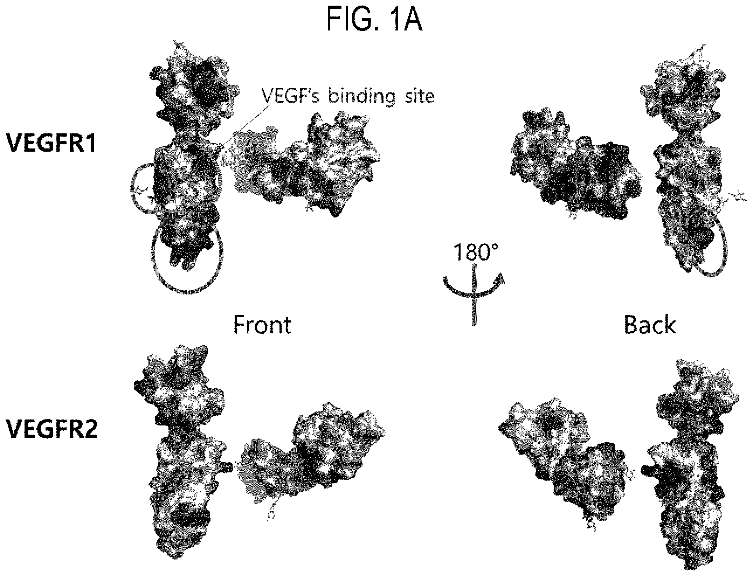

In order to secure a fusion protein containing a VEGFR receptor component with enhanced physicochemical properties, the inventors of the present disclosure used a structure containing the entire extracellular domain of VEGFR1 (PDB entry. 5t89) as a template to analyze the surface charge distribution of the protein and deduced areas with dense positive charges and positions with hydrophobic surface charges (FIG. 1a).

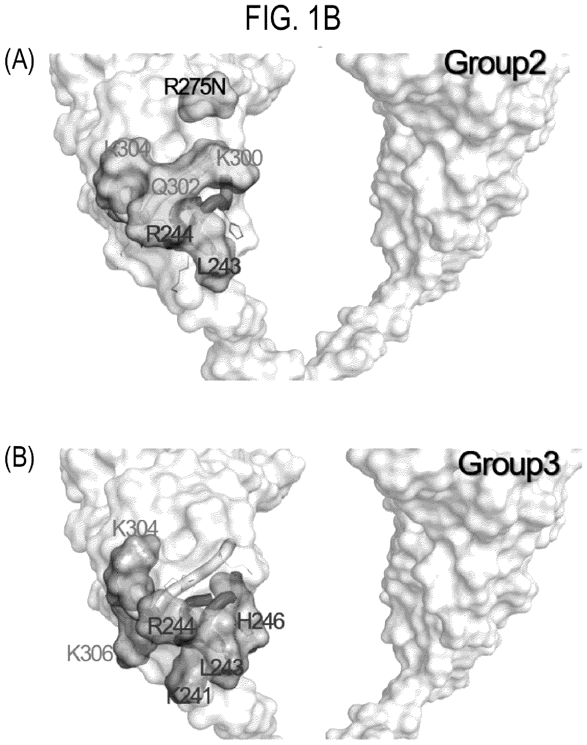

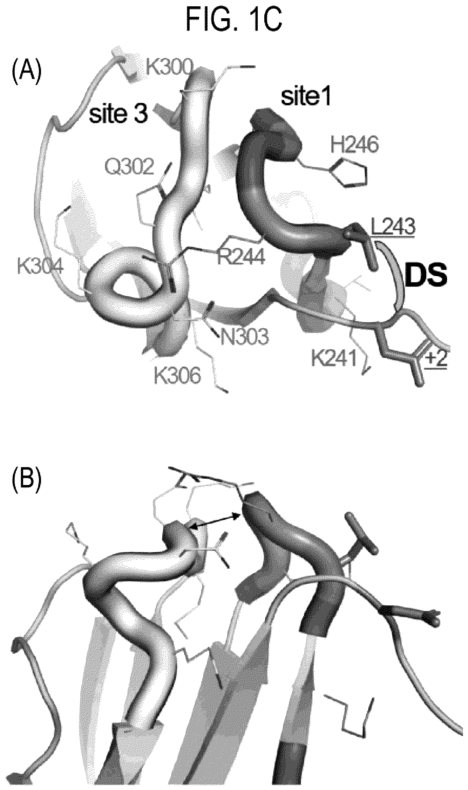

As a result, it was demonstrated that in VEGFR1 domain D3, side chains of the amino acid residue present in the β1-β2 loop (named Site 1) corresponding to amino acid residues T236 to T247, the β2-β3 loop (named Site 2) corresponding to amino acid residues T256 to V262, and the β5-β6 loop (named Site 3) corresponding to amino acid residues D299 to L308 are exposed on the surface of the fusion protein. The positions of amino acid residues where variations can be introduced were selected and inferred by substituting the positions deduced from the surface charge distribution of the protein, focusing on the amino acid residues exposed on the surface (FIGs. 1b and 1c). An attempt was made to stabilize the surface charge and structure of fusion proteins through variations in amino acid residues located in the flexible loops exposed on the surface. For example, an attempt was made to substitute amino acid residues exposed on the surface with amino acid residues present at the homologous positions of VEGFR2. In addition, amino acid residues of Site 2 are exposed on the protein surface on the side that binds to a ligand (VEGF or PLGF), and variations at this site affect binding to the ligands or solubility due to surface exposure of residues. Accordingly, in the case of amino acid residues at Site 2, an attempt was made to substitute them with, for example, an amino acid residue having a smaller side chain than, or a polar side chain of similar size to, the side chain of the existing amino acid residue to an extent that the protein structure or ligand binding is not significantly affected.

Modified fusion proteins with variations introduced at different positions were prepared as shown in Tables 32 and 33, and the variation positions of each variant are shown in Table 1. Variants were prepared according to Example 1, and their physicochemical properties and binding affinity were evaluated. The physicochemical property evaluation results of variants prepared based on the VEGFR-wild type protein (C88) are shown in Table 2, and the binding affinity evaluation results are shown in Tables 3 to 4. The physicochemical property evaluation results of variants prepared based on VEGF-Grab3 are shown in Table 5, and the binding affinity evaluation results are shown in Table 6. The binding affinity evaluation results show results of relative binding affinity evaluation as compared to VEGF-Grab3 (manufactured by Samsung Biologics).

As for the positions of surface charge variations or disulfide bonds recited in the examples or tables below, the positions and amino acid residues of variations are described based on the amino acid sequence of SEQ ID NO: 3 in the case of variants C10 to C87 (SEQ ID NO: 10 to SEQ ID NO: 70), and the positions and amino acid residues of variations are described based on the amino acid sequence of SEQ ID NO: 71 in the case of variants C97 to C114 and C119 (SEQ ID NO: 77 to SEQ ID NO: 93 and SEQ ID NO: 98).

**Table 1**

| Protei n code | Surface charge variation position | | | Linker sequence | Origi n of linker seque nce |
|---|---|---|---|---|---|
| | Site 1 | Site 2 | Site 3 | | |
| C10 | - | - | K300G | DKTHTCPPC P | IgG1 |
| C11 | L243S/R244V | - | - | DKTHTCPPC P | IgG1 |
| C12 | L243S/R244V | - | K300G | DKTHTCPPC P | IgG1 |
| C13 | K241E/L243S/R244 V/H246E | - | - | DKTHTCPPC P | IgG1 |
| C14 | K241E/L243S/R244 V/H246E | - | K300G | DKTHTCPPC P | IgG1 |
| C15 | - | - | K300G/Q302T/K30 4S | DKTHTCPPC P | IgG1 |
| C16 | - | - | K300G/Q302T/K30 4S/K306Q | DKTHTCPPC P | IgG1 |
| C17 | K241E/L243S/R244 V/H246E | - | K300G/Q302T/K30 4S/K306Q | DKTHTCPPC P | IgG1 |
| C19 | - | L258S | - | DKTHTCPPC P | IgG1 |
| C20 | - | L258A | - | DKTHTCPPC P | IgG1 |
| C25 | K241E/L243S/R244 V/H246E | L258A | K300G/Q302T/K30 4S/K306Q | DKTHTCPPC P | IgG1 |
| C26 | K241E/L243S/R244 V/H246E | L258A | K300G | DKTHTCPPC P | IgG1 |
| C27 | K241E/L243S/R244 V/H246E | L258A | K300G/Q302T/K30 4S | DKTHTCPPC P | IgG1 |
| C28 | - | L258A | K300G/Q302T/K30 4S | DKTHTCPPC P | IgG1 |
| C29 | - | L258A | K300G/Q302T/K30 4S/K306Q | DKTHTCPPC P | IgG1 |
| C30 | K241E/L243S/R244 V/H246E | | K300G/Q302T/K30 4S | DKTHTCPPC P | IgG1 |
| C97 | L243S/R244V | - | - | LEDKTHTCP PCP | IgG1 |
| C98 | K241E/L243S/R244 V/H246E | - | - | LEDKTHTCP PCP | IgG1 |
| C99 | | L258A | | LEDKTHTCP PCP | IgG1 |
| C119 | | L258D | | LEDKTHTCP PCP | IgG1 |
| C100 | - | - | K300G/Q302T/K30 4S/K306Q | LEDKTHTCP PCP | IgG1 |
| C101 | L243S/R244V | L258A | | LEDKTHTCP PCP | IgG1 |
| C102 | - | L258A | K300G/Q302T/K30 4S | LEDKTHTCP PCP | IgG1 |
| C103 | - | L258A | K300G/Q302T/K30 4S/K306Q | LEDKTHTCP PCP | IgG1 |
| C104 | K241E/L243S/R244 V/H246E | - | K300G/Q302T/K30 4S/K306Q | LEDKTHTCP PCP | IgG1 |
| C105 | K241E/L243S/R244 V/H246E | L258A | K300G/Q302T/K30 4S/K306Q | LEDKTHTCP PCP | IgG1 |
| C106 | K241E/L243S/R244 V/H246E | L258A | K300G | LEDKTHTCP PCP | IgG1 |
| C107 | K241E/L243S/R244 V/H246E | L258A | K300G/Q302T/K30 4S | LEDKTHTCP PCP | IgG1 |

**Table 2**

| Protein code | HMWS (%) | Functional monomer content (%) | LMWS (%) |
|---|---|---|---|
| C88 | 70.1 | 22.5 | 7.4 |
| VEGF-Grab3 (from Panolos Bioscience) | 60.5 | 34.5 | 5.0 |
| C97 | 70.4 | 24.2 | 5.5 |
| C98 | 55.4 | 34.3 | 10.2 |
| C99 | 56.8 | 34.6 | 8.6 |
| C119 | 57.9 | 36.8 | 5.3 |
| C100 | 54.0 | 36.1 | 9.9 |
| C101 | 69.8 | 24.8 | 5.4 |
| C102 | 52.9 | 44.0 | 3.1 |
| C103 | 54.6 | 36.0 | 9.4 |
| C104 | 56.2 | 35.5 | 8.3 |
| C105 | 56.5 | 36.3 | 7.2 |
| C106 | 64.3 | 29.9 | 5.8 |
| C107 | 57.7 | 36.0 | 6.3 |

**Table 3**

| Protein code | Binding affinity for VEGF-A | | | Binding affinity for VEGF-A vs. C88 (Fold increase) | | |
|---|---|---|---|---|---|---|
| | KD (M) | Kon (1/Ms) | Kdis (1/s) | Fold (KD) | Fold (kon) | Fold (kdis) |
| C88 | 6.08E-11 | 5.78E+05 | 3.51E-05 | 1.00 | 1.00 | 1.00 |

| VEGF-Grab3 (from Panolos Bioscience) | 3.58E-11 | 3.72E+05 | 1.33E-05 | 1.70 | 0.64 | 2.64 |
|---|---|---|---|---|---|---|
| C97 | 6.00E-12 | 2.81E+05 | 1.68E-06 | 10.15 | 0.49 | 20.89 |
| C98 | 3.56E-13 | 2.81E+05 | <1.0E-07 | > 60.86 | 0.49 | > 100 |
| C99 | 3.83E-13 | 2.61E+05 | <1.0E-07 | > 60.86 | 0.45 | > 100 |
| C119 | 4.84E-11 | 5.31E+05 | 2.57E-05 | 1.26 | 0.92 | 1.37 |
| C100 | 3.39E-13 | 2.95E+05 | <1.0E-07 | > 60.86 | 0.51 | > 100 |
| C101 | 1.17E-10 | 4.51E+05 | 5.27E-05 | 0.52 | 0.78 | 0.67 |
| C102 | 8.14E-11 | 3.85E+05 | 3.13E-05 | 0.75 | 0.67 | 1.12 |
| C103 | 2.49E-10 | 2.88E+05 | 7.15E-05 | 0.24 | 0.50 | 0.49 |
| C104 | 3.81E-13 | 2.63E+05 | <1.0E-07 | > 60.86 | 0.46 | > 100 |
| C105 | 3.75E-13 | 2.67E+05 | <1.0E-07 | > 60.86 | 0.46 | > 100 |
| C106 | 3.90E-13 | 2.57E+05 | <1.0E-07 | > 60.86 | 0.44 | > 100 |
| C107 | 4.83E-13 | 2.07E+05 | <1.0E-07 | > 60.86 | 0.36 | > 100 |

**Table 4**

| Protein code | Binding affinity for PLGF | | | Binding affinity for PLGF vs. C88 (Fold increase) | | |
|---|---|---|---|---|---|---|
| | KD(M) | Kon (1/Ms) | Kdis (1/s) | Fold (KD) | Fold (kon) | Fold (kdis) |
| C88 | 2.05E-10 | 1.21E+06 | 2.48E-04 | 1.00 | 1.00 | 1.00 |
| VEGF-Grab3 (from Panolos Bioscience) | 1.51E-10 | 1.33E+06 | 2.02E-04 | 1.36 | 1.10 | 1.23 |
| C97 | 1.52E-10 | 1.28E+06 | 1.95E-04 | 1.35 | 1.06 | 1.28 |
| C98 | 3.75E-10 | 5.49E+05 | 2.06E-04 | 0.55 | 0.45 | 1.21 |
| C99 | 3.76E-10 | 1.36E+06 | 5.11E-04 | 0.54 | 1.12 | 0.49 |
| C119 | 5.34E-10 | 1.83E+06 | 9.75E-04 | 0.38 | 1.50 | 0.25 |
| C100 | 4.53E-10 | 1.56E+06 | 7.05E-04 | 0.45 | 1.29 | 0.35 |
| C101 | 6.84E-11 | 5.60E+06 | 3.83E-04 | 2.99 | 4.61 | 0.65 |
| C102 | 2.21E-10 | 2.36E+06 | 5.23E-04 | 0.93 | 1.95 | 0.47 |
| C103 | 1.61E-10 | 2.21E+06 | 3.55E-04 | 1.27 | 1.82 | 0.70 |
| C104 | 2.23E-10 | 1.21E+06 | 2.69E-04 | 0.92 | 1.00 | 0.92 |
| C105 | 2.70E-10 | 1.76E+06 | 4.75E-04 | 0.76 | 1.45 | 0.52 |
| C106 | 2.45E-10 | 2.31E+06 | 5.64E-04 | 0.84 | 1.90 | 0.44 |
| C107 | 2.35E-10 | 1.58E+06 | 3.71E-04 | 0.87 | 1.30 | 0.67 |

**Table 5**

| Protein code | Titer (g/L) | Eluted amount (mg) | Yield (%) | HMWS (%) | Functional monomer content (%) | LMWS (%) |
|---|---|---|---|---|---|---|
| VEGF-Grab1(from Panolos Bioscience) | 0.527 | 8.6 | 46.7 | 33.1 | 43.0 | 23.8 |
| VEGF-Grab3 (from Panolos Bioscience) | 0.359 | 5.4 | 50.5 | 53.9 | 44.3 | 1.7 |
| C10 | 0.647 | 11.1 | 49.0 | 47.9 | 40.0 | 12.1 |
| C11 | 0.602 | 11.1 | 52.7 | 52.2 | 45.1 | 2.7 |
| C12 | 0.673 | 12.0 | 50.9 | 65.5 | 30.1 | 4.4 |
| C13 | 0.752 | 12.9 | 48.9 | 56.3 | 40.2 | 2.9 |
| C14 | 0.762 | 14.9 | 55.8 | 58.0 | 38.2 | 3.8 |
| C15 | 0.647 | 12.2 | 54.1 | 44.5 | 54.2 | 2.3 |
| C16 | 0.764 | 10.6 | 39.5 | 23.9 | 60.4 | 15.7 |
| C17 | 0.632 | 12.7 | 57.5 | 51.6 | 46.5 | 1.9 |
| C19 | 0.652 | 11.4 | 49.9 | 45.4 | 48.4 | 6.2 |
| C20 | 0.455 | 8.1 | 51.0 | 39.0 | 54.8 | 6.2 |
| C25 | 0.565 | 8.0 | 40.4 | 39.8 | 60.1 | 0.1 |
| C26 | 0.434 | 7.1 | 46.5 | 42.1 | 57.1 | 0.8 |
| C27 | 0.632 | 7.0 | 31.8 | 42.2 | 57.1 | 0.7 |
| C28 | 0.536 | 7.5 | 39.9 | 38.1 | 61.1 | 0.7 |
| C29 | 0.591 | 8.2 | 39.7 | 34.7 | 62.4 | 2.9 |
| C30 | 0.483 | 8.3 | 49.1 | 57.7 | 41.3 | 1.0 |

**Table 6**

| Protei n code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C25 | 0.18 | 1.34 | 0.14 | 0.56 | 1.28 | 0.44 |
| C26 | 0.03 | 1.36 | 0.02 | 0.53 | 1.34 | 0.39 |
| C27 | 0.05 | 1.43 | 0.03 | 0.70 | 2.13 | 0.33 |
| C28 | 0.88 | 0.88 | 1.00 | 0.52 | 1.02 | 0.51 |
| C29 | 0.85 | 0.85 | 1.00 | 0.57 | 1.09 | 0.52 |
| C30 | < 0.01 | 1.67 | < 0.01 | 0.51 | 2.22 | 0.23 |

Referring to the results in Tables 2 to 6, the results of the titer and protein eluted amount demonstrate that modified fusion proteins according to an embodiment of the present disclosure exhibit excellent productivity, and the functional monomer results demonstrate that the modified fusion proteins have excellent physicochemical properties. In addition, for example, the results for kon, etc. demonstrate that the modified fusion proteins also exhibit excellent properties in terms of binding affinity to ligands.

### Example 2.2 - Preparation and characterization of linker variants

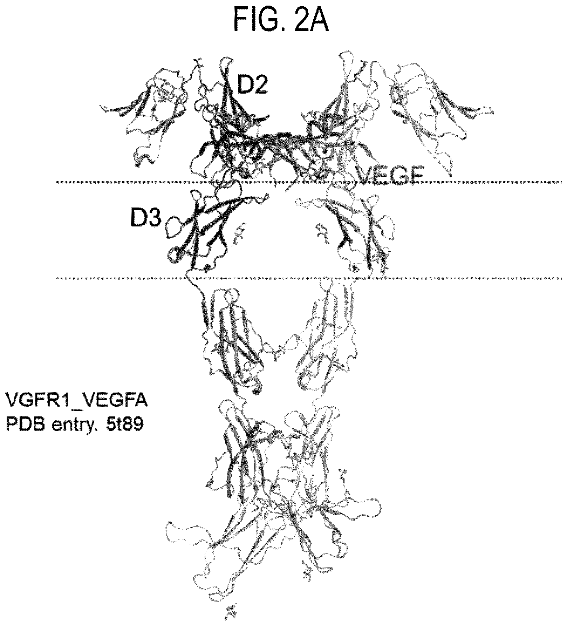

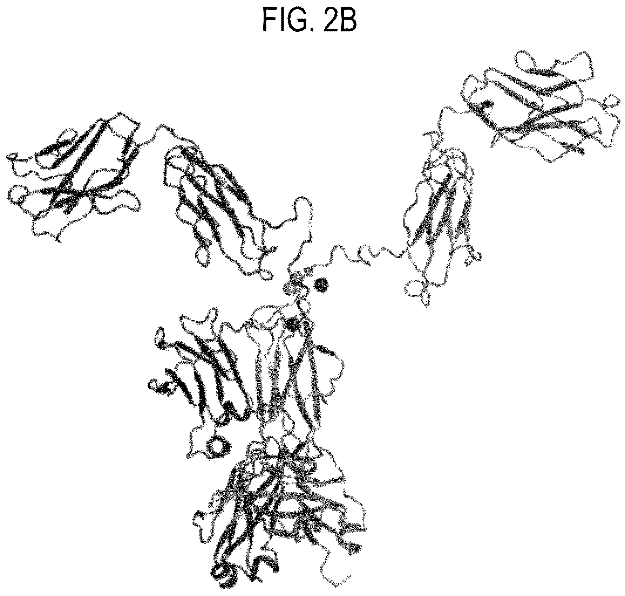

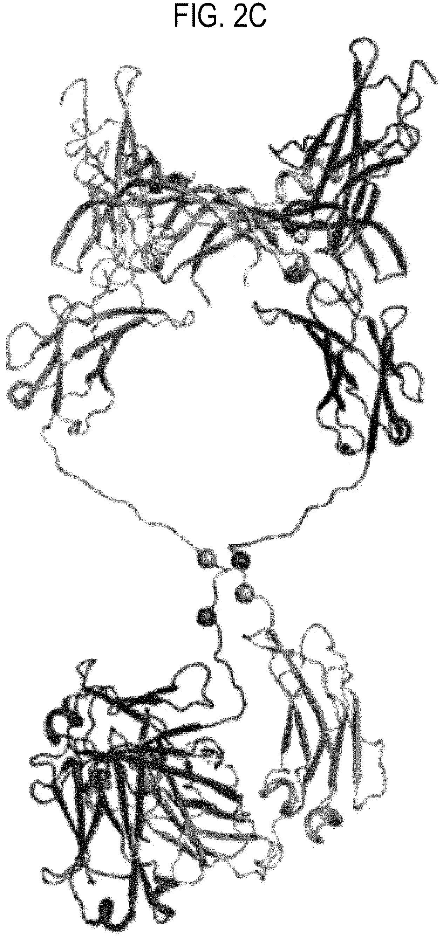

A human VEGF receptor binds to the ligand VEGF or PLGF through interaction with the immunoglobulin-like domains D2 and D3 (*see* FIG. 2a). In the case of a fusion protein using domain D2 and domain D3 (e.g., VEGF-Grab3), domain D2 and domain D3 have an open structure before binding to a ligand and then form a closed structure upon binding to the ligand (*see* FIGs. 2b and 2c). If the constructs of domain D2 and domain D3 cannot not secure enough space to bind to a ligand depending on the binding length of domain D3 and the multimerization domain or interference occurs between the constructs, binding affinity of the fusion protein to a ligand may decrease. Therefore, the binding length and binding mode of domain D3 and the multimerization domain in the fusion protein can affect the structure of the fusion protein that binds to a ligand and its binding affinity to the ligand.

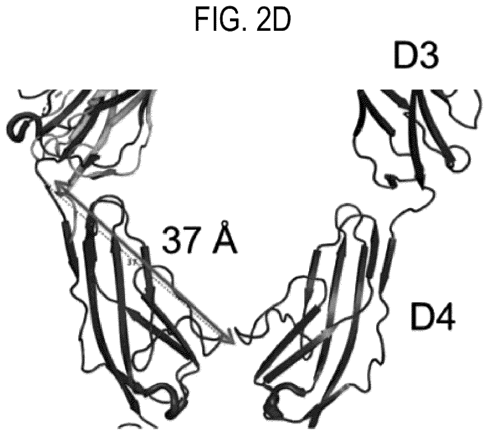

On the other hand, in the case of a human VEGF receptor in the natural state, in a structure where domain D3 and domain D4 are connected, the length from the end of domain D3 to the dimerization site of domain D4 is approximately 37 Å (*see* FIG. 2d), and in the case of a human VEGF receptor, domains from domain D4 and downstream exist in a state where the same domains are bound to each other. Domain D4 and downstream can be viewed as a structure similar to the multimerization domain in a fusion protein. Therefore, the present inventors changed the length and type of the linker in the modified fusion proteins of the present disclosure in order to emulate and secure the structural characteristics of a native human VEGF receptor. Specifically, linkers composed of a polypeptide of Gly-Ser repeat sequence, the amino acid sequence of a hinge region derived from IgG1, IgG4, or IgD, and a variant sequence of the hinges were introduced. Such linkers were intended to increase physical stability by reducing the tension generated in the loop (linker) formed between domain D3 and the multimerization domain (Fc region) upon ligand binding.

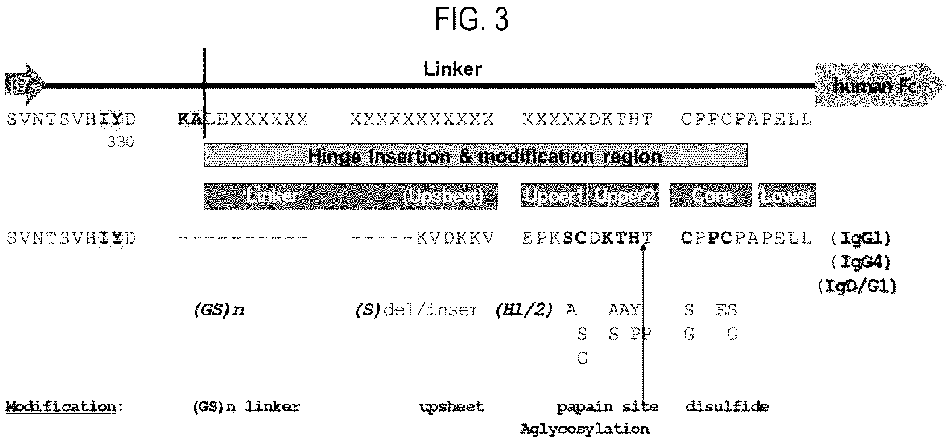

In addition, an amino acid sequence derived from a hinge region of an immunoglobulin has cleavage sites for various proteases due to its own characteristics (Vlasak and Ionescu. 2011. Mabs 3: 253-263) and can cause oligomerization after introduction into a fusion protein, through glycosylation, for example O-glycosylation (Song et. al. 2020. Computational and Structural Biotechnology Journal 18: 3925- 3935). To improve the *in vivo* stability of modified fusion protein of the present disclosure, the present inventors further modified amino acid sequences at cleavage sites by proteolytic enzymes, for example, the cleavage site for papain, and attempted to modify amino acid residues Ser or Thr where glycosylation occurs. Also, to increase the structural flexibility of the linker, attempts were made to substitute cysteines present in sequences derived from the core hinge in the Fc hinge region (e.g., CPPCP). An exemplary schematic diagram for designing a linker introduced in the present disclosure is shown in FIG. 3.

Modified fusion proteins having a linker introduced therein were prepared as shown in Tables 32 and 33, and the linker characteristics of each variant are shown in Table 7. In Table 7, bold and underlined amino acids refer to amino acid residues at the positions of amino acid variation.

Variants were prepared according to Example 1, and their physicochemical properties and binding affinity were evaluated. The physicochemical property evaluation results of variants prepared based on the VEGFR-wild type protein are shown in Table 8, and the binding affinity evaluation results are shown in Tables 9 to 10. The physicochemical property evaluation results of variants prepared based on VEGF-Grab3 are shown in Table 11, and the binding affinity evaluation results are shown in Tables 12 and 13. Tabe 12 shows results of relative binding affinity evaluation as compared to VEGF-Grab3 (manufactured by Panolos Bioscience), and Table 13 shows results of relative binding affinity evaluation as compared to VEGF-Grab3 (manufactured by Samsung Biologics). For relative binding affinity evaluation as compared to C88 or VEGF-Grab3 (manufactured by Panolos Bioscience), the comparisons were made with proteins produced in the same batch as the variants under the same conditions.

**Table 7**

| Protei n code | GS repeat sequence | Papain/glyco sylation variation | Core hinge variation | Origin of Fc hinge in linker sequence | Final linker sequence |
|---|---|---|---|---|---|
| C32 | GSSG | - | **S**PP**S**P | IgG1 | GSSGDKTHTSPPSP |
| C34 | GSSG | - | **S**PP**S**P | IgG1 | GSSGEPKSSDKTYTSPPSP |
| C36 | GS | - | - | IgG1 | GSKVDKKVEPKSSDKTH TCPPCP |
| C37 | GS | SSDKT**Y**T | - | IgG1 | GSKVDKKVEPKSSDKTY TCPPCP |
| C39 | GS | SSDT**PP**T | - | IgG1 | GSKVDKKVEPKSSDTPPT CPPCP |
| C41 | GSGGGGSGG GGSGGGGS | - | - | IgG4 | GSGGGGSGGGGSGGGGS AESKYGPPCPPCP |
| C43 | GS | SSDKT**Y**T | - | IgD/G1 | GSNTGSGGEEKKKEKEK EEQEERSSDKTYTCPPCP |
| C44 | GS | SSDT**PP**T | - | IgD/G1 | GSNTGSGGEEKKKEKEK EEQEERSSDTPPTCPPCP |
| C45 | (E)GSSGGSSG | SD**A**T**P**T | - | IgG1 | EGSSGGSSGEPKSDATPT CPPCP |
| C46 | (E)GSSGGSSG | SD**S**T**Y**T | - | IgG1 | EGSSGGSSGEPKSDSTYT CPPCP |
| C52 | GGGGS | **A**GDK**APP** | **G**PP**G**P | IgG1 | GGGGSAEPKAGDKAPPG PPGP |
| C55 | GGGGS | SSDKT**Y**T | - | IgG1 | GGGGSAEPKSSDKTYTCP PCP |
| C56 | GGGGSGGGG SGGGGSGGG GS | SSDKT**Y**T | - | IgG1 | GGGGSGGGGSGGGGSGG GGSAEPKSSDKTYTCPPC P |
| C108 | GSSG | - | **S**PP**S**P | IgG1 | GSSGDKTHTSPPSP |
| C109 | GS | - | - | IgG1 | GSKVDKKVEPKSSDKTH TCPPCP |
| C111 | (E)GSSGGSSG | SD**A**T**P**T | - | IgG1 | EGSSGGSSGEPKSDATPT CPPCP |
| C115 | | | - | - | - (No linker defined in the present disclosure) |
| C116 | GSSGGSSGGS SGGSSGGSSG GSSGGSSGGS SGGSSGGSSG | - | - | IgG1 | GSSGGSSGGSSGGSSGGS SGGSSGGSSGGSSGGSSG GSSGDKTHTCPPCP |
| C117 | GGGGSGGGG SGGGGSGGG GSGGGGSGG GGSGGGGSG GGGS | - | - | IgG1 | GGGGSGGGGSGGGGSGG GGSGGGGSGGGGSGGGG SGGGGSDKTHTCPPCP |
| C118 | GGGGSGGGG SGGGGSGGG GSGGGGSGG GGS | - | - | IgG1 | GGGGSGGGGSGGGGSGG GGSGGGGSGGGGSDKTH TCPPCP |

**Table 8**

| Protein code | HMWS (%) | Functional monomer content (%) | LMWS (%) |
|---|---|---|---|
| C88 | 70.1 | 22.5 | 7.4 |
| C108 | 61.9 | 25.1 | 13.1 |
| C109 | 65.2 | 26.9 | 7.9 |
| C111 | 65.9 | 27.7 | 6.5 |
| C115 | 65.4 | 18.5 | 16.1 |
| C116 | 70.1 | 24.0 | 5.9 |
| C117 | 78.0 | 15.2 | 6.7 |
| C118 | 68.7 | 25.0 | 6.3 |

**Table 9**

| Protein code | Binding affinity for VEGF-A | | | Binding affinity for VEGF-A vs. C88 (Fold increase) | | |
|---|---|---|---|---|---|---|
| | KD(M) | Kon (1/Ms) | Kdis (1/s) | Fold (KD) | Fold (kon) | Fold (kdis) |
| C88 | 6.08E-11 | 5.78E+05 | 3.51E-05 | 1.00 | 1.00 | 1.00 |
| C108 | 3.14E-10 | 4.83E+05 | 1.52E-04 | 0.19 | 0.84 | 0.23 |
| C109 | 2.67E-10 | 5.21E+05 | 1.39E-04 | 0.23 | 0.90 | 0.25 |
| C111 | 3.75E-11 | 6.99E+05 | 2.63E-05 | 1.62 | 1.21 | 1.34 |

**Table 10**

| Protein code | Binding affinity for PLGF | | | Binding affinity for PLGF vs. C88 (Fold increase) | | |
|---|---|---|---|---|---|---|
| | KD(M) | kon(1/Ms) | kdis(1/s) | Fold (KD) | Fold (kon) | Fold (kdis) |
| C88 | 2.05E-10 | 1.21E+06 | 2.48E-04 | 1.00 | 1.00 | 1.00 |
| C108 | 2.36E-10 | 3.16E+06 | 7.46E-04 | 0.87 | 2.61 | 0.33 |
| C109 | 2.47E-10 | 1.63E+06 | 4.02E-04 | 0.83 | 1.34 | 0.62 |
| C111 | 1.74E-10 | 2.27E+06 | 3.93E-04 | 1.18 | 1.87 | 0.63 |

**Table 11**

| Protein code | Titer (g/L) | Eluted amount(mg) | Yield (%) | HMWS (%) | Functional monomer content (%) | LMWS (%) |
|---|---|---|---|---|---|---|
| VEGF-Grab1(from Panolos Bioscience) | 0.527 | 8.6 | 46.7 | 33.1 | 43.0 | 23.8 |
| VEGF-Grab3 (from Panolos Bioscience) | 0.359 | 5.4 | 50.5 | 53.9 | 44.3 | 1.7 |
| C32 | 0.236 | 7.4 | 89.1 | 27.0 | 63.4 | 9.6 |
| C34 | 0.207 | 7.2 | 99.8 | 29.8 | 60.0 | 10.2 |
| C36 | 0.124 | 4.6 | 106.2 | 35.5 | 50.9 | 13.6 |
| C37 | 0.157 | 5.2 | 95.5 | 38.7 | 48.4 | 12.9 |
| C39 | 0.276 | 8.0 | 82.8 | 38.1 | 51.8 | 10.2 |
| C41 | 0.281 | 7.4 | 75.5 | 33.3 | 55.2 | 11.5 |
| C43 | 0.308 | 8.7 | 80.7 | 35.3 | 57.3 | 7.4 |
| C44 | 0.219 | 5.6 | 73.5 | 30.9 | 58.7 | 10.4 |
| C45 | 0.206 | 5.3 | 73.6 | 32.9 | 67.1 | 0.0 |
| C46 | 0.241 | 5.4 | 64.5 | 36.6 | 63.4 | 0.0 |
| C52 | 0.221 | 9.1 | 120.6 | 25.2 | 70.0 | 4.8 |
| C55 | 0.283 | 9.1 | 97.7 | 25.5 | 70.5 | 4.0 |
| C56 | 0.095 | 4.0 | 132.2 | 33.3 | 60.6 | 6.1 |

**Table 12**

| Protein code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C32 | 2.13 | 1.34 | 1.59 | 3.08 | 0.98 | 3.14 |
| C34 | 1.22 | 1.40 | 0.87 | 2.28 | 0.61 | 3.76 |
| C36 | 0.73 | 1.19 | 0.61 | 1.54 | 1.47 | 1.05 |
| C37 | 0.83 | 1.19 | 0.70 | 0.94 | 0.86 | 1.09 |
| C39 | 0.77 | 1.30 | 0.59 | 4.64 | 2.14 | 2.17 |
| C41 | 0.40 | 1.36 | 0.30 | 3.63 | 2.09 | 1.73 |
| C43 | 0.43 | 1.42 | 0.30 | 1.86 | 0.76 | 2.44 |
| C44 | 0.35 | 1.50 | 0.23 | 1.73 | 1.49 | 1.16 |
| C45 | 0.56 | 1.08 | 0.52 | 1.81 | 1.47 | 1.23 |
| C52 | 0.24 | 1.19 | 0.20 | 1.67 | 1.42 | 1.17 |
| C55 | 0.30 | 1.06 | 0.29 | 1.47 | 1.09 | 1.36 |
| C56 | 0.26 | 1.16 | 0.22 | 2.16 | 1.44 | 1.70 |

**Table 13**

| Protein code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C32 | 0.57 | 1.53 | 0.37 | 4.40 | 1.14 | 3.85 |
| C34 | 0.33 | 1.60 | 0.20 | 3.26 | 0.71 | 4.60 |
| C36 | 0.20 | 1.36 | 0.14 | 2.21 | 1.72 | 1.29 |
| C37 | 0.22 | 1.36 | 0.16 | 1.35 | 1.01 | 1.33 |
| C39 | 0.21 | 1.49 | 0.14 | 6.64 | 2.50 | 2.65 |
| C41 | 0.11 | 1.56 | 0.07 | 5.19 | 2.45 | 2.12 |
| C43 | 0.12 | 1.63 | 0.07 | 2.67 | 0.89 | 2.99 |
| C44 | 0.09 | 1.71 | 0.05 | 2.48 | 1.74 | 1.42 |
| C45 | 0.15 | 1.23 | 0.12 | 2.59 | 1.72 | 1.50 |
| C52 | 0.64 | 1.64 | 0.39 | 1.20 | 1.53 | 0.78 |
| C55 | 0.82 | 1.45 | 0.56 | 1.06 | 1.17 | 0.91 |
| C56 | 0.69 | 1.59 | 0.44 | 1.56 | 1.56 | 1.14 |

Referring to the results in Tables 8 to 13, the results of the titer and protein eluted amount demonstrate that modified fusion proteins according to an embodiment of the present disclosure exhibit excellent productivity, and the functional monomer results demonstrate that the modified fusion proteins have excellent physicochemical properties. In addition, for example, the results for kon, etc. demonstrate that the modified fusion proteins also exhibit excellent properties in terms of binding affinity to ligands.

On the other hand, in the case of variants (C115 to C118) having a linker sequence that deviates from the linker length of a linker variant according to an embodiment of the present disclosure, it is demonstrated that the variant exhibit significantly decreased titers or functional monomer ratios compared to modified fusion proteins of the present disclosure (e.g., variant C108).

### Example 2.3 - Preparation and characterization of disulfide bond variants

Since fusion proteins are combinations of heterologous proteins, stability is expected to be decreased due to non-specific cleavage at the linking site. The end of domain D3 is a loopforming part that may lack secondary structure and is expected to have weak stability because it is the site where connection to the multimerization domain, a heterologous protein, begins. By introducing a covalent disulfide bond, the inventors aimed to induce inter-domain conjugation near the site where heterogeneous proteins are connected, thereby preventing physical cleavage of the protein and improving its stability.

Positions where a disulfide bond can be introduced on the fusion protein was identified through tertiary structure-based analysis. Among the residues located in the variable loop adjacent to the terminal region of domain D3, residues whose side chain is extending toward the linker and for which the distance between the terminal atom of the side chain and the Y329 Cα atom is within 6 Å were selected as variation residues for disulfide bonding. After securing the three-dimensional structure of the fusion protein through a structural modeling program (Phyre2, Swiss model), a structural model having a disulfide bond introduced therein was obtained through in-silico variation of the introduction site. Thereafter, changes in the energy level of the fusion protein due to the introduction of the disulfide bond were predicted through a tertiary structure energy prediction program (FoldX in YASARA, Schymkowitz et al. 2005 Nucleic Acids Research. 33: W382-388).

As positions that are physically close to the end of domain D3 in the fusion protein and capable of forming a disulfide bond, amino acid residues on the β1-β2 loop or β5-β6 loop of domain D3 were selected, and in the case of the end of domain D3, amino acid residues present at positions -2, -1, 0, +1, +2, and +3 relative to Y329 was selected.

Modified fusion proteins having a disulfide bond introduced therein were prepared as shown in Tables 32 and 33, and the disulfide bond characteristics of each variant are shown in Table 14. The energy values of the variants calculated through the tertiary structure energy prediction program are shown in Table 15. Variants were prepared according to Example 1, and their physicochemical properties and binding affinity were evaluated. The physicochemical property evaluation results of variants prepared based on the VEGFR-wild type protein are shown in Table 16, and the binding affinity evaluation results are shown in Tables 17 to 18. The physicochemical property evaluation results of variants prepared based on VEGF-Grab3 are shown in Table 19, and the binding affinity evaluation results are shown in Tables 20 and 21. Tabe 20 shows results of relative binding affinity evaluation as compared to VEGF-Grab3 (manufactured by Panolos Bioscience), and Table 21 shows results of relative binding affinity evaluation as compared to VEGF-Grab3 (manufactured by Samsung Biologics). For relative binding affinity evaluation as compared to VEGF-Grab3 (manufactured by Panolos Bioscience), the comparisons were made with proteins produced in the same batch as the variants under the same conditions.

**Table 14**

| Protein code | Amino acid variation for disulfide bond | Linker sequence | Origin of Fc hinge in linker sequence |
|---|---|---|---|
| C37 | Control | GSKVDKKVEPKSSDKTYT CPPCP | IgG1 |
| C47 | K241C/332C | GCKVDKKVEPKSSDKTYT CPPCP | IgG1 |
| C48 | L243C/Y329C | GSKVDKKVEPKSSDKTYT CPPCP | IgG1 |
| C49 | L243C/331C | CSKVDKKVEPKSSDKTYT CPPCP | IgG1 |
| C50 | R244C/Y329C | GSKVDKKVEPKSSDKTYT CPPCP | IgG1 |
| C51 | N303C/Y329C | GSKVDKKVEPKSSDKTYT CPPCP | IgG1 |
| C88 | Control | LEDKTHTCPPCP | IgG1 |
| C109 | Control | GSKVDKKVEPKSSDKTHT CPPCP | IgG1 |
| C112 | L243C/331C | CSKVDKKVEPKSSDKTYT CPPCP | IgG1 |

**Table 15**

| Protein code | Protein structure energy value (kcal/mol) |
|---|---|
| C37 | 322.64 |
| C47 | 320.01 |
| C48 | 323.96 |
| C49 | 326.73 |
| C50 | 315.08 |
| C51 | 320.02 |

**Table 16**

| Protein code | Titer (g/L) | Eluted amount(m g) | Yield (%) | HMWS (%) | Functional monomer content (%) | LMWS (%) |
|---|---|---|---|---|---|---|
| C88 | 0.223 | 4.9 | 74.1 | 70.1 | 22.5 | 7.4 |
| C109 | 0.133 | 3.2 | 77.8 | 65.2 | 26.9 | 7.9 |
| C112 | 0.202 | 6.2 | 98.6 | 32.2 | 53.6 | 14.2 |

**Table 17**

| Protein code | Binding affinity for VEGF-A | | | Binding affinity for VEGF-A vs. C88 (Fold increase) | | |
|---|---|---|---|---|---|---|
| | KD(M) | kon(1/Ms) | kdis(1/s) | Fold (KD) | Fold (kon) | Fold (kdis) |
| C88 | 6.08E-11 | 5.78E+05 | 3.51E-05 | 1.00 | 1.00 | 1.00 |
| C109 | 2.67E-10 | 5.21E+05 | 1.39E-04 | 0.23 | 0.90 | 0.25 |
| C112 | 2.07E-10 | 6.78E+05 | 1.40E-04 | 0.29 | 1.17 | 0.25 |

**Table 18**

| Protein code | Binding affinity for PLGF | | | Binding affinity for PLGF vs. C88 (Fold increase) | | |
|---|---|---|---|---|---|---|
| | KD(M) | kon(1/Ms) | kdis(1/s) | Fold (KD) | Fold (kon) | Fold (kdis) |
| C88 | 2.05E-10 | 1.21E+06 | 2.48E-04 | 1.00 | 1.00 | 1.00 |
| C109 | 2.47E-10 | 1.63E+06 | 4.02E-04 | 0.83 | 1.34 | 0.62 |
| C112 | 4.38E-11 | 1.03E+06 | 4.49E-05 | 4.68 | 0.85 | 5.53 |

**Table 19**

| Protein code | Titer (g/L) | Eluted amount(mg ) | Yield (%) | HMWS (%) | Functional monomer content (%) | LMWS (%) |
|---|---|---|---|---|---|---|
| VEGF-Grab1(from Panolos Bioscience) | 0.527 | 8.6 | 46.7 | 33.1 | 43.0 | 23.8 |
| VEGF-Grab3 (from Panolos Bioscience) | 0.359 | 5.4 | 50.5 | 53.9 | 44.3 | 1.7 |
| C47 | 0.230 | 7.1 | 88.2 | 35.8 | 53.3 | 10.9 |
| C48 | 0.316 | 9.3 | 83.9 | 46.6 | 53.4 | 0.0 |
| C49 | 0.243 | 5.1 | 60.2 | 11.1 | 88.9 | 0.0 |
| C50 | 0.214 | 5.1 | 68.4 | 45.1 | 54.9 | 0.0 |
| C51 | 0.156 | 6.7 | 123.1 | 53.8 | 46.2 | 0.0 |

**Table 20**

| Protein code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C47 | 0.70 | 0.91 | 0.77 | 1.55 | 0.74 | 2.11 |
| C48 | 0.95 | 1.15 | 0.83 | 2.26 | 1.31 | 1.72 |
| C49 | 1.43 | 0.99 | 1.44 | 1.40 | 0.88 | 1.60 |
| C50 | 0.30 | 1.89 | 0.16 | 2.98 | 2.08 | 1.43 |
| C51 | 0.39 | 1.62 | 0.24 | 2.39 | 1.05 | 2.26 |

**Table 21**

| Protein code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C47 | 0.19 | 1.04 | 0.18 | 2.22 | 0.86 | 2.58 |
| C48 | 0.26 | 1.31 | 0.19 | 3.23 | 1.54 | 2.10 |
| C49 | 0.38 | 1.14 | 0.34 | 2.01 | 1.03 | 1.96 |
| C50 | 0.08 | 2.16 | 0.04 | 4.26 | 2.43 | 1.75 |
| C51 | 0.10 | 1.85 | 0.06 | 3.41 | 1.23 | 2.77 |

Referring to the results in Tables 16 to 21, the results of the titer and protein eluted amount demonstrate that modified fusion proteins according to an embodiment of the present disclosure exhibit excellent productivity, and the functional monomer results demonstrate that the modified fusion proteins have excellent physicochemical properties. This is also demonstrated by the calculation results for the energy levels of the structural model. According to Table 15, the energy value of a fusion protein structure having a disulfide bond introduced therein was equal to or lower than the energy value of a structure without the introduction of a disulfide bond, indicating that structural stabilization can be secured through the introduction of a disulfide bond. In addition, for example, the results for kon, etc. demonstrate that the modified fusion proteins also exhibit excellent properties in terms of binding affinity to ligands.

### Example 2.4 - Preparation and characterization of combinatorial variants

Combination variants were prepared that have a combination of the characteristics of variants showing relatively better characteristics among the variants obtained from Examples 2.1 to 2.3. Characteristics of each variant are shown in Table 22. Variants were prepared according to Example 1, and their physicochemical properties and binding affinity were evaluated. The physicochemical property evaluation results of variants prepared based on the VEGFR-wild type protein are shown in Table 23, and the binding affinity evaluation results are shown in Tables 24 to 25 (For comparison, the VEGFR-wild type protein (C88), and variants C65 and C73 were evaluated together). The physicochemical property evaluation results of variants prepared based on VEGF-Grab3 are shown in Table 26, and the binding affinity evaluation results are shown in Tables 27 and 28. Tabe 27 shows results of relative binding affinity evaluation as compared to VEGF-Grab3 (manufactured by Panolos Bioscience), and Table 28 shows results of relative binding affinity evaluation as compared to VEGF-Grab3 (manufactured by Samsung Biologics). For relative binding affinity evaluation as compared to VEGF-Grab3 (manufactured by Panolos Bioscience), the comparisons were made with proteins produced in the same batch as the variants under the same conditions.

**Table 22**

| Protein code | Surface charge variation position | Linker sequence | Origin of Fc hinge in linker sequence | Amino acid variation for disulfide bond |
|---|---|---|---|---|
| C61 | K241E/R244V/H246E/L2 58A/K300G/Q302T/K304 S | CSSGDATPTSPPSP | IgG1 | L243C/331C |
| C62 | K241E/R244V/H246E/L2 58A/K300G/Q302T/K304 S | CSKVDKKVEPKSSDTPPTC PPCP | IgG1 | L243C/331C |
| C63 | K241E/R244V/H246E/L2 58A/K300G/Q302T/K304 S | CSGGGGSAEPKAGDATPPT CPPCP | IgG1 | L243C/331C |
| C64 | K241E/R244V/H246E/L2 58A/K300G/Q302T/K304 S | CSGGGGSGGGGSGGGGSA ESKYGPPCPPCP | IgG4 | L243C/331C |
| C65 | K241E/R244V/H246E/L2 58A/K300G/Q302T/K304 S | CSNTGSGGEEKKKEKEKEE QEERSSDTPPTCPPCP | IgD/G1 | L243C/331C |
| C66 | K241E/R244V/H246E/L2 58A/K300G/Q302T/K304 S | CGSSGGSSGEPKSDATPTC PPCP | IgG1 | L243C/331C |
| C67 | K241E/R244V/H246E/L2 58A/K300G/Q302T/K304 S | CSKVDKKVEPKSSDKTYT CPPCP | IgG1 | L243C/331C |
| C68 | K241E/R244V/H246E/L2 58S/K300G/Q302T/K304 S | CSKVDKKVEPKSSDTPPTC PPCP | IgG1 | L243C/331C |
| C69 | K241E/R244V/H246E/L2 58D/K300G/Q302T/K304 S | CSKVDKKVEPKSSDTPPTC PPCP | IgG1 | L243C/331C |
| C70 | K241E/R244V/H246E/L2 58S/K300G/Q302T/K304 S | CSGGGGSGGGGSGGGGSA ESKYGPPCPPCP | IgG4 | L243C/331C |
| C71 | K241E/R244V/H246E/L2 58D/K300G/Q302T/K304 S | CSGGGGSGGGGSGGGGSA ESKYGPPCPPCP | IgG4 | L243C/331C |
| C72 | K241E/R244V/H246E/L2 58S/K300G/Q302T/K304 S | CGSSGGSSGEPKSDATPTC PPCP | IgG1 | L243C/331C |
| C73 | K241E/R244V/H246E/L2 58D/K300G/Q302T/K304 S | CGSSGGSSGEPKSDATPTC PPCP | IgG1 | L243C/331C |
| C74 | K241E/R244V/H246E/L2 58D/K300G/Q302T/K304 S | CSSGDATPTSPPSP | IgG1 | L243C/331C |
| C75 | K241E/R244V/H246E/L2 58D/K300G/Q302T/K304 S | CSNTGSGGEEKKKEKEKEE QEERSSDTPPTCPPCP | IgD/G1 | L243C/331C |
| C76 | L258A/K300G/Q302T/K 304S/K306Q | CSSGDATPTSPPSP | IgG1 | L243C /331C |
| C77 | K241T/L258A/K300G/Q 302T/K304S/K306Q | CSKVDKKVEPKSSDTPPTC PPCP | IgG1 | L243C /331C |
| C78 | L258A/K300G/Q302T/K 304S/K306Q | CSGGGGSAEPKAGDATPPT CPPCPPCP | IgG1 | L243C /331C |
| C79 | L258A/K300G/Q302T/K 304S/K306Q | CSGGGGSGGGGSGGGGSA ESKYGPPCPPCP | IgG4 | L243C /331C |
| C80 | L258A/K300G/Q302T/K 304S/K306Q | CSNTGSGGEEKKKEKEKEE QEERSSDTPPTCPPCP | IgD/G1 | L243C /331C |
| C81 | L258A/K300G/Q302T/K 304S/K306Q | CGSSGGSSGEPKSDATPTC PPCP | IgG1 | L243C /331C |
| C82 | L258D/K300G/Q302T/K 304S/K306Q | CSSGDATPTSPPSP | IgG1 | L243C /331C |
| C83 | L258D/K300G/Q302T/K 304S/K306Q | CSKVDKKVEPKSSDTPPTC PPCP | IgG1 | L243C /331C |
| C84 | L258D/K300G/Q302T/K 304S/K306Q | CSGGGGSAEPKAGDATPPT CPPCPPCP | IgG1 | L243C /331C |
| C85 | L258D/K300G/Q302T/K 304S/K306Q | CSGGGGSGGGGSGGGGSA ESKYGPPCPPCP | IgG4 | L243C /331C |
| C86 | L258D/K300G/Q302T/K 304S/K306Q | CSNTGSGGEEKKKEKEKEE QEERSSDTPPTCPPCP | IgD/G1 | L243C /331C |
| C87 | L258D/K300G/Q302T/K 304S/K306Q | CGSSGGSSGEPKSDATPTC PPCP | IgG1 | L243C /331C |
| C113 | K241E/R244V/H246E/L2 58A/K300G/Q302T/K304 S | CSNTGSGGEEKKKEKEKEE QEERSSDTPPTCPPCP | IgD/G1 | L243C/331C |
| C114 | K241E/R244V/H246E/L2 58D/K300G/Q302T/K304 S | CGSSGGSSGEPKSDATPTC PPCP | IgG1 | L243C/331C |

**Table 23**

| Protein code | Titer (g/L) | Eluted amount(m g) | Yield (%) | HMWS (%) | Functional monomer content (%) | LMWS (%) |
|---|---|---|---|---|---|---|
| C88 | 0.223 | 4.9 | 74.1 | 70.1 | 22.5 | 7.4 |
| C113 | 0.276 | 7.3 | 87.0 | 41.4 | 55.5 | 3.2 |
| C114 | 0.475 | 10.8 | 78.7 | 55.8 | 37.7 | 6.5 |
| C65 | 0.185 | 6.9 | 123.1 | 36.1 | 62.9 | 1.1 |
| C73 | 0.143 | 5.0 | 116.4 | 20.2 | 79.8 | 0.0 |

**Table 24**

| Protei n code | Binding affinity for VEGF-A | | | Binding affinity for VEGF-A vs. C88 (Fold increase) | | |
|---|---|---|---|---|---|---|
| | KD(M) | kon(1/Ms) | kdis(1/s) | Fold (KD) | Fold (kon) | Fold (kdis) |
| C88 | 6.08E-11 | 5.78E+05 | 3.51E-05 | 1.00 | 1.00 | 1.00 |
| C113 | 2.20E-10 | 5.56E+05 | 1.23E-04 | 0.28 | 0.96 | 0.29 |
| C114 | 1.83E-13 | 5.47E+05 | <1.0E-07 | > 60.86 | 0.95 | > 100 |
| C65 | 8.46E-11 | 5.16E+05 | 4.37E-05 | 0.72 | 0.89 | 0.80 |
| C73 | 1.53E-13 | 6.52E+05 | <1.0E-07 | > 60.86 | 1.13 | > 100 |

**Table 25**

| Protei n code | Binding affinity for PLGF | | | Binding affinity for PLGF vs. C88 (Fold increase) | | |
|---|---|---|---|---|---|---|
| | KD(M) | kon(1/Ms) | kdis(1/s) | Fold (KD) | Fold (kon) | Fold (kdis) |
| C88 | 2.05E-10 | 1.21E+06 | 2.48E-04 | 1.00 | 1.00 | 1.00 |
| C113 | 1.14E-10 | 1.47E+06 | 1.68E-04 | 1.80 | 1.22 | 1.48 |
| C114 | 1.60E-10 | 1.86E+06 | 2.98E-04 | 1.28 | 1.53 | 0.83 |
| C65 | 1.37E-10 | 2.41E+06 | 3.31E-04 | 1.49 | 1.99 | 0.75 |
| C73 | 1.68E-10 | 2.22E+06 | 3.72E-04 | 1.22 | 1.83 | 0.67 |

**Table 26**

| Protein code | Titer (g/L) | Eluted amount(mg) | Yield (%) | HMWS (%) | Functional monomer content (%) | LMWS (%) |
|---|---|---|---|---|---|---|
| VEGF-Grab1 | 0.527 | 8.6 | 46.7 | 33.1 | 43.0 | 23.8 |
| VEGF-Grab3 | 0.359 | 5.4 | 50.5 | 53.9 | 44.3 | 1.7 |
| C61 | 0.537 | 10 | 62.2 | 10.2 | 89.8 | 0.0 |
| C62 | 0.231 | 5.9 | 85.7 | 11.2 | 88.8 | 0.0 |
| C63 | 0.505 | 9.6 | 63.4 | 9.5 | 90.5 | 0.0 |
| C64 | 0.171 | 6.2 | 121.6 | 13.7 | 86.3 | 0.0 |
| C65 | 0.66 | 8.5 | 42.7 | 9.7 | 90.3 | 0.0 |
| C66 | 0.212 | 6.2 | 98.1 | 20.7 | 79.4 | 0.0 |
| C67 | 0.437 | 5.8 | 43.9 | 5 | 95.1 | 0.0 |
| C68 | 0.153 | 6.3 | 137.3 | 45 | 55.0 | 0.0 |
| C69 | 0.378 | 5 | 44.4 | 2.6 | 97.4 | 0.0 |
| C70 | 0.61 | 8 | 43.9 | 13.2 | 86.9 | 0.0 |
| C71 | 0.698 | 7.1 | 33.8 | 4.6 | 95.4 | 0.0 |
| C72 | 0.451 | 7.3 | 54.1 | 9.5 | 90.5 | 0.0 |
| C73 | 0.408 | 7.1 | 58.3 | 3.1 | 96.9 | 0.0 |
| C74 | 0.535 | 5.8 | 35.9 | 6.7 | 93.3 | 0.0 |
| C75 | 0.295 | 8.2 | 92.2 | 0.4 | 99.6 | 0.0 |
| C76 | 0.767 | 11.3 | 49.0 | 22.2 | 77.8 | 0.0 |
| C77 | 0.530 | 9.7 | 61.1 | 26.8 | 73.2 | 0.0 |
| C78 | 0.920 | 5.9 | 21.3 | 28.5 | 71.5 | 0.0 |
| C79 | 0.542 | 7.3 | 44.6 | 32.6 | 67.4 | 0.0 |
| C80 | 0.409 | 6.8 | 55.7 | 30.2 | 69.8 | 0.0 |
| C81 | 0.249 | 4.3 | 57.0 | 40.8 | 59.2 | 0.0 |
| C82 | 0.974 | 4.5 | 15.4 | 24.5 | 75.2 | 0.0 |
| C83 | 0.771 | 6.5 | 28.0 | 32.9 | 67.1 | 0.0 |
| C84 | 0.504 | 10.7 | 70.6 | 33.0 | 67.0 | 0.0 |
| C85 | 0.401 | 6.1 | 50.4 | 13.4 | 86.6 | 0.0 |
| C86 | 0.669 | 6.7 | 33.2 | 30.1 | 69.9 | 0.0 |
| C87 | 0.715 | 8.0 | 37.5 | 21.9 | 78.1 | 0.0 |

**Table 27**

| Protei n code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C61 | 1.35 | 0.74 | 1.83 | 1.24 | 0.76 | 1.62 |
| C62 | > 100 | 1.27 | > 100 | 1.93 | 1.70 | 1.13 |
| C63 | 1.54 | 0.70 | 2.21 | 1.38 | 0.89 | 1.56 |
| C64 | 2.54 | 1.39 | 1.83 | 1.82 | 1.70 | 1.07 |
| C65 | 2.12 | 0.72 | 2.93 | 1.83 | 0.91 | 2.00 |
| C66 | 1.46 | 1.24 | 1.18 | 3.77 | 1.25 | 3.01 |
| C67 | 1.95 | 0.94 | 2.06 | 1.03 | 0.96 | 1.07 |
| C68 | > 100 | 1.83 | > 100 | 1.21 | 2.43 | 0.50 |
| C69 | > 100 | 1.37 | > 100 | 0.83 | 1.24 | 0.67 |
| C70 | 3.01 | 1.20 | 2.52 | 1.38 | 1.30 | 1.06 |
| C71 | 3.06 | 0.99 | 3.10 | 1.43 | 0.82 | 1.75 |
| C72 | 0.95 | 0.82 | 1.15 | 1.48 | 0.70 | 2.11 |
| C73 | > 100 | 1.29 | > 100 | 2.10 | 0.66 | 3.18 |
| C74 | > 100 | 2.09 | > 100 | 1.07 | 2.06 | 0.52 |
| C75 | 0.27 | 1.38 | 0.20 | 0.88 | 2.07 | 0.42 |
| C76 | > 100 | 1.25 | > 100 | 0.82 | 1.75 | 0.47 |
| C77 | > 100 | 1.17 | > 100 | 0.87 | 1.62 | 0.54 |
| C78 | > 100 | 1.37 | > 100 | 0.91 | 1.67 | 0.55 |
| C79 | > 100 | 1.38 | > 100 | 0.82 | 1.39 | 0.59 |
| C80 | > 100 | 1.50 | > 100 | 0.87 | 1.71 | 0.51 |
| C81 | 1.12 | 1.44 | 0.77 | 8.77 | 1.03 | 8.51 |
| C82 | 1.53 | 1.40 | 1.09 | 0.91 | 1.23 | 0.74 |
| C83 | 1.48 | 1.38 | 1.07 | 0.81 | 1.37 | 0.59 |
| C84 | > 100 | 1.38 | > 100 | 0.76 | 1.29 | 0.59 |
| C85 | > 100 | 1.28 | > 100 | 0.84 | 0.97 | 0.86 |
| C86 | > 100 | 1.08 | > 100 | 1.41 | 0.59 | 2.39 |
| C87 | > 100 | 0.93 | > 100 | 0.64 | 1.42 | 0.45 |

**Table 28**

| Protei n code | Binding affinity for VEGF-A (Fold increase) | | | Binding affinity for PLGF (Fold increase) | | |
|---|---|---|---|---|---|---|
| | Fold (KD) | Fold (kon) | Fold (kdis) | Fold (KD) | Fold (kon) | Fold (kdis) |
| C61 | < 0.01 | 0.94 | < 0.01 | 1.53 | 1.14 | 1.35 |
| C62 | 1.83 | 0.55 | 1.00 | 2.39 | 2.54 | 0.94 |
| C63 | < 0.01 | 0.99 | < 0.01 | 1.71 | 1.32 | 1.30 |
| C64 | 0.01 | 0.50 | < 0.01 | 2.26 | 2.54 | 0.89 |
| C65 | < 0.01 | 0.96 | < 0.01 | 2.26 | 1.36 | 1.66 |
| C66 | < 0.01 | 0.56 | < 0.01 | 4.67 | 1.87 | 2.50 |
| C67 | < 0.01 | 0.73 | < 0.01 | 1.27 | 1.43 | 0.89 |
| C68 | 2.65 | 0.38 | 1.00 | 1.49 | 3.63 | 0.41 |
| C69 | 0.42 | 0.51 | 0.21 | 1.03 | 1.86 | 0.56 |
| C70 | 0.01 | 0.58 | < 0.01 | 1.70 | 1.93 | 0.88 |
| C71 | 0.01 | 0.70 | < 0.01 | 1.77 | 1.22 | 1.45 |
| C72 | < 0.01 | 0.84 | < 0.01 | 1.84 | 1.05 | 1.75 |
| C73 | 1.87 | 0.54 | 1.00 | 2.60 | 0.99 | 2.64 |
| C74 | 1.17 | 1.17 | 1.00 | 1.47 | 2.54 | 0.58 |
| C75 | < 0.01 | 0.77 | < 0.01 | 1.20 | 2.56 | 0.47 |
| C76 | 0.69 | 0.69 | 1.00 | 1.13 | 2.16 | 0.52 |
| C77 | 0.65 | 0.65 | 1.00 | 1.19 | 2.00 | 0.60 |
| C78 | 0.76 | 0.76 | 1.00 | 1.25 | 2.06 | 0.61 |
| C79 | 0.77 | 0.77 | 1.00 | 1.13 | 1.72 | 0.66 |
| C80 | 0.84 | 0.84 | 1.00 | 1.20 | 2.11 | 0.57 |
| C81 | < 0.01 | 0.80 | 0.01 | 12.01 | 1.27 | 9.41 |
| C82 | 0.01 | 0.78 | 0.01 | 1.25 | 1.52 | 0.82 |
| C83 | 0.01 | 0.77 | 0.01 | 1.11 | 1.69 | 0.65 |
| C84 | 0.77 | 0.77 | 1.00 | 1.04 | 1.60 | 0.65 |
| C85 | 0.71 | 0.71 | 1.00 | 1.15 | 1.20 | 0.95 |
| C86 | 0.60 | 0.60 | 1.00 | 1.93 | 0.73 | 2.65 |
| C87 | 0.52 | 0.52 | 1.00 | 0.88 | 1.76 | 0.50 |

Referring to the results in Tables 23 to 28, the results of the titer and protein eluted amount demonstrate that modified fusion proteins according to an embodiment of the present disclosure exhibit excellent productivity, and the functional monomer results demonstrate that the modified fusion proteins have excellent physicochemical properties. In addition, for example, the results for kon, etc. demonstrate that the modified fusion proteins also exhibit excellent properties in terms of binding affinity to ligands. These results demonstrated that the characteristics introduced in the process of structural design of modified fusion proteins in Examples 2.1 to 2.3 were effective and that combinations of these characteristics produce a synergistic effect.

### Example 2.5 - Preparation and characterization of other variants

In the fusion protein, the amino acid sequence DKA located at the end of D3 is structurally the site where connection with the multimerization domain begins, and since it is connected to the linker to form a loop, it is a physically weak site and is prone to cleavage during protein expression and purification. During protein expression and purification, the presence of physically weak regions may lead to generation of fragments. Accordingly, in Examples 2.1 to 2.3 above, modified fusion proteins were prepared in which KA was deleted from the end of domain D3 in most sequences. To ascertain that the amino acid sequence deletion at the end of domain D3 does not affect the improvement of physicochemical properties of a modified fusion protein of the present disclosure, deletion variants involving the amino acid sequence KALE as the site where the C terminus of domain D3 and the linker are connected were prepared and their physicochemical properties were determined. Specifically, a variant (C89) in which the amino acids KA were deleted from the VEGFR-wild type protein (C88), and variants (C90 and C91) in which KA and KAL were deleted from VEGF-Grab3 were prepared.

When producing a fusion protein containing a VEGFR1 extracellular domain, variations may occur in the N-terminal sequence of domain D2 depending on the type or characteristics of the expression vector. To ascertain that such variations do not affect physicochemical properties of the fusion protein, variants having an amino acid variation at the N terminus of domain D2 were prepared. Specifically, considering that the variants in Examples 2.1 to 2.3 all contain the amino acid sequence EF derived from the vector at the N terminus of domain D2, variants containing the amino acid sequence EF (C4 and C5), variants with the amino acid sequence EF deleted (C8 and C9), and variants (C6 and C7) starting with SDT comprising the sequence of original VEGFR1 at the N terminus were prepared.

To ascertain that the amino acid substitution D126E, amino acid substitution L128M, and/or amino acid deletion K217 based on SEQ ID NO: 99 in the multimerization domain used in the production of a fusion protein do not affect physicochemical properties or efficacy of the fusion protein, variants were manufactured. Specifically, a variant (C95) having the amino acid substitution D126E and the amino acid substitution L128M and a variant (C96) having the amino acid deletion K217 were prepared based on VEGF-Grab3.

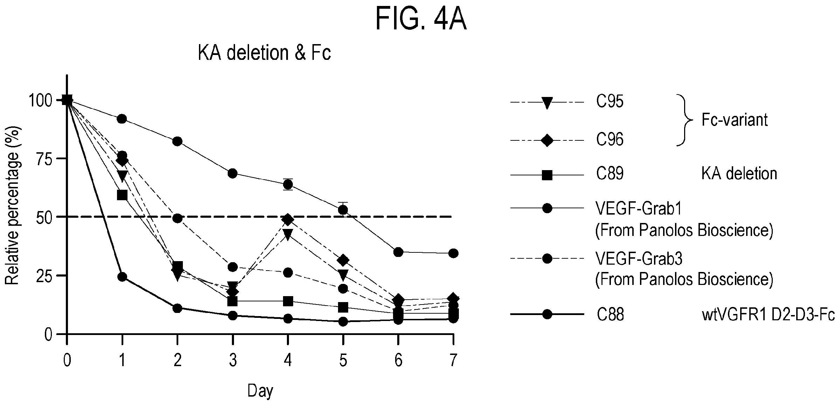

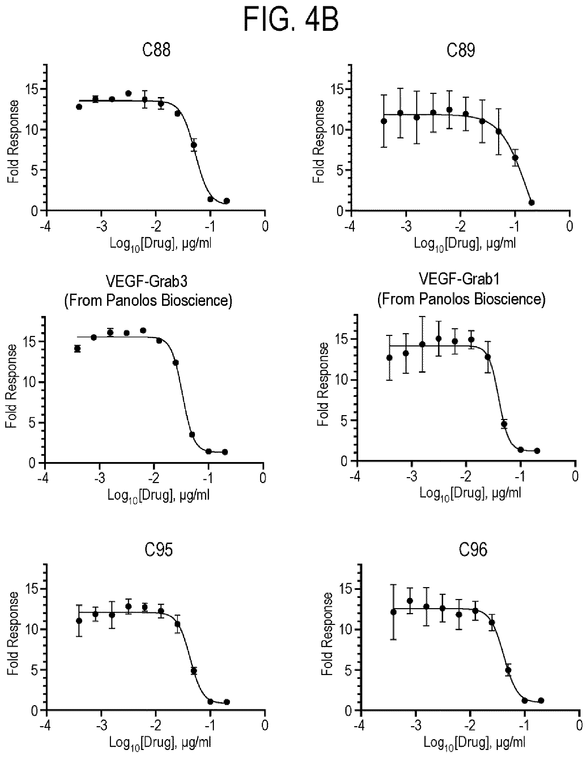

Modified fusion proteins having the above-described additional variations introduced therein were prepared as shown in Table 33. Variants were prepared according to Example 1, and their physicochemical properties, serum stability, or VEGF-A inhibitory ability were evaluated. Physicochemical properties were evaluated according to the method described in Example 1.2, serum stability was evaluated according to the method described in Example 3, and VEGF-A inhibitory ability was evaluated according to the method described in Example 6. The evaluation results are shown in Tables 29 to 31 and FIG. 4.

**Table 29**

| Protein code | Physicochemical properties | | | Serum stability (%) | VEGF-A inhibition (IC₅₀, µg/ml) |
|---|---|---|---|---|---|
| | HMWS (%) | Functional monomer content (%) | LMWS (%) | | |
| C88 | 73.1 | 22.5 | 4.5 | 6.6 | 0.05284 |
| C89 | 73.9 | 21.4 | 4.7 | 8.8 | 0.17220 |
| VEGF-Grab3 | 60.5 | 34.5 | 5.0 | 12.2 | 0.03341 |
| VEGF-Grab1 | 61.7 | 26.3 | 12.1 | 34.4 | 0.03984 |
| C90 | 59.0 | 35.2 | 5.8 | - | - |
| C91 | 59.2 | 34.4 | 6.4 | - | 0.03890 |

**Table 30**

| Protein code | Titer (g/L) | Eluted amount(mg ) | Yield (%) | HMWS (%) | Functional monomer content (%) | LMWS (%) |
|---|---|---|---|---|---|---|
| VEGF-Grab1 | 0.527 | 8.6 | 46.7 | 33.1 | 43.0 | 23.8 |
| VEGF-Grab3 | 0.359 | 5.4 | 50.5 | 53.9 | 44.3 | 1.7 |
| C4 | 0.312 | 6.2 | 56.5 | 43.4 | 35.7 | 20.9 |
| C5 | 0.545 | 8.0 | 41.8 | 51.1 | 41.9 | 7.0 |
| C6 | 0.468 | 7.3 | 44.9 | 50.1 | 45.9 | 4.1 |
| C7 | 0.340 | 7.6 | 63.9 | 52.4 | 42.2 | 5.4 |
| C8 | 0.305 | 7.7 | 71.7 | 35.4 | 32.8 | 13.8 |
| C9 | 0.429 | 7.7 | 51.5 | 45.9 | 34.0 | 20.1 |

**Table 31**

| Protein code | Physicochemical properties | | | Serum stability (%) | VEGF-A inhibition (IC₅₀, µg/ml) |
|---|---|---|---|---|---|
| | HMWS (%) | Functional monomer content (%) | LMWS (%) | | |
| VEGF-Grab3 | 60.5 | 34.5 | 5.0 | 12.2 | 0.03341 |
| C95 | 62.0 | 34.4 | 3.6 | 13.6 | 0.04262 |
| C96 | 65.9 | 30.7 | 3.4 | 15.2 | 0.04143 |

The results in Tables 29 to 31 demonstrate that variations at the end of domain D3, variations at the N terminus of domain D2, and variations in the multimerization domain do not affect the improvement of physicochemical properties, serum stability, or VEGF-A inhibitory ability of the modified fusion proteins.

Characteristics of the variants described in Examples 2.1 to 2.4 above are summarized in Table 32 below.

**Table 32**

| Protei n code | Surface charge variation position | Linker sequence | Origin of Fc hinge in linker sequence | Disulfide bond position |
|---|---|---|---|---|
| C10 | K300G | DKTHTCPPCP | IgG1 | - |
| C11 | L243S/R244V | DKTHTCPPCP | IgG1 | - |
| C12 | L243S/R244V/K300G | DKTHTCPPCP | IgG1 | - |
| C13 | K241E/L243S/R244V/H 246E | DKTHTCPPCP | IgG1 | - |
| C14 | K241E/L243S/R244V/H 246E/K300G | DKTHTCPPCP | IgG1 | - |
| C15 | K300G/Q302T/K304S | DKTHTCPPCP | IgG1 | - |
| C16 | K300G/Q302T/K304S/ K306Q | DKTHTCPPCP | IgG1 | - |
| C17 | K241E/L243S/R244V/H 246E/K300G/Q302T/K3 04S/K306Q | DKTHTCPPCP | IgG1 | - |
| C19 | L258S | DKTHTCPPCP | IgG1 | - |
| C20 | L258A | DKTHTCPPCP | IgG1 | - |
| C25 | K241E/L243S/R244V/H 246E/L258A/K300G/Q3 02T/K304S/K306Q | DKTHTCPPCP | IgG1 | - |
| C26 | K241E/L243S/R244V/H 246E/L258A/K300G | DKTHTCPPCP | IgG1 | - |
| C27 | K241E/L243S/R244V/H 246E/L258A/K300G/Q3 02T/K304S | DKTHTCPPCP | IgG1 | - |
| C28 | L258A/K300G/Q302T/ K304S | DKTHTCPPCP | IgG1 | - |
| C29 | L258A/K300G/Q302T/ K304S/K306Q | DKTHTCPPCP | IgG1 | - |
| C30 | K241E/L243S/R244V/H 246E/K300G/Q302T/K3 04S | DKTHTCPPCP | IgG1 | - |
| C32 | - | GSSGDKTHTSPPSP | IgG1 | - |
| C34 | - | GSSGEPKSSDKTYTSPPSP | IgG1 | - |
| C36 | - | GSKVDKKVEPKSSDKTHT CPPCP | IgG1 | - |
| C37 | - | GSKVDKKVEPKSSDKTYT CPPCP | IgG1 | - |
| C39 | - | GSKVDKKVEPKSSDTPPT CPPCP | IgG1 | - |
| C41 | - | GSGGGGSGGGGSGGGGS AESKYGPPCPPCP | IgG4 | - |
| C43 | - | GSNTGSGGEEKKKEKEKE EQEERSSDKTYTCPPCP | IgD/G1 | - |
| C44 | - | GSNTGSGGEEKKKEKEKE EQEERSSDTPPTCPPCP | IgD/G1 | - |
| C45 | - | EGSSGGSSGEPKSDATPTC PPCP | IgG1 | - |
| C46 | - | EGSSGGSSGEPKSDSTYTC PPCP | IgG1 | - |
| C47 | - | GCKVDKKVEPKSSDKTY TCPPCP | IgG1 | K241C/332 C |
| C48 | - | GSKVDKKVEPKSSDKTYT CPPCP | IgG1 | L243C/Y32 9C |
| C49 | - | CSKVDKKVEPKSSDKTYT CPPCP | IgG1 | L243C/331 C |
| C50 | - | GSKVDKKVEPKSSDKTYT CPPCP | IgG1 | R244C/Y32 9C |
| C51 | - | GSKVDKKVEPKSSDKTYT CPPCP | IgG1 | N303C/Y32 9C |
| C52 | - | GGGGSAEPKAGDKAPPGP PGP | IgG1 | |
| C55 | - | GGGGSAEPKSSDKTYTCP PCP | IgG1 | |
| C56 | - | GGGGSGGGGSGGGGSGG GGSAEPKSSDKTYTCPPC P | IgG1 | |
| C61 | K241E/R244V/H246E/L 258A/K300G/Q302T/K3 04S | CSSGDATPTSPPSP | IgG1 | L243C/331 C |
| C62 | K241E/R244V/H246E/L 258A/K300G/Q302T/K3 04S | CSKVDKKVEPKSSDTPPT CPPCP | IgG1 | L243C/331 C |
| C63 | K241E/R244V/H246E/L 258A/K300G/Q302T/K3 04S | CSGGGGSAEPKAGDATPP TCPPCP | IgG1 | L243C/331 C |
| C64 | K241E/R244V/H246E/L 258A/K300G/Q302T/K3 04S | CSGGGGSGGGGSGGGGS AESKYGPPCPPCP | IgG4 | L243C/331 C |
| C65 | K241E/R244V/H246E/L 258A/K300G/Q302T/K3 04S | CSNTGSGGEEKKKEKEKE EQEERSSDTPPTCPPCP | IgD/G1 | L243C/331 C |
| C66 | K241E/R244V/H246E/L 258A/K300G/Q302T/K3 04S | CGSSGGSSGEPKSDATPT CPPCP | IgG1 | L243C/331 C |
| C67 | K241E/R244V/H246E/L 258A/K300G/Q302T/K3 04S | CSKVDKKVEPKSSDKTYT CPPCP | IgG1 | L243C/331 C |
| C68 | K241E/R244V/H246E/L 258S/K300G/Q302T/K3 04S | CSKVDKKVEPKSSDTPPT CPPCP | IgG1 | L243C/331 C |
| C69 | K241E/R244V/H246E/L 258D/K300G/Q302T/K3 04S | CSKVDKKVEPKSSDTPPT CPPCP | IgG1 | L243C/331 C |
| C70 | K241E/R244V/H246E/L 258S/K300G/Q302T/K3 04S | CSGGGGSGGGGSGGGGS AESKYGPPCPPCP | IgG4 | L243C/331 C |
| C71 | K241E/R244V/H246E/L 258D/K300G/Q302T/K3 04S | CSGGGGSGGGGSGGGGS AESKYGPPCPPCP | IgG4 | L243C/331 C |
| C72 | K241E/R244V/H246E/L 258S/K300G/Q302T/K3 04S | CGSSGGSSGEPKSDATPT CPPCP | IgG1 | L243C/331 C |
| C73 | K241E/R244V/H246E/L 258D/K300G/Q302T/K3 04S | CGSSGGSSGEPKSDATPT CPPCP | IgG1 | L243C/331 C |
| C74 | K241E/R244V/H246E/L 258D/K300G/Q302T/K3 04S | CSSGDATPTSPPSP | IgG1 | L243C/331 C |
| C75 | K241E/R244V/H246E/L 258D/K300G/Q302T/K3 04S | CSNTGSGGEEKKKEKEKE EQEERSSDTPPTCPPCP | IgD/G1 | L243C/331 C |
| C76 | L258A/K300G/Q302T/ K304S/K306Q | CSSGDATPTSPPSP | IgG1 | L243C/331 C |
| C77 | L258A/K300G/Q302T/ K304S/K306Q | CSKVDKKVEPKSSDTPPT CPPCP | IgG1 | L243C/331 C |
| C78 | L258A/K300G/Q302T/ K304S/K306Q | CSGGGGSAEPKAGDATPP TCPPCPPCP | IgG1 | L243C/331 C |
| C79 | L258A/K300G/Q302T/ K304S/K306Q | CSGGGGSGGGGSGGGGS AESKYGPPCPPCP | IgG4 | L243C/331 C |
| C80 | L258A/K300G/Q302T/ K304S/K306Q | CSNTGSGGEEKKKEKEKE EQEERSSDTPPTCPPCP | IgD/G1 | L243C/331 C |
| C81 | L258A/K300G/Q302T/ K304S/K306Q | CGSSGGSSGEPKSDATPT CPPCP | IgG1 | L243C/331 C |
| C82 | L258D/K300G/Q302T/ K304S/K306Q | CSSGDATPTSPPSP | IgG1 | L243C/331 C |
| C83 | L258D/K300G/Q302T/ K304S/K306Q | CSKVDKKVEPKSSDTPPT CPPCP | IgG1 | L243C/331 C |
| C84 | L258D/K300G/Q302T/ K304S/K306Q | CSGGGGSAEPKAGDATPP TCPPCPPCP | IgG1 | L243C/331 C |
| C85 | L258D/K300G/Q302T/ K304S/K306Q | CSGGGGSGGGGSGGGGS AESKYGPPCPPCP | IgG4 | L243C/331 C |
| C86 | L258D/K300G/Q302T/ K304S/K306Q | CSNTGSGGEEKKKEKEKE EQEERSSDTPPTCPPCP | IgD/G1 | L243C/331 C |
| C87 | L258D/K300G/Q302T/ K304S/K306Q | CGSSGGSSGEPKSDATPT CPPCP | IgG1 | L243C/331 C |
| C97 | L243S/R244V | LEDKTHTCPPCP | IgG1 | - |
| C98 | K241E/L243S/R244V/H 246E | LEDKTHTCPPCP | IgG1 | - |
| C99 | L258A | LEDKTHTCPPCP | IgG1 | - |
| C119 | L258D | LEDKTHTCPPCP | IgG1 | - |
| C100 | K300G/Q302T/K304S/ K306Q | LEDKTHTCPPCP | IgG1 | - |
| C101 | L243S/R244V/L258A | LEDKTHTCPPCP | IgG1 | - |
| C102 | L258A/K300G/Q302T/ K304S | LEDKTHTCPPCP | IgG1 | - |
| C103 | L258A/K300G/Q302T/ K304S/K306Q | LEDKTHTCPPCP | IgG1 | - |
| C104 | K241E/L243S/R244V/H 246E/K300G/Q302T/K3 04S/K306Q | LEDKTHTCPPCP | IgG1 | - |
| C105 | K241E/L243S/R244V/H 246E/L258A/K300G/Q3 02T/K304S/K306Q | LEDKTHTCPPCP | IgG1 | - |
| C106 | K241E/L243S/R244V/H 246E/L258A/K300G | LEDKTHTCPPCP | IgG1 | - |
| C107 | K241E/L243S/R244V/H 246E/L258A/K300G/Q3 02T/K304S | LEDKTHTCPPCP | IgG1 | - |
| C108 | - | GSSGDKTHTSPPSP | IgG1 | - |
| C109 | - | GSKVDKKVEPKSSDKTHT CPPCP | IgG1 | - |
| C111 | - | EGSSGGSSGEPKSDATPTC PPCP | IgG1 | - |
| C112 | - | CSKVDKKVEPKSSDKTYT CPPCP | IgG1 | L243C/331 C |
| C113 | K241E/R244V/H246E/L 258A/K300G/Q302T/K3 04S | CSNTGSGGEEKKKEKEKE EQEERSSDTPPTCPPCP | IgD/G1 | L243C/331 C |
| C114 | K241E/R244V/H246E/L 258D/K300G/Q302T/K3 04S | CGSSGGSSGEPKSDATPT CPPCP | IgG1 | L243C/331 C |
| C115 | | - (No linker defined in the present disclosure) | | |
| C116 | - | GSSGGSSGGSSGGSSGGSS GGSSGGSSGGSSGGSSGG SSGDKTHTCPPCP | IgG1 | - |
| C117 | - | GGGGSGGGGSGGGGSGG GGSGGGGSGGGGSGGGG SGGGGSDKTHTCPPCP | IgG1 | - |
| C118 | - | GGGGSGGGGSGGGGSGG GGSGGGGSGGGGSDKTH TCPPCP | IgG1 | - |

Identification codes, amino acid sequences, and SEQ ID NOs of the proteins described in the present disclosure are listed in Table 33.

**Table 33**

| Protein code | Amino acid sequence | SEQ ID NO |
|---|---|---|
| VEGFR1 (FLT1) | MVSYWDTGVLLCALLSCLLLTGSSSGSKLKDPELSLKGT QHIMQAGQTLHLQ CRGEAAHKWSLPEMVSKESERLSITKSACGRNGKQFCSTL TLNTAQANHTGF YSCKYLAVPTSKKKETESAIYIFISDTGRPFVEMYSEIPEIIH MTEGRELVI PCRVTSPNITVTLKKFPLDTLIPDGKRIIWDSRKGFIISNAT YKEIGLLTCE ATVNGHLYKTNYLTHRQTNTIIDVQISTPRPVKLLRGHTL VLNCTATTPLNT RVQMTWSYPDEKNKRASVRRRIDQSNSHANIFYSVLTIDK MQNKDKGLYTCR VRSGPSFKSVNTSVHIYDKAFITVKHRKQQVLETVAGKRS YRLSMKVKAFPS PEVVWLKDGLPATEKSARYLTRGYSLIIKDVTEEDAGNYT ILLSIKQSNVFK | SEQ ID NO: 1 |
| | NLTATLIVNVKPQIYEKAVSSFPDPALYPLGSRQILTCTAY GIPQPTIKWFW HPCNHNHSEARCDFCSNNEESFILDADSNMGNRIESITQR MAIIEGKNKMAS TLVVADSRISGIYICIASNKVGTVGRNISFYITDVPNGFHVN LEKMPTEGED LKLSCTVNKFLYRDVTWILLRTVNNRTMHYSISKQKMAI TKEHSITLNLTIM NVSLQDSGTYACRARNVYTGEEILQKKEITIRDQEAPYLL RNLSDHTVAISS STTLDCHANGVPEPQITWFKNNHKIQQEPGIILGPGSSTLFI ERVTEEDEGV YHCKATNQKGSVESSAYLTVQGTSDKSNLELITLTCTCVA ATLFWLLLTLFI RKMKRSSSEIKTDYLSIIMDPDEVPLDEQCERLPYDASKW EFARERLKLGKS LGRGAFGKVVQASAFGIKKSPTCRTVAVKMLKEGATASE YKALMTELKILTH IGHHLNVVNLLGACTKQGGPLMVIVEYCKYGNLSNYLKS KRDLFFLNKDAAL HMEPKKEKMEPGLEQGKKPRLDSVTSSESFASSGFQEDKS LSDVEEEEDSDG FYKEPITMEDLISYSFQVARGMEFLSSRKCIHRDLAARNIL LSENNVVKICD FGLARDIYKNPDYVRKGDTRLPLKWMAPESIFDKIYSTKS DVWSYGVLLWEI FSLGGSPYPGVQMDEDFCSRLREGMRMRAPEYSTPEIYQI | |
| | MLDCWHRDPKER PRFAELVEKLGDLLQANVQQDGKDYIPINAILTGNSGFTY STPAFSEDFFKE SISAPKFNSGSSDDVRYVNAFKFMSLERIKTFEELLPNATS MFDDYQGDSST LLASPMLKRFTWTDSKPKASLKIDLRVTSKSKESGLSDVS RPSFCHSSCGHV SEGKRRFTYDHAELERKIACCSPPPDYNSVVLYSTPPI | |
| VEGF-Grab 1 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKRASVRRRIDQSNSHANIFYS VLTIDKMQNKDKGLYTCRVRSGPSFKSVNTSVHIYDKAL EDKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPGK | SEQ ID NO: 2 |
| VEGF-Grab3 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDKA LEDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTIS | SEQ ID NO: 3 |
| | KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPGK | |
| C4 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDKD KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPG | SEQ ID NO: 4 |
| C5 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDKET KTPECPAPELLGGPSVFLFPPKPKDTLMISRTPEVTCVVVD VSHEDPEVKFNWYVDGVEVHNAKTKPREEQYNSTYRVV SVLTVLHQDWLNGKEYKCKVSNKALPAPIEKTISKAKGQ PREPQVYTLPPSREEMTKNQVSLTCLVKGFYPSDIAVEWE SNGQPENNYKTTPPVLDSDGSFFLYSKLTVDKSRWQQGN VFSCSVMHEALHNHYTQKSLSLSPG | SEQ ID NO: 5 |
| C6 | SDTGRPFVEMYSEIPEIIHMTEGRELVIPCRVTSPNITVTLK KFPLDTLIPDGKRIIWDSRKGFIISNATYKEIGLLTCEATVN | SEQ ID NO: 6 |
| | GHLYKTNYLTHRQTNTIIDVQISTPSPVTLLRGHTLVLNCT ATTPLNTRVQMTWSYPDEKNKNASVRRRIDQSNSHANIF YSVLTIDKMQNKDKGLYTCRVRSGPSFKSVNTSVHIYDK DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSREEMTKNQVSLTCLVKGFYPSDIA VEWESNGQPENNYKTTPPVLDSDGSFFLYSKLTVDKSRW QQGNVFSCSVMHEALHNHYTQKSLSLSPG | |
| C7 | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVQISTPSPVTLLRGHTLVLNCT ATTPLNTRVQMTWSYPDEKNKNASVRRRIDQSNSHANIF YSVLTIDKMQNKDKGLYTCRVRSGPSFKSVNTSVHIYDK ETKTPECPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPG | SEQ ID NO: 7 |
| C8 | GRPFVEMYSEIPEIIHMTEGRELVIPCRVTSPNITVTLKKFP LDTLIPDGKRIIWDSRKGFIISNATYKEIGLLTCEATVNGHL YKTNYLTHRQTNTIIDVQISTPSPVTLLRGHTLVLNCTATT PLNTRVQMTWSYPDEKNKNASVRRRIDQSNSHANIFYSV LTIDKMQNKDKGLYTCRVRSGPSFKSVNTSVHIYDKDKT HTCPPCPAPELLGGPSVFLFPPKPKDTLMISRTPEVTCVVV DVSHEDPEVKFNWYVDGVEVHNAKTKPREEQYNSTYRV | SEQ ID NO: 8 |
| | VSVLTVLHQDWLNGKEYKCKVSNKALPAPIEKTISKAKG QPREPQVYTLPPSREEMTKNQVSLTCLVKGFYPSDIAVEW ESNGQPENNYKTTPPVLDSDGSFFLYSKLTVDKSRWQQG NVFSCSVMHEALHNHYTQKSLSLSPG | |
| C9 | GRPFVEMYSEIPEIIHMTEGRELVIPCRVTSPNITVTLKKFP LDTLIPDGKRIIWDSRKGFIISNATYKEIGLLTCEATVNGHL YKTNYLTHRQTNTIIDVQISTPSPVTLLRGHTLVLNCTATT PLNTRVQMTWSYPDEKNKNASVRRRIDQSNSHANIFYSV LTIDKMQNKDKGLYTCRVRSGPSFKSVNTSVHIYDKETKT PECPAPELLGGPSVFLFPPKPKDTLMISRTPEVTCVVVDVS HEDPEVKFNWYVDGVEVHNAKTKPREEQYNSTYRVVSV LTVLHQDWLNGKEYKCKVSNKALPAPIEKTISKAKGQPR EPQVYTLPPSREEMTKNQVSLTCLVKGFYPSDIAVEWESN GQPENNYKTTPPVLDSDGSFFLYSKLTVDKSRWQQGNVF SCSVMHEALHNHYTQKSLSLSPG | SEQ ID NO: 9 |
| C10 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDGMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 10 |
| C11 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLSVGHTLVLNCTA TTPLNTRVQMTWSYPDEKNKNASVRRRIDQSNSHANIFY SVLTIDKMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 11 |
| C12 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLSVGHTLVLNCTA TTPLNTRVQMTWSYPDEKNKNASVRRRIDQSNSHANIFY SVLTIDGMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 12 |
| C13 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPLNTRVQMTWSYPDEKNKNASVRRRIDQSNSHANIFY SVLTIDKMQNKDKGLYTCRVRSGPSFKSVNTSVHIYDED | SEQ ID NO: 13 |
| | KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | |
| C14 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPLNTRVQMTWSYPDEKNKNASVRRRIDQSNSHANIFY SVLTIDGMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 14 |
| C15 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDGMTNSDKGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE | SEQ ID NO: 15 |
| | WESNGQPENNYKTTPPVLDSDGSFFLYSKLTVDKSRWQQ GNVFSCSVMHEALHNHYTQKSLSLSPGK | |
| C16 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDGMTNSDQGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | SEQ ID NO: 16 |
| C17 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPLNTRVQMTWSYPDEKNKNASVRRRIDQSNSHANIFY SVLTIDGMTNSDQGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | SEQ ID NO: 17 |
| C19 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA | SEQ ID NO: 18 |
| | TTPSNTRVQMTWSYPDEKNKNASVRRRIDQSNSHANIFYS VLTIDKMQNKDKGLYTCRVRSGPSFKSVNTSVHIYDKAL EDKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPGK | |
| C20 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPANTRVQMTWSYPDEKNKNASVRRRIDQSNSHANIFY SVLTIDKMQNKDKGLYTCRVRSGPSFKSVNTSVHIYDKA LEDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPGK | SEQ ID NO: 19 |
| C25 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPANTRVQMTWSYPDEKNKNASVRRRIDQSNSHANIFY SVLTIDGMTNSDQGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK | SEQ ID NO: 20 |
| | GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | |
| C26 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPANTRVQMTWSYPDEKNKNASVRRRIDQSNSHANIFY SVLTIDGMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 21 |
| C27 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPANTRVQMTWSYPDEKNKNASVRRRIDQSNSHANIFY SVLTIDGMTNSDKGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | SEQ ID NO: 22 |
| C28 | EFGRPFVEMYSEIPEIIHMTEGRELVIPCRVTSPNITVTLKK FPLDTLIPDGKRIIWDSRKGFIISNATYKEIGLLTCEATVNG | SEQ ID NO: 23 |
| | HLYKTNYLTHRQTNTIIDVQISTPSPVTLLRGHTLVLNCTA TTPANTRVQMTWSYPDEKNKNASVRRRIDQSNSHANIFY SVLTIDGMTNSDKGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | |
| C29 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPANTRVQMTWSYPDEKNKNASVRRRIDQSNSHANIFY SVLTIDGMTNSDQGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | SEQ ID NO: 24 |
| C30 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELSVGETLVLNCTA TTPLNTRVQMTWSYPDEKNKNASVRRRIDQSNSHANIFY SVLTIDGMTNSDKGLYTCRVRSGPSFKSVNTSVHIYDEDK THTCPPCPAPELLGGPSVFLFPPKPKDTLMISRTPEVTCVV VDVSHEDPEVKFNWYVDGVEVHNAKTKPREEQYNSTYR | SEQ ID NO: 25 |
| | VVSVLTVLHQDWLNGKEYKCKVSNKALPAPIEKTISKAK GQPREPQVYTLPPSREEMTKNQVSLTCLVKGFYPSDIAVE WESNGQPENNYKTTPPVLDSDGSFFLYSKLTVDKSRWQQ GNVFSCSVMHEALHNHYTQKSLSLSPGK | |
| C32 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSS GDKTHTSPPSPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPG | SEQ ID NO: 26 |
| C34 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSS GEPKSSDKTYTSPPSPAPELLGGPSVFLFPPKPKDTLMISRT PEVTCVVVDVSHEDPEVKFNWYVDGVEVHNAKTKPREE QYNSTYRVVSVLTVLHQDWLNGKEYKCKVSNKALPAPIE KTISKAKGQPREPQVYTLPPSREEMTKNQVSLTCLVKGFY PSDIAVEWESNGQPENNYKTTPPVLDSDGSFFLYSKLTVD KSRWQQGNVFSCSVMHEALHNHYTQKSLSLSPG | SEQ ID NO: 27 |
| C36 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSK VDKKVEPKSSDKTHTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 28 |
| C37 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 29 |
| C39 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSK | SEQ ID NO: 30 |
| | VDKKVEPKSSDTPPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | |
| C41 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSG GGGSGGGGSGGGGSAESKYGPPCPPCPAPELLGGPSVFLF PPKPKDTLMISRTPEVTCVVVDVSHEDPEVKFNWYVDGV EVHNAKTKPREEQYNSTYRVVSVLTVLHQDWLNGKEYK CKVSNKALPAPIEKTISKAKGQPREPQVYTLPPSREEMTK NQVSLTCLVKGFYPSDIAVEWESNGQPENNYKTTPPVLDS DGSFFLYSKLTVDKSRWQQGNVFSCSVMHEALHNHYTQ KSLSLSPG | SEQ ID NO: 31 |
| C43 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSN TGSGGEEKKKEKEKEEQEERSSDKTYTCPPCPAPELLGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALPAPIEKTISKAKGQPREPQVYTLPPSREE | SEQ ID NO: 32 |
| | | |
| C44 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGSN TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPP VLDSDGSFFLYSKLTVDKSRWQQGNVFSCSVMHEALHN HYTQKSLSLSPG | SEQ ID NO: 33 |
| C45 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDEGS SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 34 |
| C46 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDEGS SGGSSGEPKSDSTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 35 |
| C47 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVCLLRGHTLVLNCTA TTPLNTRVQMTWSYPDEKNKNASVRRRIDQSNSHANIFY SVLTIDKMQNKDKGLYTCRVRSGPSFKSVNTSVHIYDGC KVDKKVEPKSSDKTYTCPPCPAPELLGGPSVFLFPPKPKD TLMISRTPEVTCVVVDVSHEDPEVKFNWYVDGVEVHNA KTKPREEQYNSTYRVVSVLTVLHQDWLNGKEYKCKVSN KALPAPIEKTISKAKGQPREPQVYTLPPSREEMTKNQVSLT CLVKGFYPSDIAVEWESNGQPENNYKTTPPVLDSDGSFFL YSKLTVDKSRWQQGNVFSCSVMHEALHNHYTQKSLSLSP G | SEQ ID NO: 36 |
| C48 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPLNTRVQMTWSYPDEKNKNASVRRRIDQSNSHANIFY | SEQ ID NO: 37 |
| | SVLTIDKMQNKDKGLYTCRVRSGPSFKSVNTSVHICDGSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | |
| C49 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPLNTRVQMTWSYPDEKNKNASVRRRIDQSNSHANIFY SVLTIDKMQNKDKGLYTCRVRSGPSFKSVNTSVHIYDCSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 38 |
| C50 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLCGHTLVLNCTA TTPLNTRVQMTWSYPDEKNKNASVRRRIDQSNSHANIFY SVLTIDKMQNKDKGLYTCRVRSGPSFKSVNTSVHICDGSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL | SEQ ID NO: 39 |
| | VKGFYPSDIAVEWESNGQPENNYKTTPPVLDSDGSFFLYS KLTVDKSRWQQGNVFSCSVMHEALHNHYTQKSLSLSPG | |
| C51 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQCKDKGLYTCRVRSGPSFKSVNTSVHICDGSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 40 |
| C52 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGG GGSAEPKAGDKAPPGPPGPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 41 |
| C55 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA | SEQ ID NO: 42 |
| | TTPLNTRVQMTWSYPDEKNKNASVRRRIDQSNSHANIFY SVLTIDKMQNKDKGLYTCRVRSGPSFKSVNTSVHIYDGG GGSAEPKSSDKTYTCPPCPAPELLGGPSVFLFPPKPKDTLM ISRTPEVTCVVVDVSHEDPEVKFNWYVDGVEVHNAKTKP REEQYNSTYRVVSVLTVLHQDWLNGKEYKCKVSNKALP APIEKTISKAKGQPREPQVYTLPPSREEMTKNQVSLTCLV KGFYPSDIAVEWESNGQPENNYKTTPPVLDSDGSFFLYSK LTVDKSRWQQGNVFSCSVMHEALHNHYTQKSLSLSPG | |
| C56 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDGG GGSGGGGSGGGGSGGGGSAEPKSSDKTYTCPPCPAPELL GGPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVYTLP PSREEMTKNQVSLTCLVKGFYPSDIAVEWESNGQPENNY KTTPPVLDSDGSFFLYSKLTVDKSRWQQGNVFSCSVMHE ALHNHYTQKSLSLSPG | SEQ ID NO: 43 |
| C61 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSS GDATPTSPPSPAPELLGGPSVFLFPPKPKDTLMISRTPEVTC VVVDVSHEDPEVKFNWYVDGVEVHNAKTKPREEQYNST | SEQ ID NO: 44 |
| | YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | |
| C62 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSK VDKKVEPKSSDTPPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 45 |
| C63 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSG GGGSAEPKAGDATPPTCPPCPPCPAPELLGGPSVFLFPPKP KDTLMISRTPEVTCVVVDVSHEDPEVKFNWYVDGVEVH NAKTKPREEQYNSTYRVVSVLTVLHQDWLNGKEYKCKV SNKALPAPIEKTISKAKGQPREPQVYTLPPSRDELTKNQVS LTCLVKGFYPSDIAVEWESNGQPENNYKTTPPVLDSDGSF FLYSKLTVDKSRWQQGNVFSCSVMHEALHNHYTQKSLSL SPG | SEQ ID NO: 46 |
| C64 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSG GGGSGGGGSGGGGSAESKYGPPCPPCPAPELLGGPSVFLF PPKPKDTLMISRTPEVTCVVVDVSHEDPEVKFNWYVDGV EVHNAKTKPREEQYNSTYRVVSVLTVLHQDWLNGKEYK CKVSNKALPAPIEKTISKAKGQPREPQVYTLPPSRDELTKN QVSLTCLVKGFYPSDIAVEWESNGQPENNYKTTPPVLDSD GSFFLYSKLTVDKSRWQQGNVFSCSVMHEALHNHYTQK SLSLSPG | SEQ ID NO: 47 |
| C65 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSN TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL TKNQVSLTCLVKGFYPSDIAVEWESNGQPENNYKTTPPVL DSDGSFFLYSKLTVDKSRWQQGNVFSCSVMHEALHNHY TQKSLSLSPG | SEQ ID NO: 48 |
| C66 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA | SEQ ID NO: 49 |
| | TTPANTRVQMTWSYPDEKNKNASVRRRIDQSNSHANIFY SVLTIDGMTNSDKGLYTCRVRSGPSFKSVNTSVHIYDCGS SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | |
| C67 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPANTRVQMTWSYPDEKNKNASVRRRIDQSNSHANIFY SVLTIDGMTNSDKGLYTCRVRSGPSFKSVNTSVHIYDCSK VDKKVEPKSSDKTYTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 50 |
| C68 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPSNTRVQMTWSYPDEKNKNASVRRRIDQSNSHANIFYS VLTIDGMTNSDKGLYTCRVRSGPSFKSVNTSVHIYDCSKV DKKVEPKSSDTPPTCPPCPAPELLGGPSVFLFPPKPKDTLM ISRTPEVTCVVVDVSHEDPEVKFNWYVDGVEVHNAKTKP REEQYNSTYRVVSVLTVLHQDWLNGKEYKCKVSNKALP | SEQ ID NO: 51 |
| | APIEKTISKAKGQPREPQVYTLPPSRDELTKNQVSLTCLVK GFYPSDIAVEWESNGQPENNYKTTPPVLDSDGSFFLYSKL TVDKSRWQQGNVFSCSVMHEALHNHYTQKSLSLSPG | |
| C69 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPDNTRVQMTWSYPDEKNKNASVRRRIDQSNSHANIFY SVLTIDGMTNSDKGLYTCRVRSGPSFKSVNTSVHIYDCSK VDKKVEPKSSDTPPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 52 |
| C70 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPSNTRVQMTWSYPDEKNKNASVRRRIDQSNSHANIFYS VLTIDGMTNSDKGLYTCRVRSGPSFKSVNTSVHIYDCSGG GGSGGGGSGGGGSAESKYGPPCPPCPAPELLGGPSVFLFP PKPKDTLMISRTPEVTCVVVDVSHEDPEVKFNWYVDGVE VHNAKTKPREEQYNSTYRVVSVLTVLHQDWLNGKEYKC KVSNKALPAPIEKTISKAKGQPREPQVYTLPPSRDELTKNQ VSLTCLVKGFYPSDIAVEWESNGQPENNYKTTPPVLDSDG SFFLYSKLTVDKSRWQQGNVFSCSVMHEALHNHYTQKSL SLSPG | SEQ ID NO: 53 |
| C71 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPDNTRVQMTWSYPDEKNKNASVRRRIDQSNSHANIFY SVLTIDGMTNSDKGLYTCRVRSGPSFKSVNTSVHIYDCSG GGGSGGGGSGGGGSAESKYGPPCPPCPAPELLGGPSVFLF PPKPKDTLMISRTPEVTCVVVDVSHEDPEVKFNWYVDGV EVHNAKTKPREEQYNSTYRVVSVLTVLHQDWLNGKEYK CKVSNKALPAPIEKTISKAKGQPREPQVYTLPPSRDELTKN QVSLTCLVKGFYPSDIAVEWESNGQPENNYKTTPPVLDSD GSFFLYSKLTVDKSRWQQGNVFSCSVMHEALHNHYTQK SLSLSPG | SEQ ID NO: 54 |
| C72 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPSNTRVQMTWSYPDEKNKNASVRRRIDQSNSHANIFYS VLTIDGMTNSDKGLYTCRVRSGPSFKSVNTSVHIYDCGSS GGSSGEPKSDATPTCPPCPAPELLGGPSVFLFPPKPKDTLM ISRTPEVTCVVVDVSHEDPEVKFNWYVDGVEVHNAKTKP REEQYNSTYRVVSVLTVLHQDWLNGKEYKCKVSNKALP APIEKTISKAKGQPREPQVYTLPPSRDELTKNQVSLTCLVK GFYPSDIAVEWESNGQPENNYKTTPPVLDSDGSFFLYSKL TVDKSRWQQGNVFSCSVMHEALHNHYTQKSLSLSPG | SEQ ID NO: 55 |
| C73 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPDNTRVQMTWSYPDEKNKNASVRRRIDQSNSHANIFY | SEQ ID NO: 56 |
| | SVLTIDGMTNSDKGLYTCRVRSGPSFKSVNTSVHIYDCGS SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | |
| C74 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPDNTRVQMTWSYPDEKNKNASVRRRIDQSNSHANIFY SVLTIDGMTNSDKGLYTCRVRSGPSFKSVNTSVHIYDCSS GDATPTSPPSPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 57 |
| C75 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVELCVGETLVLNCTA TTPDNTRVQMTWSYPDEKNKNASVRRRIDQSNSHANIFY SVLTIDGMTNSDKGLYTCRVRSGPSFKSVNTSVHIYDCSN TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL | SEQ ID NO: 58 |
| | TKNQVSLTCLVKGFYPSDIAVEWESNGQPENNYKTTPPVL DSDGSFFLYSKLTVDKSRWQQGNVFSCSVMHEALHNHY TQKSLSLSPG | |
| C76 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCSS GDATPTSPPSPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 59 |
| C77 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCSK VDKKVEPKSSDTPPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 60 |
| C78 | EFGRPFVEMYSEIPEIIHMTEGRELVIPCRVTSPNITVTLKK FPLDTLIPDGKRIIWDSRKGFIISNATYKEIGLLTCEATVNG | SEQ ID NO: 61 |
| | HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCSG GGGSAEPKAGDATPPTCPPCPPCPAPELLGGPSVFLFPPKP KDTLMISRTPEVTCVVVDVSHEDPEVKFNWYVDGVEVH NAKTKPREEQYNSTYRVVSVLTVLHQDWLNGKEYKCKV SNKALPAPIEKTISKAKGQPREPQVYTLPPSRDELTKNQVS LTCLVKGFYPSDIAVEWESNGQPENNYKTTPPVLDSDGSF FLYSKLTVDKSRWQQGNVFSCSVMHEALHNHYTQKSLSL SPG | |
| C79 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCSG GGGSGGGGSGGGGSAESKYGPPCPPCPAPELLGGPSVFLF PPKPKDTLMISRTPEVTCVVVDVSHEDPEVKFNWYVDGV EVHNAKTKPREEQYNSTYRVVSVLTVLHQDWLNGKEYK CKVSNKALPAPIEKTISKAKGQPREPQVYTLPPSRDELTKN QVSLTCLVKGFYPSDIAVEWESNGQPENNYKTTPPVLDSD GSFFLYSKLTVDKSRWQQGNVFSCSVMHEALHNHYTQK SLSLSPG | SEQ ID NO: 62 |
| C80 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCSN | SEQ ID NO: 63 |
| | TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL TKNQVSLTCLVKGFYPSDIAVEWESNGQPENNYKTTPPVL DSDGSFFLYSKLTVDKSRWQQGNVFSCSVMHEALHNHY TQKSLSLSPG | |
| C81 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPANTRVQMTWSYPDEKNKNASVRRRIDQSNSHANIFY SVLTIDGMTNSDQGLYTCRVRSGPSFKSVNTSVHIYDCGS SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 64 |
| C82 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCSS GDATPTSPPSPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA | SEQ ID NO: 65 |
| | VEWESNGQPENNYKTTPPVLDSDGSFFLYSKLTVDKSRW QQGNVFSCSVMHEALHNHYTQKSLSLSPG | |
| C83 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCSK VDKKVEPKSSDTPPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 66 |
| C84 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCSG GGGSAEPKAGDATPPTCPPCPPCPAPELLGGPSVFLFPPKP KDTLMISRTPEVTCVVVDVSHEDPEVKFNWYVDGVEVH NAKTKPREEQYNSTYRVVSVLTVLHQDWLNGKEYKCKV SNKALPAPIEKTISKAKGQPREPQVYTLPPSRDELTKNQVS LTCLVKGFYPSDIAVEWESNGQPENNYKTTPPVLDSDGSF FLYSKLTVDKSRWQQGNVFSCSVMHEALHNHYTQKSLSL SPG | SEQ ID NO: 67 |
| C85 | EFGRPFVEMYSEIPEIIHMTEGRELVIPCRVTSPNITVTLKK FPLDTLIPDGKRIIWDSRKGFIISNATYKEIGLLTCEATVNG | SEQ ID NO: 68 |
| | HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCSG GGGSGGGGSGGGGSAESKYGPPCPPCPAPELLGGPSVFLF PPKPKDTLMISRTPEVTCVVVDVSHEDPEVKFNWYVDGV EVHNAKTKPREEQYNSTYRVVSVLTVLHQDWLNGKEYK CKVSNKALPAPIEKTISKAKGQPREPQVYTLPPSRDELTKN QVSLTCLVKGFYPSDIAVEWESNGQPENNYKTTPPVLDSD GSFFLYSKLTVDKSRWQQGNVFSCSVMHEALHNHYTQK SLSLSPG | |
| C86 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCSN TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL TKNQVSLTCLVKGFYPSDIAVEWESNGQPENNYKTTPPVL DSDGSFFLYSKLTVDKSRWQQGNVFSCSVMHEALHNHY TQKSLSLSPG | SEQ ID NO: 69 |
| C87 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLCRGHTLVLNCTA TTPDNTRVQMTWSYPDEKNKNASVRRRIDQSNSHANIFY SVLTIDGMTNSDQGLYTCRVRSGPSFKSVNTSVHIYDCGS | SEQ ID NO: 70 |
| | SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | |
| C88 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDK ALEDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKT ISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPG | SEQ ID NO: 71 |
| C89 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA | SEQ ID NO: 72 |
| | VEWESNGQPENNYKTTPPVLDSDGSFFLYSKLTVDKSRW QQGNVFSCSVMHEALHNHYTQKSLSLSPG | |
| C90 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDLED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 73 |
| C91 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSREEMTKNQVSLTCLVKGFYPSDIAV EWESNGQPENNYKTTPPVLDSDGSFFLYSKLTVDKSRWQ QGNVFSCSVMHEALHNHYTQKSLSLSPGK | SEQ ID NO: 74 |
| C95 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA | SEQ ID NO: 75 |
| | TTPLNTRVQMTWSYPDEKNKNASVRRRIDQSNSHANIFY SVLTIDKMQNKDKGLYTCRVRSGPSFKSVNTSVHIYDKA LEDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTIS KAKGQPREPQVYTLPPSRDELTKNQVSLTCLVKGFYPSDI AVEWESNGQPENNYKTTPPVLDSDGSFFLYSKLTVDKSR WQQGNVFSCSVMHEALHNHYTQKSLSLSPGK | |
| C96 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPSPVTLLRGHTLVLNCTA TTPLNTRVQMTWSYPDEKNKNASVRRRIDQSNSHANIFY SVLTIDKMQNKDKGLYTCRVRSGPSFKSVNTSVHIYDKA LEDKTHTCPPCPAPELLGGPSVFLFPPKPKDTLMISRTPEV TCVVVDVSHEDPEVKFNWYVDGVEVHNAKTKPREEQYN STYRVVSVLTVLHQDWLNGKEYKCKVSNKALPAPIEKTIS KAKGQPREPQVYTLPPSREEMTKNQVSLTCLVKGFYPSDI AVEWESNGQPENNYKTTPPVLDSDGSFFLYSKLTVDKSR WQQGNVFSCSVMHEALHNHYTQKSLSLSPG | SEQ ID NO: 76 |
| C97 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLSVGHTLVLNCT ATTPLNTRVQMTWSYPDEKNKRASVRRRIDQSNSHANIF YSVLTIDKMQNKDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK | SEQ ID NO: 77 |
| | AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | |
| C98 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELSVGETLVLNCTA TTPLNTRVQMTWSYPDEKNKRASVRRRIDQSNSHANIFYS VLTIDKMQNKDKGLYTCRVRSGPSFKSVNTSVHIYDLED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPG | SEQ ID NO: 78 |
| C99 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPANTRVQMTWSYPDEKNKRASVRRRIDQSNSHANIF YSVLTIDKMQNKDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 79 |
| C100 | EFGRPFVEMYSEIPEIIHMTEGRELVIPCRVTSPNITVTLKK FPLDTLIPDGKRIIWDSRKGFIISNATYKEIGLLTCEATVNG | SEQ ID NO: 80 |
| | HLYKTNYLTHRQTNTIIDVQISTPRPVKLLRGHTLVLNCT ATTPLNTRVQMTWSYPDEKNKRASVRRRIDQSNSHANIF YSVLTIDGMTNSDQGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | |
| C101 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLSVGHTLVLNCT ATTPANTRVQMTWSYPDEKNKRASVRRRIDQSNSHANIF YSVLTIDKMQNKDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 81 |
| C102 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPANTRVQMTWSYPDEKNKRASVRRRIDQSNSHANIF YSVLTIDGMTNSDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST | SEQ ID NO: 82 |
| | YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | |
| C103 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPANTRVQMTWSYPDEKNKRASVRRRIDQSNSHANIF YSVLTIDGMTNSDQGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 83 |
| C104 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELSVGETLVLNCTA TTPLNTRVQMTWSYPDEKNKRASVRRRIDQSNSHANIFYS VLTIDGMTNSDQGLYTCRVRSGPSFKSVNTSVHIYDLEDK THTCPPCPAPELLGGPSVFLFPPKPKDTLMISRTPEVTCVV VDVSHEDPEVKFNWYVDGVEVHNAKTKPREEQYNSTYR VVSVLTVLHQDWLNGKEYKCKVSNKALPAPIEKTISKAK GQPREPQVYTLPPSRDELTKNQVSLTCLVKGFYPSDIAVE WESNGQPENNYKTTPPVLDSDGSFFLYSKLTVDKSRWQQ GNVFSCSVMHEALHNHYTQKSLSLSPG | SEQ ID NO: 84 |
| C105 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELSVGETLVLNCTA TTPANTRVQMTWSYPDEKNKRASVRRRIDQSNSHANIFY SVLTIDGMTNSDQGLYTCRVRSGPSFKSVNTSVHIYDLED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPG | SEQ ID NO: 85 |
| C106 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELSVGETLVLNCTA TTPANTRVQMTWSYPDEKNKRASVRRRIDQSNSHANIFY SVLTIDGMQNKDKGLYTCRVRSGPSFKSVNTSVHIYDLED KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPG | SEQ ID NO: 86 |
| C107 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELSVGETLVLNCTA TTPANTRVQMTWSYPDEKNKRASVRRRIDQSNSHANIFY SVLTIDGMTNSDKGLYTCRVRSGPSFKSVNTSVHIYDLED | SEQ ID NO: 87 |
| | KTHTCPPCPAPELLGGPSVFLFPPKPKDTLMISRTPEVTCV VVDVSHEDPEVKFNWYVDGVEVHNAKTKPREEQYNSTY RVVSVLTVLHQDWLNGKEYKCKVSNKALPAPIEKTISKA KGQPREPQVYTLPPSRDELTKNQVSLTCLVKGFYPSDIAV EWESNGQPENNYKTTPPVLDSDGSFFLYSKLTVDKSRWQ QGNVFSCSVMHEALHNHYTQKSLSLSPG | |
| C108 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDGS SGDKTHTSPPSPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSRDELTKNQVSLTCLVKGFYPSDI AVEWESNGQPENNYKTTPPVLDSDGSFFLYSKLTVDKSR WQQGNVFSCSVMHEALHNHYTQKSLSLSPG | SEQ ID NO: 88 |
| C109 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDGS KVDKKVEPKSSDKTHTCPPCPAPELLGGPSVFLFPPKPKD TLMISRTPEVTCVVVDVSHEDPEVKFNWYVDGVEVHNA KTKPREEQYNSTYRVVSVLTVLHQDWLNGKEYKCKVSN KALPAPIEKTISKAKGQPREPQVYTLPPSRDELTKNQVSLT CLVKGFYPSDIAVEWESNGQPENNYKTTPPVLDSDGSFFL | SEQ ID NO: 89 |
| | YSKLTVDKSRWQQGNVFSCSVMHEALHNHYTQKSLSLSP G | |
| C111 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDE GSSGGSSGEPKSDATPTCPPCPAPELLGGPSVFLFPPKPKD TLMISRTPEVTCVVVDVSHEDPEVKFNWYVDGVEVHNA KTKPREEQYNSTYRVVSVLTVLHQDWLNGKEYKCKVSN KALPAPIEKTISKAKGQPREPQVYTLPPSRDELTKNQVSLT CLVKGFYPSDIAVEWESNGQPENNYKTTPPVLDSDGSFFL YSKLTVDKSRWQQGNVFSCSVMHEALHNHYTQKSLSLSP G | SEQ ID NO: 90 |
| C112 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLCRGHTLVLNCT ATTPLNTRVQMTWSYPDEKNKRASVRRRIDQSNSHANIF YSVLTIDKMQNKDKGLYTCRVRSGPSFKSVNTSVHIYDCS KVDKKVEPKSSDKTYTCPPCPAPELLGGPSVFLFPPKPKD TLMISRTPEVTCVVVDVSHEDPEVKFNWYVDGVEVHNA KTKPREEQYNSTYRVVSVLTVLHQDWLNGKEYKCKVSN KALPAPIEKTISKAKGQPREPQVYTLPPSRDELTKNQVSLT CLVKGFYPSDIAVEWESNGQPENNYKTTPPVLDSDGSFFL YSKLTVDKSRWQQGNVFSCSVMHEALHNHYTQKSLSLSP G | SEQ ID NO: 91 |
| C113 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELCVGETLVLNCTA TTPANTRVQMTWSYPDEKNKRASVRRRIDQSNSHANIFY SVLTIDGMTNSDKGLYTCRVRSGPSFKSVNTSVHIYDCSN TGSGGEEKKKEKEKEEQEERSSDTPPTCPPCPAPELLGGPS VFLFPPKPKDTLMISRTPEVTCVVVDVSHEDPEVKFNWYV DGVEVHNAKTKPREEQYNSTYRVVSVLTVLHQDWLNGK EYKCKVSNKALPAPIEKTISKAKGQPREPQVYTLPPSRDEL TKNQVSLTCLVKGFYPSDIAVEWESNGQPENNYKTTPPVL DSDGSFFLYSKLTVDKSRWQQGNVFSCSVMHEALHNHY TQKSLSLSPG | SEQ ID NO: 92 |
| C114 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVELCVGETLVLNCTA TTPDNTRVQMTWSYPDEKNKRASVRRRIDQSNSHANIFY SVLTIDGMTNSDKGLYTCRVRSGPSFKSVNTSVHIYDCGS SGGSSGEPKSDATPTCPPCPAPELLGGPSVFLFPPKPKDTL MISRTPEVTCVVVDVSHEDPEVKFNWYVDGVEVHNAKT KPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYS KLTVDKSRWQQGNVFSCSVMHEALHNHYTQKSLSLSPG | SEQ ID NO: 93 |
| C115 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF | SEQ ID NO: 94 |
| | YSVLTIDKMQNKDKGLYTCRVRSGPSFKSVNTSVHIYDK AAPELLGGPSVFLFPPKPKDTLMISRTPEVTCVVVDVSHE DPEVKFNWYVDGVEVHNAKTKPREEQYNSTYRVVSVLT VLHQDWLNGKEYKCKVSNKALPAPIEKTISKAKGQPREP QVYTLPPSRDELTKNQVSLTCLVKGFYPSDIAVEWESNGQ PENNYKTTPPVLDSDGSFFLYSKLTVDKSRWQQGNVFSCS VMHEALHNHYTQKSLSLSPG | |
| C116 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDK AGSSGGSSGGSSGGSSGGSSGGSSGGSSGGSSGGSSGGSS GDKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTIS KAKGQPREPQVYTLPPSRDELTKNQVSLTCLVKGFYPSDI AVEWESNGQPENNYKTTPPVLDSDGSFFLYSKLTVDKSR WQQGNVFSCSVMHEALHNHYTQKSLSLSPG | SEQ ID NO: 95 |
| C117 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDK AGGGGSGGGGSGGGGSGGGGSGGGGSGGGGSGGGGSG GGGSDKTHTCPPCPAPELLGGPSVFLFPPKPKDTLMISRTP EVTCVVVDVSHEDPEVKFNWYVDGVEVHNAKTKPREEQ | SEQ ID NO: 96 |
| | YNSTYRVVSVLTVLHQDWLNGKEYKCKVSNKALPAPIEK TISKAKGQPREPQVYTLPPSRDELTKNQVSLTCLVKGFYP SDIAVEWESNGQPENNYKTTPPVLDSDGSFFLYSKLTVDK SRWQQGNVFSCSVMHEALHNHYTQKSLSLSPG | |
| C118 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPLNTRVQMTWSYPDEKNKRASVRRRIDQSNSHANIF YSVLTIDKMQNKDKGLYTCRVRSGPSFKSVNTSVHIYDK AGGGGSGGGGSGGGGSGGGGSGGGGSGGGGSDKTHTCP PCPAPELLGGPSVFLFPPKPKDTLMISRTPEVTCVVVDVSH EDPEVKFNWYVDGVEVHNAKTKPREEQYNSTYRVVSVL TVLHQDWLNGKEYKCKVSNKALPAPIEKTISKAKGQPRE PQVYTLPPSRDELTKNQVSLTCLVKGFYPSDIAVEWESNG QPENNYKTTPPVLDSDGSFFLYSKLTVDKSRWQQGNVFS CSVMHEALHNHYTQKSLSLSPG | SEQ ID NO: 97 |
| C119 | EFGRPFVEMYSEIPEIIHMTEGRELVIPCRVTSPNITVTLKK FPLDTLIPDGKRIIWDSRKGFIISNATYKEIGLLTCEATVNG HLYKTNYLTHRQTNTIIDVQISTPRPVKLLRGHTLVLNCT ATTPDNTRVQMTWSYPDEKNKRASVRRRIDQSNSHANIF YSVLTIDKMQNKDKGLYTCRVRSGPSFKSVNTSVHIYDLE DKTHTCPPCPAPELLGGPSVFLFPPKPKDTLMISRTPEVTC VVVDVSHEDPEVKFNWYVDGVEVHNAKTKPREEQYNST YRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPG | SEQ ID NO: 98 |
| FC region | APELLGGPSVFLFPPKPKDTLMISRTPEVTCVVVDVSHEDP EVKFNWYVDGVEVHNAKTKPREEQYNSTYRVVSVLTVL HQDWLNGKEYKCKVSNKALPAPIEKTISKAKGQPREPQV YTLPPSRDELTKNQVSLTCLVKGFYPSDIAVEWESNGQPE NNYKTTPPVLDSDGSFFLYSKLTVDKSRWQQGNVFSCSV MHEALHNHYTQKSLSLSPGK | SEQ ID NO: 99 |
| Ig-like domains D2 and D3 of VEGFR1 | GRPFVEMYSEIPEIIHMTEGRELVIPCRVTSPNITVTLKKFP LDTLIPDGKRIIWDSRKGFIISNATYKEIGLLTCEATVNGHL YKTNYLTHRQTNTIIDVQISTPRPVKLLRGHTLVLNCTATT PLNTRVQMTWSYPDEKNKRASVRRRIDQSNSHANIFYSV LTIDKMQNKDKGLYTCRVRSGPSFKSVNTSVHIYDKA | SEQ ID NO: 100 |
| Zaltrap | SDTGRPFVEMYSEIPEIIHMTEGRELVIPCRVTSPNITVTLK KFPLDTLIPDGKRIIWDSRKGFIISNATYKEIGLLTCEATVN GHLYKTNYLTHRQTNTIIDVVLSPSHGIELSVGEKLVLNCT ARTELNVGIDFNWEYPSSKHQHKKLVNRDLKTQSGSEMK KFLSTLTIDGVTRSDQGLYTCAASSGLMTKKNSTFVRVHE KLEDKTHTCPPCPAPELLGGPSVFLFPPKPKDTLMISRTPE VTCVVVDVSHEDPEVKFNWYVDGVEVHNAKTKPREEQY NSTYRVVSVLTVLHQDWLNGKEYKCKVSNKALPAPIEKT ISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPG | SEQ ID NO: 101 |

### [Example 3] Stability evaluation in a blood-simulating environment

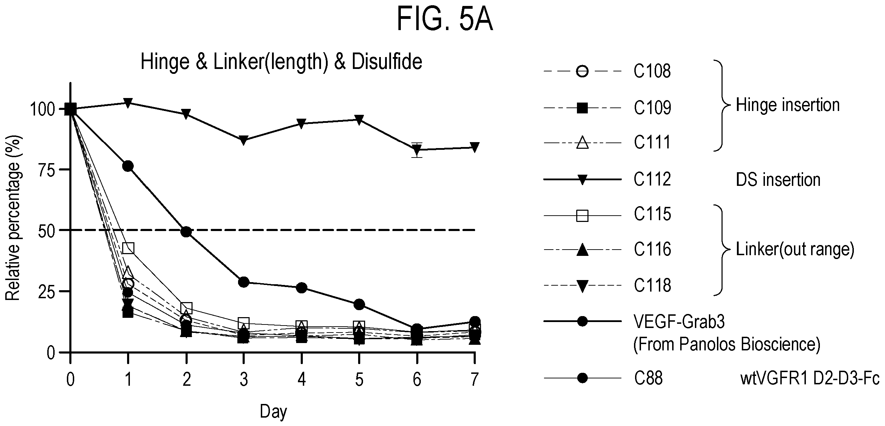

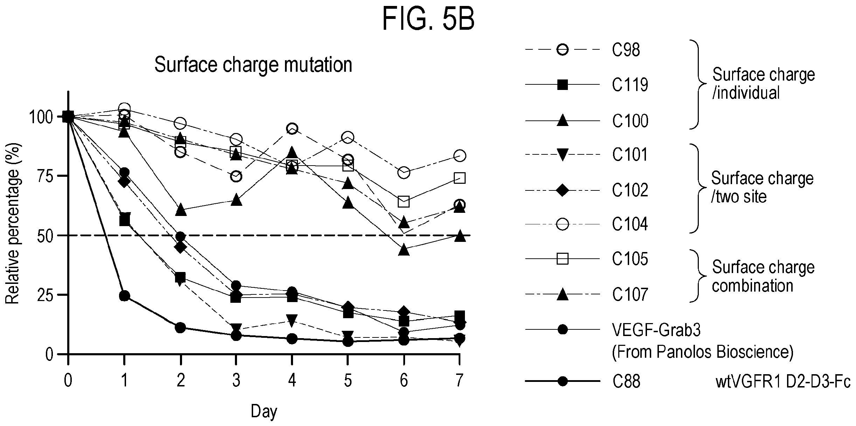

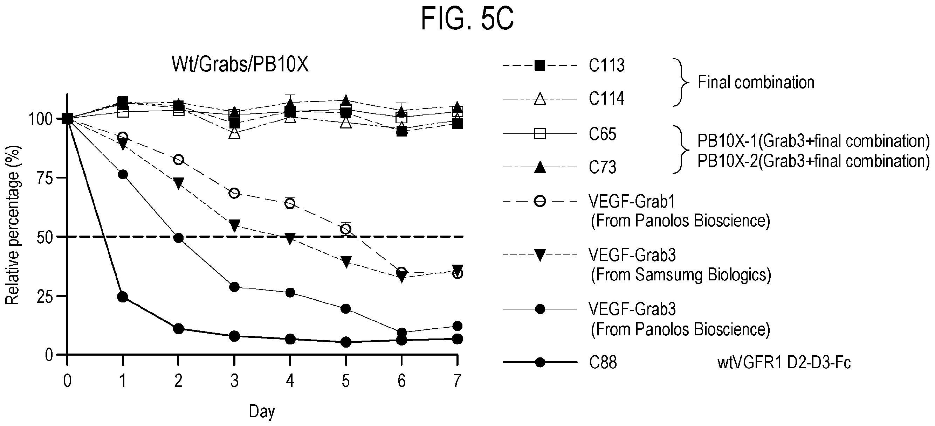

In an indirect analysis of the *in vivo* stability of a modified fusion protein of the present disclosure, the concentration of protein variants remaining at specific times after reaction with blank serum collected from rats was measured through ELISA. VEGF-Grab3, a modified fusion protein, and Zaltrap (SANOFI; SEQ ID NO: 101) were each mixed with rat blank serum to a final concentration of 10 µg/ml and stored in an incubator at 37°C at 168 hours, 96 hours, 48 hours, 24 hours, 4 hours, 2 hours, 1 hour, and 0 hours before the test initiation time, respectively. The day before the test, human VEGF-A (R&D systems) was coated on the plates through overnight incubation at 4°C and washed with 0.1% PBS-T washing buffer to remove insufficiently bound human VEGF-A. Thereafter, a blocking process was performed by treating with 0.1% PBS-T containing 5% skim milk for 1 hour at room temperature. Standards used for comparison of concentrations were prepared by serial dilution of VEGF-Grab3 and Zaltrap each in a concentration range of 50 to 0.39 µg/ml. A serial dilution of a standard and the protein-serum mixtures prepared at different time points were diluted 200-fold in blocking buffer, and then 100 µl each was added to wells of the plate and reacted at room temperature for 2 hours. Thereafter, all of the reaction solution in the wells was removed, and the plate was washed using a washing buffer. The detection antibody, HRP Goat anti-human IgG Fc Cross-Absorbed Secondary antibody (Invitrogen), was reacted at room temperature for 1 hour, and then washed to remove unreacted detection antibody. Then, 100 µl of 3, 3', 5,5'-tetramethylbenzidine (TMB) solution (Sigma) was added to each well and reacted at room temperature for 10 minutes, and then the reaction was stopped by adding a stop solution (Sigma). Absorbance was measured at 450 nm using a plate reader. A standard curve was plotted based on the measured absorbance of the standards (VEGF-Grab3 and Zaltrap), and the remaining amount of the modified fusion protein was calculated and compared on a relative basis. From among the calculated results, the concentration at 168 hours (after 7 days) as compared to the concentration at 0 hours was calculated, and the relative absorbance % is shown in Table 34, Table 35, and FIG. 5.

**Table 34**

| Protein code | Relative absorbance of variant % (after 7 days) |
|---|---|
| C88 | 6.6 |
| VEGF-Grab3 (from Panolos Bioscience) | 12.2 |
| VEGF-Grab3 (from Samsung Biologics) | 35.5 |
| VEGF-Grab1(from Panolos Bioscience) | 34.4 |
| C98 | 62.7 |
| C119 | 16.1 |
| C100 | 49.9 |
| C101 | 5.5 |
| C102 | 13.4 |
| C104 | 83.3 |
| C105 | 74.0 |
| C107 | 61.6 |
| C108 | 8.5 |
| C109 | 6.7 |
| C111 | 8.6 |
| C112 | 84.0 |
| C113 | 97.7 |
| C114 | 99.2 |
| C115 | 8.8 |
| C116 | 5.8 |
| C118 | 6.9 |
| C65 | 103.1 |
| C73 | 105.1 |

**Table 35**

| Protein code | Relative absorbance of variant % (after 7 days) |
|---|---|
| VEGF-Grab3 (from Panolos Bioscience) | 20.26 |
| VEGF-Grab3 (from Samsung Biologics) | 45.52 |
| C61 | 98.35 |
| C62 | 98.69 |
| C63 | 99.34 |
| C64 | 97.43 |
| C65 | 97.42 |
| C66 | 98.04 |
| C67 | 99.99 |
| C68 | 97.17 |
| C69 | 97.19 |
| C70 | 97.83 |
| C71 | 101.48 |
| C72 | 101.28 |
| C73 | 101.4 |
| Zaltrap | 99.1 |

According to Table 34 and FIG. 5, modified fusion proteins of the present disclosure showed superior serum stability compared to the VEGFR-wild type protein. Variant C112 having a disulfide bond introduced showed significantly better serum stability than variant C109, which has the same linker sequence but no disulfide bond introduced (6.7%). Variants C115, C116, and C118, which have linker sequences that deviate from the linker length of a linker variant according to an embodiment of the present disclosure, did not show superior serum stability compared to the VEGFR-wild type protein.

According to Table 35, modified fusion proteins of the present disclosure showed serum stability exceeding about 97%, thus demonstrated to have a serum stability equivalent to or better than that of Zaltrap^{®}, a commercially available drug. In all of these, no protein degradation pattern was observed within the experimental period (7 days). From these results, it can be seen that a modified fusion protein of the present disclosure has excellent stability *in vivo* and, when compared to Zaltrap^{®}, has stability equivalent to that of a pharmaceutical product. It is indirectly predicted that when the modified fusion protein of the present disclosure is introduced into the body, it may affect efficacy by improving the persistence time of the protein.

### [Example 4] Isoelectric point analysis

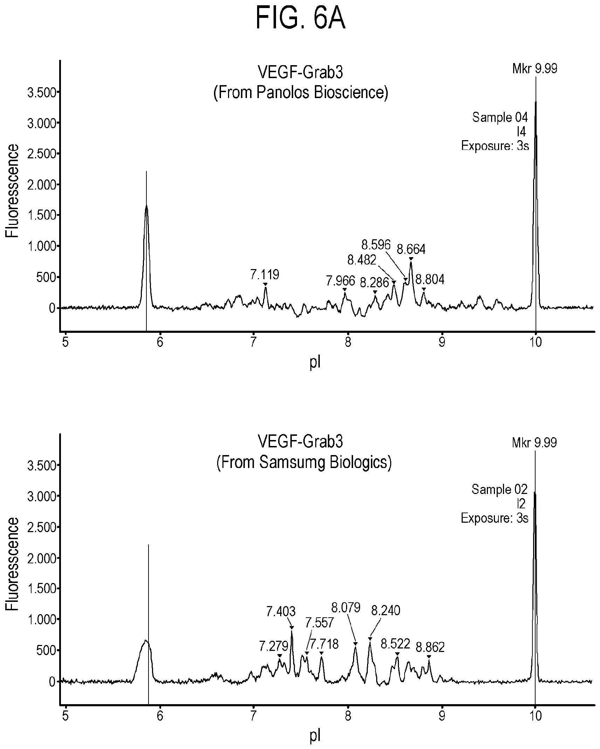

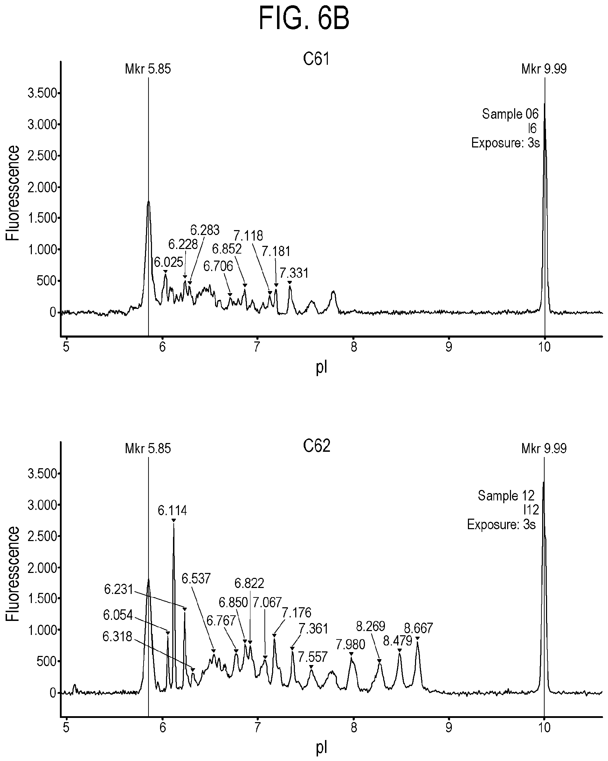

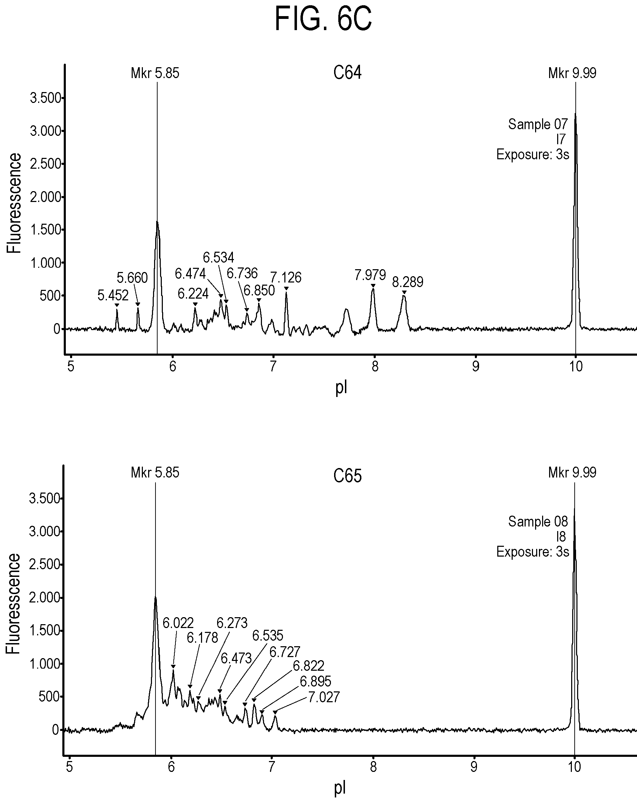

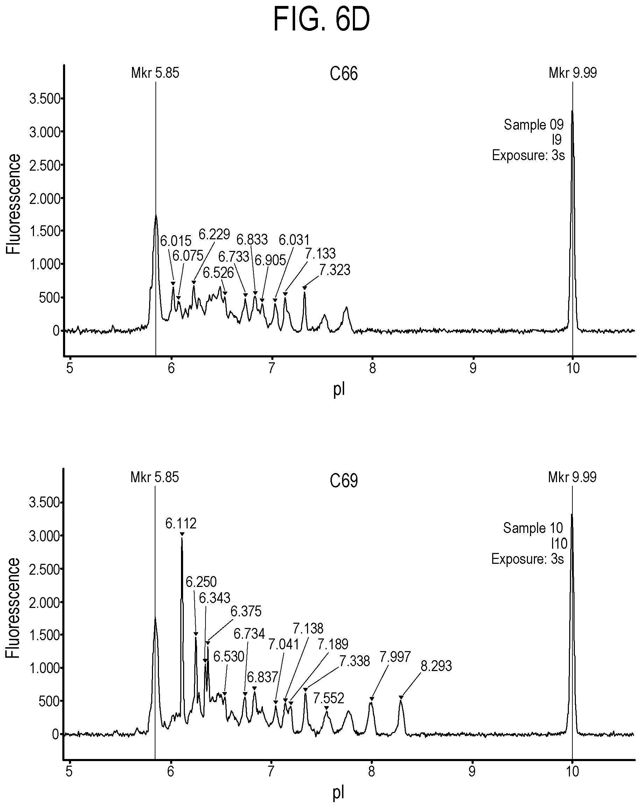

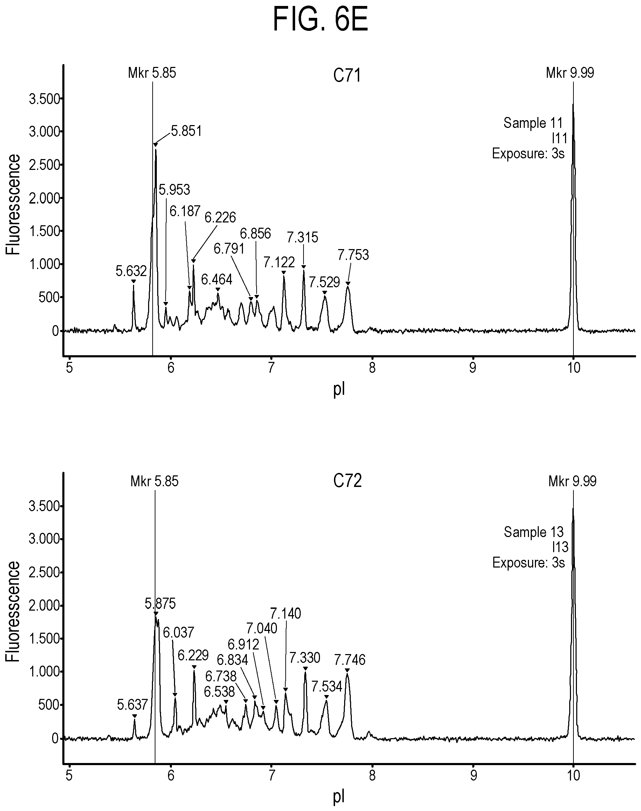

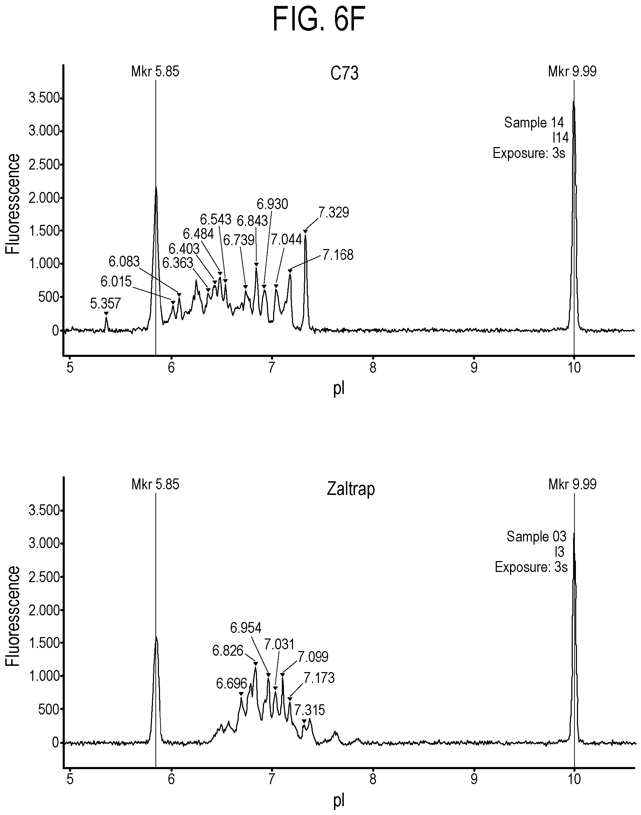

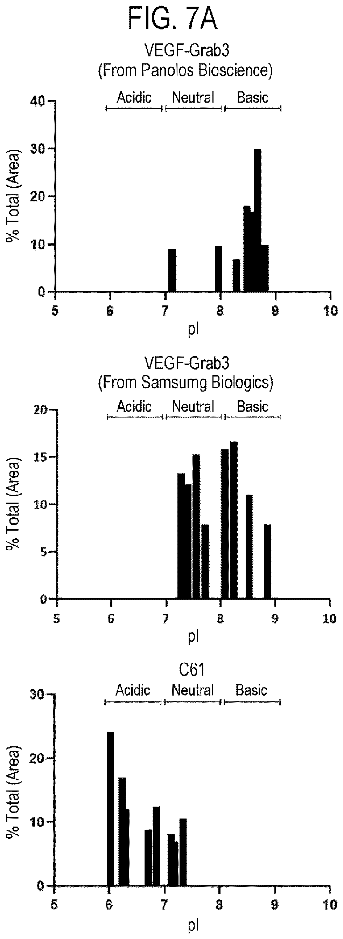

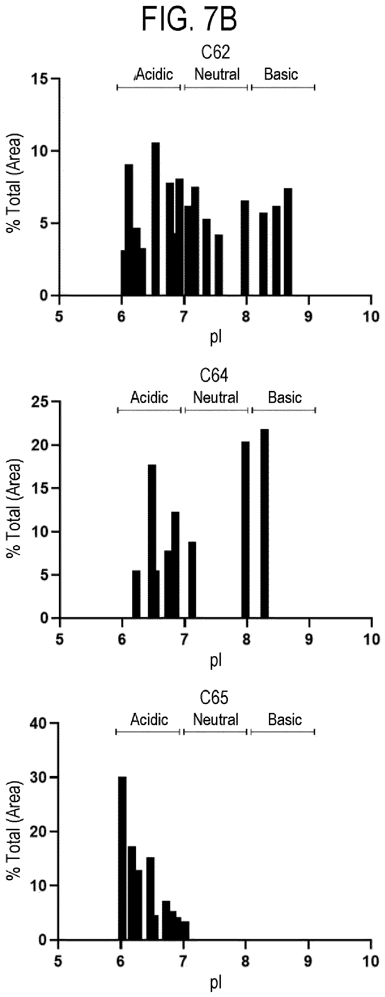

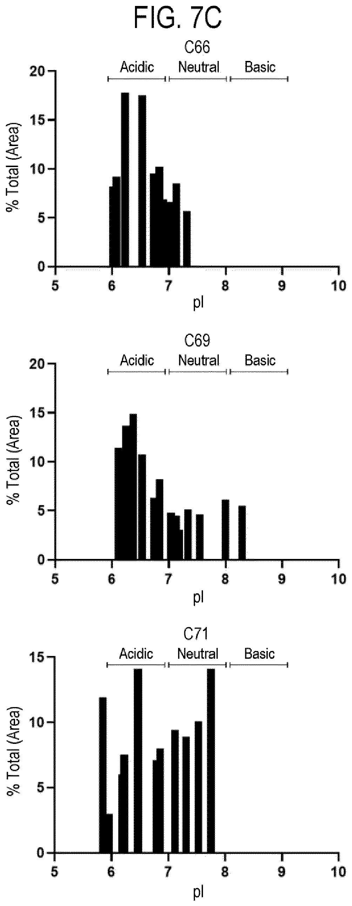

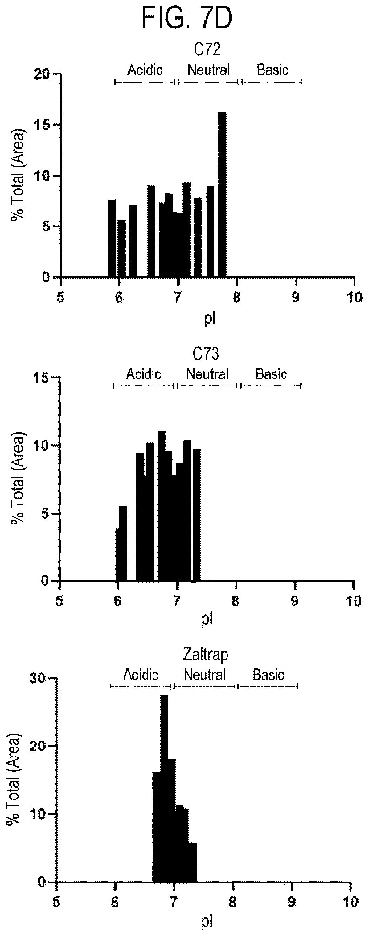

The isoelectric points of VEGF-Grab3 and modified fusion proteins of the present disclosure were analyzed using icIEF (imaged capillary isoelectric focusing). 40 µl of a modified fusion protein diluted to a concentration of 2 mg/ml and 160 µl of pre-prepared Master mix solution (SERVALYT^{™} + low pI marker (5.85) (ProteinSimple) + high pI marker (9.99) (ProteinSimple) + 1% methyl cellulose (ProteinSimple) + DW + 500 mM arginine (Sigma) + 200 mM iminodiacetic acid (Sigma) + 10 M urea (Sigma)) were admixed, centrifuged to remove precipitates and air bubbles, and then inserted into each well of the instrument (Maurice, ProteinSimple). 2 ml each of Catholyte solution and Anolyte solution were placed in the OH- and H+ positions of the cartridge, and the cartridge was mounted on the instrument. Thereafter, isoelectric points were measured according to the manual of the instrument. Since the measured pI appears not as a single peak but as multiple peaks in a certain range, the pI distribution of a protein was defined by dividing it into three sections: acidic (pI 6-7), neutral (pI 7-8), and basic (pI 8<). The sections with the highest percentage distribution were compared. The results are shown in Table 36 and FIGs. 6 and 7.

**Table 36**

| Total % | Acidic | Neutral | Basic | Sum |
|---|---|---|---|---|
| VEGF-Grab3 (from Panolos Bioscience) | | 18.6 | 81.3 | 99.9 |
| VEGF-Grab3 (from Samsung Biologics) | | 48.6 | 51.3 | 99.9 |
| C61 | 74.5 | 25.5 | | 100 |
| C62 | 51 | 29.8 | 19.3 | 100.1 |
| C64 | 48.8 | 29.2 | 21.8 | 99.8 |
| C65 | 96.8 | 3.4 | | 100.2 |
| C69 | 66.5 | 28.1 | 5.5 | 100.1 |
| C71 | 57.6 | 42.5 | | 100.1 |
| C72 | 51.3 | 48.7 | | 100 |
| C73 | 71.1 | 28.8 | | 99.9 |
| Zaltrap | 61.8 | 38.2 | | 100 |

As shown in Table 36 and FIGs. 6 and 7, the existing material VEGF-Grab3 was determined to have a wider and higher pI than Zaltrap. The section where the highest proportion of charge variants exist is the basic section (pI 8.0 <), showing a contrast with Zaltrap, which has the highest distribution in the acidic section (pI 6.0-7.0). In the case of modified fusion proteins of the present disclosure, the distribution in the basic section was reduced (C62) or disappeared (C61, C65, C71, C72, C73), and the section with the maximum distribution moved to the acidic section. Accordingly, a modified fusion protein of the present disclosure is expected to have reduced non-specific interactions with the matrix and cell surface. Additionally, due to this reduction in non-specific interactions, intracellular recycling of the modified fusion protein of the present disclosure may be improved, and the half-life *in vivo* and efficacy of the modified fusion protein of the present disclosure may be improved.

### [Example 5] Thermal stability analysis

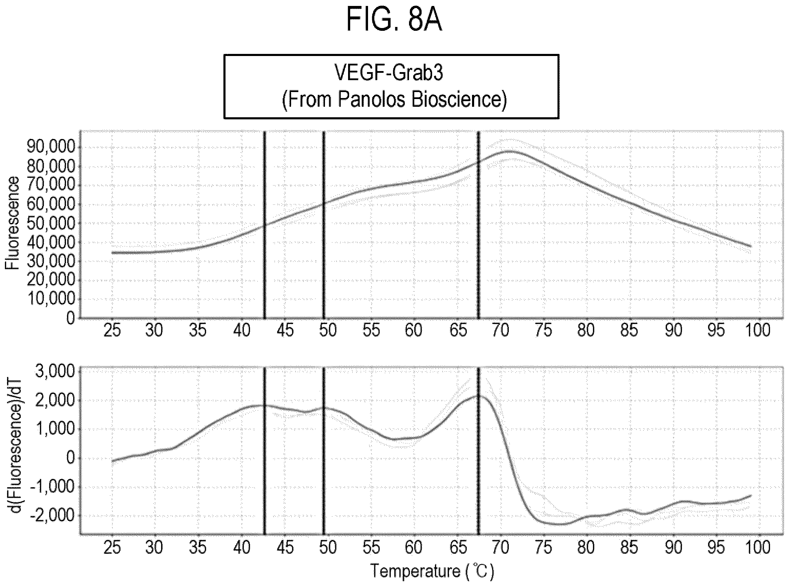

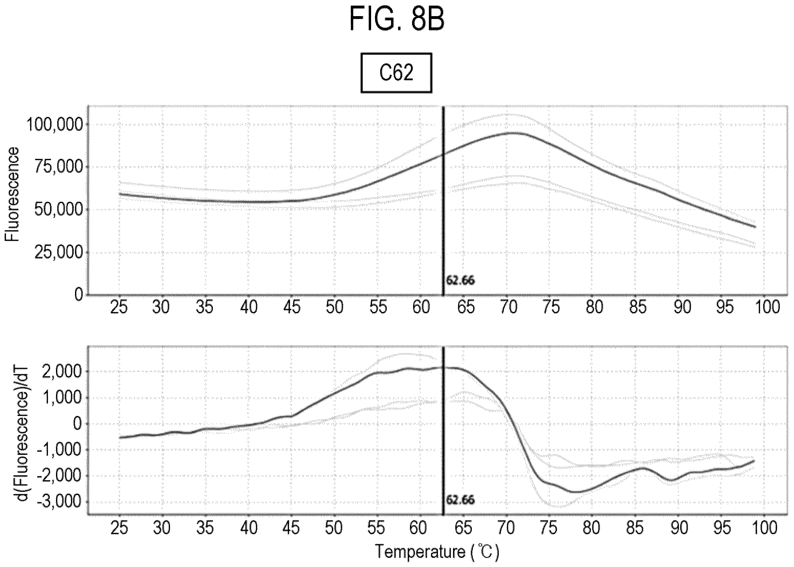

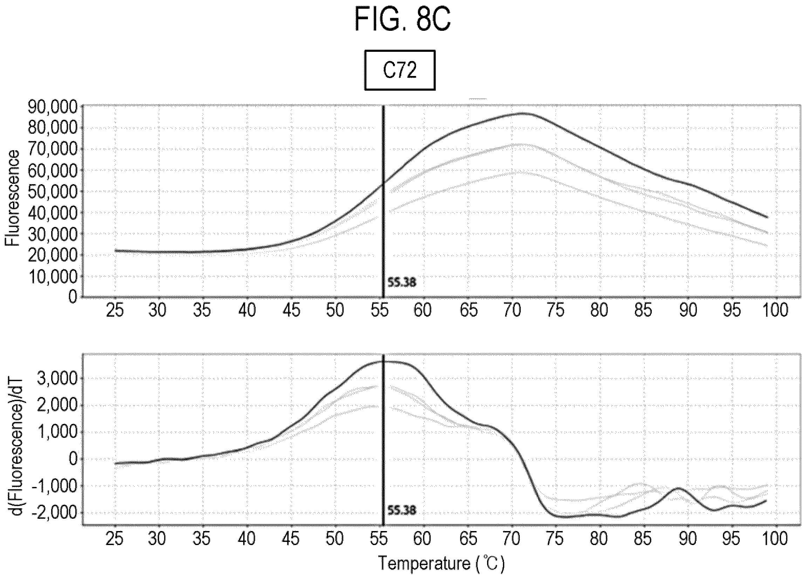

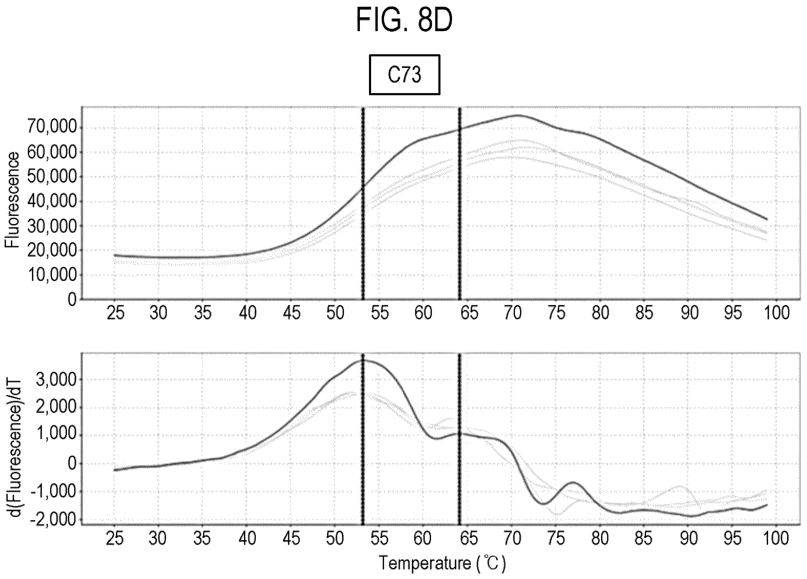

VEGF-Grab3 and modified fusion proteins (C62, C72, C73) of the present disclosure were diluted to a final concentration of 2 mg/ml using 1x PBS buffer. The final reaction solution was prepared by adding 2.5 µl of 8X Protein Thermal Shift^{™} dye (Life Technologies) and 5.0 µl of Protein Thermal Shift^{™} buffer (Life Technologies) to 12.5 µl of protein. 20 µl of the prepared reaction solution was placed in a PCR tube, arranged on a tray of QuantStudio^{™} Real-time PCR equipment, and the Tm value was measured. All analyses were performed in four replicates. The melting curve plot and Tm1, Tm2, and Tm3 values for each protein are shown in FIG. 8 and Table 37.

**Table 37**

| Protein code | Tm1 | Tm2 | Tm3 |
|---|---|---|---|
| VEGF-Grab3 | 41.83 | 48.50 | 67.55 |
| C62 | 61.51 | - | - |
| C72 | 55.12 | - | - |
| C73 | 52.90 | - | - |

As shown in FIG. 8 and Table 37, the existing material VEGF-Grab3 was observed to have melting points in three sections: Tm1 = 41.83°C, Tm2 = 48.50°C, and Tm3 = 67.55°C. For each of the modified fusion proteins (C62, C72, and C73) of the present disclosure, a single Tm point was observed, and the Tm was increased by at least 11°C and up to 20°C compared to the first Tm of VEGF-Grab3. This demonstrates that a modified fusion protein of the present disclosure possesses excellent thermal stability.

### [Example 6] Analysis of VEGF-A inhibition ability

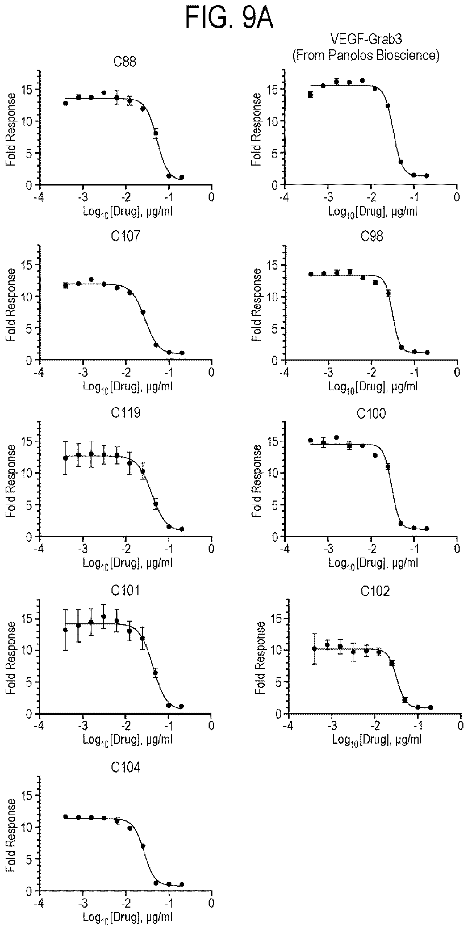

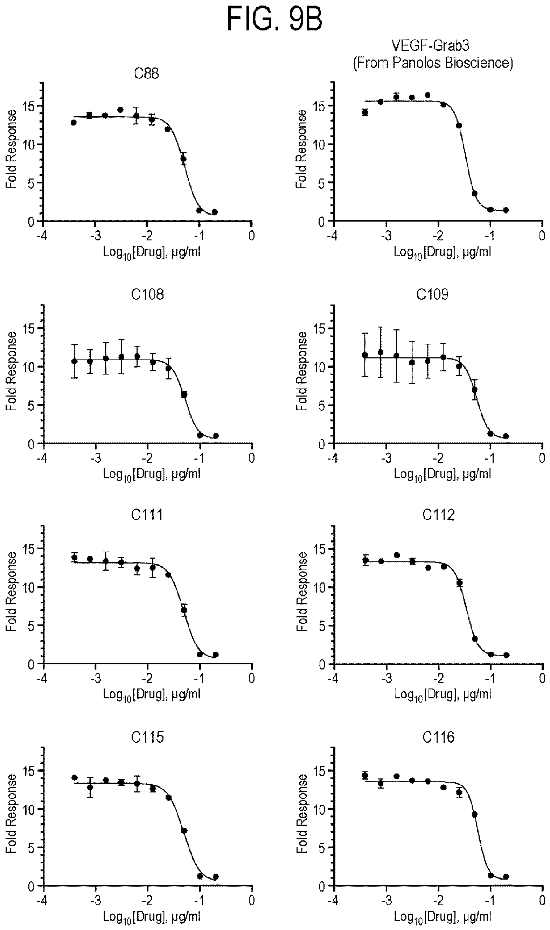

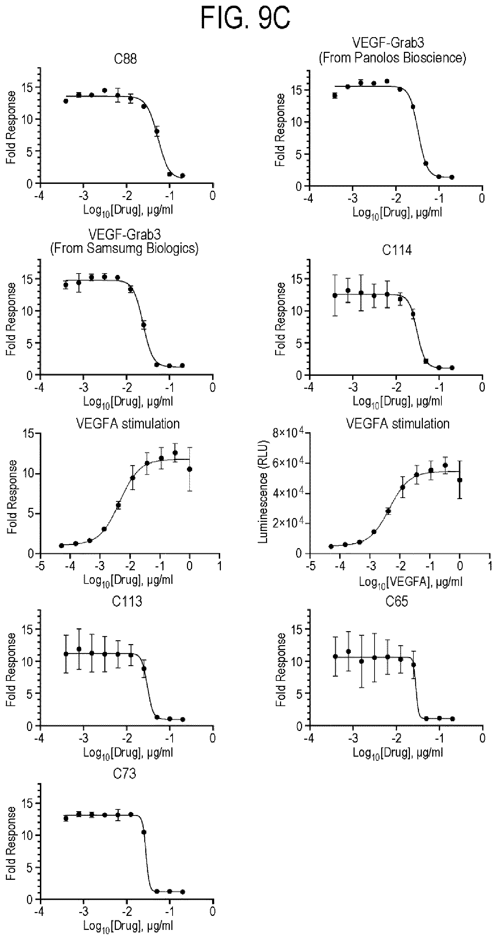

To quantitatively analyze the inhibitory ability against VEGF-A, a genetically engineered cell line (KDR/NFAT-RE HEK293, Promega) having introduced therein a luciferase reporter system based on the expression of VEGFR-2 (KDR) and VEGF-A/VEGFR-2 interaction was used. After dispensing 25 µl of cells at a density of 1.6 x 10⁶ cells/ml is added to a 96-well plate, 25 µl of VEGF-Grab3 or a modified fusion protein of the present disclosure diluted to different concentrations were reacted in an incubator at 37°C, a 5% CO₂ for 6 hours. The plate was then cooled to room temperature for 15 minutes, and then 75 µl of Bio-Glo reagent (Promega, luciferase assay buffer + substrate mixture) was added. After reacting in a light-blocked state for 10 minutes, luminescence was measured using a plate reader. IC₅₀ values for individual substances were calculated by analysis through GraphPad Prism, and the results are shown in Tables 38 to 41 and FIG. 9. In FIG. 9c, the VEGFA stimulation and VEGF stimulation graphs represent the results when only VEGFA or VEGF was treated without any variants.

The data in Tables 38 to 41 each describe the values for proteins produced under the same conditions and batches, and accordingly, the values for VEGF-Grab3 (manufactured by Panolos Bioscience) used as a control are respectively recited.

**Table 38**

| Protein code | IC₅₀ (µg/ml) |
|---|---|
| C88 | 0.05284 |
| VEGF-Grab3 (from Panolos Bioscience) | 0.03341 |
| VEGF-Grab3 (from Samsung Biologics) | 0.02420 |
| VEGF-Grab1(from Panolos Bioscience) | 0.03984 |
| C98 | 0.03115 |
| C119 | 0.04029 |
| C100 | 0.03051 |
| C101 | 0.04404 |
| C102 | 0.03250 |
| C104 | 0.02661 |
| C107 | 0.02775 |
| C108 | 0.05194 |
| C109 | 0.05478 |
| C111 | 0.04882 |
| C112 | 0.03397 |
| C113 | 0.02993 |
| C114 | 0.03100 |
| C115 | 0.04901 |
| C116 | 0.05728 |
| C65 | 0.02867 |
| C73 | 0.02796 |

**Table 39**

| Protein code | IC₅₀ (µg/ml) |
|---|---|
| VEGF-Grab3 (from Panolos Bioscience) | 0.04353 |
| C61 | 0.03218 |
| C62 | 0.02953 |
| C63 | 0.03275 |
| C64 | 0.03324 |
| C65 | 0.04045 |
| C66 | 0.03915 |
| C67 | 0.02917 |
| C68 | 0.03036 |
| C69 | ND |
| C70 | 0.0349 |
| C71 | 0.03073 |
| C72 | 0.03669 |
| C73 | 0.02925 |

**Table 40**

| Protein code | IC₅₀ (µg/ml) |
|---|---|
| VEGF-Grab3 (from Panolos Bioscience) | 0.01314 |
| C74 | 0.007035 |
| C75 | 0.01365 |
| C76 | 0.007567 |
| C77 | 0.00624 |
| C78 | 0.01083 |
| C79 | 0.00881 |
| C80 | 0.01014 |
| C82 | 0.02168 |
| C83 | 0.01982 |
| C85 | 0.02103 |
| C86 | 0.01932 |
| C87 | 0.01808 |

**Table 41**

| Protein code | IC₅₀ (µg/ml) |
|---|---|
| VEGF-Grab3 (from Panolos Bioscience) | 0.003805 |
| C81 | 0.00268 |
| C84 | 0.006157 |

According to FIG. 9 and Tables 38 to 41, it can be seen that both VEGF-Grab3 and modified fusion proteins of the present disclosure exhibit low IC₅₀ values and have excellent VEGF-A inhibitory ability. In the case of variants C115 and C116 having linker sequences that deviate from the linker length of a linker variant according to an embodiment of the present disclosure, they did not exhibit superior VEGF-A inhibition ability compared to the VEGFR-wild type protein.

From the above description, a person of ordinary skill in the art to which the present disclosure pertains will be able to understand that the present disclosure can be implemented in other specific forms without changing its technical idea or essential features. In this regard, the embodiments described above should be understood in all respects as illustrative and not restrictive. The scope of the present disclosure should be construed as including the meaning and scope of the claims described below rather than the detailed description above, and all changes or modified forms derived from the equivalent concept thereof are included in the scope of the present disclosure.

## Claims

1. A modified fusion protein comprising a VEGFR1 extracellular domain, a linker and a multimerization domain,
wherein the VEGFR1 extracellular domain comprises an immunoglobulin (Ig)-like domain D2 and an Ig-like domain D3 of VEGFR1, and the linker is located between the Ig-like domain D3 and the multimerization domain, and
wherein the modified fusion protein has one or more of characteristics (a) to (c):
(a) one or more amino acid residues on a β1-β2 loop of domain D3, one or more amino acid residues on a β2-β3 loop of domain D3, and/or one or more amino acid residues on a β5-β6 loop of domain D3 have been substituted with an amino acid that reduces the net pI of a protein, an amino acid having a negatively charged side chain, or an amino acid having an electronegative side chain,
wherein, the β1-β2 loop of domain D3 includes amino acid residues T236 to T247 of the amino acid sequence of SEQ ID NO: 1, the β2-β3 loop includes amino acid residues T256 to V262 of the amino acid sequence of SEQ ID NO: 1, and the β5-β6 loop includes amino acid residues D299 to L308 of the amino acid sequence of SEQ ID NO: 1;
(b) the linker has a length of about 13 to about 35 amino acids; and
(c) a disulfide bond is present in the fusion protein, and one of the amino acid residues on the β1-β2 loop of domain D3 or one of the amino acid residues on the β5-β6 loop of domain D3, and one of the amino acid residues at positions -2, -1, 0, +1, +2, and +3 relative to Y329 of domain D3 are substituted with cysteine.

2. The modified fusion protein of claim 1, wherein the amino acid substitution in characteristic (a) is a substitution with:
an amino acid residue of the VEGFR2 homologous sequence,
an amino acid residue that is serine, threonine, tyrosine, cysteine, asparagine, glutamine, aspartic acid, or glutamic acid, or
an amino acid residue having a short side chain which is alanine or glycine.

3. The modified fusion protein of claim 1, wherein in characteristic (a), one or more amino acid residues on a β1-β2 loop of domain D3 and one or more amino acid residues on a β5-β6 loop of domain D3 have been substituted with an amino acid that reduces the net pI of a protein, an amino acid having a negatively charged side chain, or an amino acid having an electronegative side chain.

4. The modified fusion protein of claim 3, wherein in characteristic (a), one or more amino acid residues on the β2-β3 loop of domain D3 have been substituted with an amino acid residue having a smaller side chain than the side chain of the existing amino acid residue or an amino acid residue having a polar side chain of a similar size to the side chain of the existing amino acid residue.

5. The modified fusion protein of claim 1, wherein characteristic (a) is substitution of:
one or more of the amino acid residues K241, L243, R244, and H246 on the β1-β2 loop of domain D3;
amino acid residue L258 on the β2-β3 loop of domain D3; and/or
one or more of the amino acid residues K300, Q302, K304, and K306 on the β5-β6 loop of domain D3.

6. The modified fusion protein of claim 5, wherein characteristic (a) comprises:
one or more amino acid substitutions selected from K241T, K241E, L243S, R244V, and H246E on the β1-β2 loop of domain D3;
amino acid substitution L258A, L258S, or L258D on the β2-β3 loop of domain D3; and/or
one or more amino acid substitutions selected from K300G, Q302T, K304S, and K306Q on the β5-β6 loop of domain D3.

7. The modified fusion protein of claim 6, wherein characteristic (a) comprises:
(i) amino acid substitutions L243S and R244V, or (ii) amino acid substitutions K241E, L243S, R244V, and H246E on the β1-β2 loop of domain D3;
amino acid substitution L258A, L258S, or L258D on the β2-β3 loop of domain D3; and/or
(i) amino acid substitution K300G, (ii) amino acid substitutions K300G, Q302T, and K304S, or (iii) amino acid substitutions K300G, Q302T, K304S, and K306Q on the β5-β6 loop of domain D3.

8. The modified fusion protein of claim 7, wherein characteristic (a) comprises:
amino acid substitutions K241E, L243S, R244V, and H246E on the β1-β2 loop of domain D3;
amino acid substitution L258A, L258S, or L258D on the β2-β3 loop of domain D3; and
amino acid substitutions K300G, Q302T, and K304S on the β5-β6 loop of domain D3.

9. The modified fusion protein of claim 1, wherein the modified fusion protein comprises amino acid substitutions R238S and R275N in domain D3.

10. The modified fusion protein of claim 1, wherein the amino acid sequence of the C terminus of domain D3 connected to the linker is KALE based on the amino acid residue K331, provided that one or more of the amino acids K, A, L and E may be deleted.

11. The modified fusion protein of claim 1, wherein the N terminus of domain D2 begins with the amino acid sequence SDT or the amino acid sequence EF.

12. The modified fusion protein of claim 1, wherein in characteristic (b), the linker has a length of about 14 amino acids or more.

13. The modified fusion protein of claim 1, wherein in characteristic (b),
the linker comprises a GS repeat sequence, and
the GS repeat sequence is a 2 to 60 amino acids long amino acid sequence consisting only of G and S, and the N-terminal amino acid of the linker may be glutamic acid.

14. The modified fusion protein of claim 13, wherein the GS repeat sequence is (GS)ₙ, (GSSG)ₙ, (GGGGS)ₙ, or (GS)ₘ(GGGGS)ₙ, wherein n is an integer of 1 to 10, and m is an integer of 0 to 10.

15. The modified fusion protein of claim 14, wherein the GS repeat sequence is selected from the group consisting of GS, GSSG, (GSSG)₂, GGGGS, (GGGGS)₄, GS(GGGGS), and GS(GGGGS)₃.

16. The modified fusion protein of claim 1, wherein in characteristic (b), the linker comprises an amino acid sequence of a hinge region derived from an immunoglobulin, and the amino acid sequence of the hinge region may be modified.

17. The modified fusion protein of claim 16, wherein the hinge region derived from an immunoglobulin comprises a sequence derived from the CH1 region, an upper hinge, and/or a core hinge.

18. The modified fusion protein of claim 17, wherein the hinge region derived from an immunoglobulin is derived from human IgA, IgD, IgM, IgE, or IgG.

19. The modified fusion protein of claim 16, wherein in characteristic (b), a papain recognition site or a glycosylation site present in the hinge region derived from an immunoglobulin has an amino acid variation.

20. The modified fusion protein of claim 19, wherein in characteristic (b),
the amino acid variation in the papain recognition site is substitution of one or more amino acid residues in the papain recognition site with alanine, serine, tyrosine, proline, or threonine, or insertion of a 1 to 10 amino acids long amino acid sequence comprising at least one amino acid having an aromatic or cyclic carbon in the side chain into the papain recognition site, and
the amino acid variation in the glycosylation site is (i) deletion of serine or threonine present in the hinge region derived from an immunoglobulin, or (ii) substitution of serine or threonine present in the hinge region derived from an immunoglobulin with an amino acid other than serine, asparagine, or threonine.

21. The modified fusion protein of claim 20, wherein the amino acid other than serine, asparagine, or threonine is glycine or alanine.

22. The modified fusion protein of claim 17, wherein cysteine present in the core hinge is substituted with serine or glycine.

23. The modified fusion protein of claim 1, wherein in characteristic (b), the linker comprises:
(i) an amino acid sequence selected from the group consisting of GS, GSSG, (GSSG)₂, GGGGS, (GGGGS)₄, GS(GGGGS) and GS(GGGGS)₃; and
(ii) a hinge region derived from an immunoglobulin selected from the group consisting of human IgG1, IgG4, or IgD/G1,
in that order starting from the N terminus, wherein the amino acid sequence of the hinge region may be modified.

24. The modified fusion protein of claim 1, wherein in characteristic (b), the linker is an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP.

25. The modified fusion protein of claim 1, wherein in characteristic (c), said one of the amino acid residues on the β1-β2 loop is one of the amino acid residues on the amino acid sequence of V240 to T247.

26. The modified fusion protein of claim 1, wherein in characteristic (c),
one of amino acid residues T241, L243, and R244 on the β1-β2 loop of domain D3 or amino acid residue N303 on the β5-β6 loop is substituted with cysteine, and
one of the amino acid residues at positions 0, +2, and +3 relative to Y329 of domain D3 is substituted with cysteine.

27. The modified fusion protein of claim 26, wherein in characteristic (c),
T241 and the amino acid residue at position +3 relative to Y329 of domain D3 are substituted with cysteine;
amino acid variations L243C and Y329C are introduced;
L243 and the amino acid residue at position +2 relative to Y329 of domain D3 are substituted with cysteine;
amino acid variations R244C and Y329C are introduced; or
amino acid variations N303C and Y329C are introduced.

28. The modified fusion protein of claim 1, wherein the modified fusion protein has all of characteristics (a) to (c):
(a) one or more amino acid substitutions selected from K241T, K241E, L243S, R244V, and H246E on the β1-β2 loop of domain D3,
amino acid substitution L258A, L258S, or L258D on the β2-β3 loop of domain D3, and/or
one or more amino acid substitutions selected from K300G, Q302T, K304S, and K306Q on the β5-β6 loop of domain D3
are introduced;
(b) the linker is an amino acid sequence selected from the group consisting of GSSGDKTHTSPPSP, GSSGEPKSSDKTYTSPPSP, GSKVDKKVEPKSSDKTHTCPPCP, GSKVDKKVEPKSSDKTYTCPPCP, GSKVDKKVEPKSSDTPPTCPPCP, GSGGGGSGGGGSGGGGSAESKYGPPCPPCP, GSNTGSGGEEKKKEKEKEEQEERSSDKTYTCPPCP, GSNTGSGGEEKKKEKEKEEQEERSSDTPPTCPPCP, EGSSGGSSGEPKSDATPTCPPCP, EGSSGGSSGEPKSDSTYTCPPCP, GCKVDKKVEPKSSDKTYTCPPCP, CSKVDKKVEPKSSDKTYTCPPCP, GGGGSAEPKAGDKAPPGPPGP, GGGGSAEPKSSDKTYTCPPCP, GGGGSGGGGSGGGGSGGGGSAEPKSSDKTYTCPPCP, CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, and CSGGGGSAEPKAGDATPPTCPPCPPCP; and
(c) one of amino acid residues T241, L243, and R244 on the β1-β2 loop of domain D3 or amino acid residue N303 on the β5-β6 loop is substituted with cysteine, and
one of the amino acid residues at positions 0, +2, and +3 relative to Y329 of domain D3 is substituted with cysteine.

29. The modified fusion protein of claim 1, wherein the modified fusion protein has all of characteristics (a) to (c):
(a) (i) amino acid substitutions L243S and R244V, or (ii) amino acid substitutions K241E, L243S, R244V, and H246E on the β1-β2 loop of domain D3,
amino acid substitution L258A, L258S, or L258D on the β2-β3 loop of domain D3, and/or
(i) amino acid substitution K300G, (ii) amino acid substitutions K300G, Q302T, and K304S, or (iii) amino acid substitutions K300G, Q302T, K304S, and K306Q on the β5-β6 loop of domain D3
are introduced;
(b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP; and
(c) L243 and the amino acid residue at position +2 relative to Y329 of domain D3 are substituted with cysteine.

30. The modified fusion protein of claim 1, wherein the modified fusion protein has all of characteristics (a) to (c):
(a) amino acid substitutions K241E, L243S, R244V, and H246E on the β1-β2 loop of domain D3,
amino acid substitution L258A, L258S, or L258D on the β2-β3 loop of domain D3, and/or
amino acid substitutions K300G, Q302T, and K304S on the β5-β6 loop of domain D3 are introduced;
(b) the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CGSSGGSSGEPKSDATPTCPPCP, CSKVDKKVEPKSSDKTYTCPPCP, and CSGGGGSAEPKAGDATPPTCPPCPPCP; and
(c) L243 and the amino acid residue at position +2 relative to Y329 of domain D3 are substituted with cysteine.

31. The modified fusion protein of claim 1, wherein the modified fusion protein consists of an amino acid sequence selected from the group consisting of SEQ ID NO: 10 to SEQ ID NO: 70, SEQ ID NO: 77 to SEQ ID NO: 93, and SEQ ID NO: 98.

32. The modified fusion protein of claim 1, wherein the multimerization domain is:
(a) Fc region of an immunoglobulin;
(b) CH3 region of IgG1, IgG2, IgG3, or IgG4;
(c) CH2 and CH3 regions of IgG1, IgG2, IgG3, or IgG4;
(d) Fc region of an immunoglobulin comprising an amino acid sequence having at least 85% identity to SEQ ID NO:99; or
(e) Fc region of an immunoglobulin comprising the amino acid sequence of SEQ ID NO: 99.

33. The modified fusion protein of claim 1, wherein the multimerization domain is the Fc region of an immunoglobulin and starts from the lower hinge region of the Fc region.

34. The modified fusion protein of claim 1, wherein the multimerization domain is the Fc region of IgG1 consisting of SEQ ID NO: 99.

35. The modified fusion protein of claim 34, wherein the multimerization domain has amino acid substitution D126E, amino acid substitution L128M, and/or the amino acid deletion of K217 based on SEQ ID NO: 99.

36. The modified fusion protein of any one of claims 1 to 35, which is in dimer or multimer form.

37. A pharmaceutical composition for the prevention or treatment of an autoimmune disease, inflammatory disease, neoplastic disease, cancer, angiogenesis-related disease, or ocular disease, comprising the modified fusion protein of any one of claims 1 to 35 as an active ingredient.

38. A nucleic acid molecule encoding the modified fusion protein of any one of claims 1 to 35.

39. A host cell comprising a nucleotide sequence encoding the modified fusion protein of any one of claims 1 to 35.

40. A vector comprising a nucleotide sequence encoding the modified fusion protein of any one of claims 1 to 35.

41. The vector of claim 40, which is a recombinant viral vector.

42. A pharmaceutical composition for delivering a viral vector to a subject, comprising the recombinant viral vector of claim 41, wherein the fusion protein encoded by the recombinant viral vector is expressed in the subject and is intended for the prevention or treatment of an autoimmune disease, inflammatory disease, neoplastic disease, cancer, angiogenesis-related disease, or ocular disease.

43. The pharmaceutical composition of claim 42, wherein the recombinant viral vector is a recombinant adeno-associated viral vector.
